(19) Europäisches Patentamt
European Patent Office
Office européen des brevets

(11) Publication number: **0 537 008 B1**

# (12) EUROPEAN PATENT SPECIFICATION

(45) Date of publication of patent specification: **25.10.95**

(51) Int. Cl.$^{6}$: **A61K 31/66**

(21) Application number: **92309185.4**

(22) Date of filing: **08.10.92**

## (54) Use of biphosphonates for the manufacture of a medicament for blocking neoplastic transformation of cells induced by ras oncogenes.

(30) Priority: **11.10.91 US 774911**

(43) Date of publication of application:
**14.04.93 Bulletin 93/15**

(45) Publication of the grant of the patent:
**25.10.95 Bulletin 95/43**

(84) Designated Contracting States:
**AT BE CH DE DK ES FR GB GR IE IT LI LU MC NL PT SE**

(56) References cited:
**EP-A- 0 409 181**
**EP-A- 0 513 760**

**CELL vol. 65, no. 1, 1991, pages 1 - 4; JACKSON B. GIBBS: 'Ras C-Terminal processing enzymes-New Drug targets?'**

**MOL.CELL.BIOL. vol. 10, no. 11, 1990, pages 5945 - 5949; ROSALIND KIM ET AL.:'Prenylation of mammalian ras protein in xenopus oocytes'**

(73) Proprietor: **E.R. SOUIBB & SONS, INC.**
**Lawrenceville-Princeton Road**
**Princeton**
**New Jersey 08543-4000 (US)**

(72) Inventor: **Biller, Scott A.**
**136 Nancy Lane**
**Ewing, NJ (US)**
Inventor: **Barbacid, Mairiano**
**5 Stoney Creek Pl.**
**Lawrenceville, Nj (US)**
Inventor: **Gordon, Eric M.**
**560 Sand Hill Rd.**
**Palo Alto, CA (US)**
Inventor: **Magnin, David R.**
**40 Cottage Court**
**Hamilton, NJ (US)**
Inventor: **Meyers, Chester A.**
**5 Fox Trail**
**Medford, NJ (US)**
Inventor: **Manne, Veeraswamy**
**260 Marble Court**
**Yardley, PA (US)**

EP 0 537 008 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**JOURNAL OF MEDICINAL CHEMISTRY vol. 31, no. 10, 1988, pages 1869 - 1871; SCOTT A. BILLER ET AL.: 'Isoprenoid (phosphinyl-methyl)phosphonates as inhibitors of squalene synthetase'**

**CELL vol. 62, 1990, pages 81 - 88; YUVAL REISS ET AL.: 'Inhibition of purified p21ras farnesyl: protein transferase by cys-aax tetrapeptides'**

(74) Representative: **Thomas, Roger Tamlyn et al**
**D. Young & Co.**
**21 New Fetter Lane**
**London EC4A 1DA (GB)**

## Description

The present invention relates to the use of biphosphonates for the manufacture of a medicament for treating and/or preventing tumors by blocking the prenylation of CAAX box containing proteins, including ras oncogene products, by administering a therapeutic amount of a protein-prenyl transferase inhibitor which is a bisphosphonate or analog thereof. Other aspects of the invention are set forth in the claims.

The products of ras genes comprise a family of guanine nucleotide binding proteins that are involved in the control of eukaryotic cell proliferation. Specific point mutations result in ras oncoproteins which have the ability to neoplasticly transform mammalian cells, and activated ras genes have been observed in at least 10% of all human tumors. Their incidence in certain malignancies, such as in colorectal and pancreatic cancers, is far greater.

Genetic studies first established that ras proteins, referred to as ras p21, must be formed by post-translational modification of a precursor protein with a defined carboxy-terminal structure, in order to exert their biological function. This structure, known as the CAAX box, is formed of a conserved cysteine residue located four amino acid-residues from the carboxy terminus, which in the case of ras is position 186 (except in the K-ras4B p21 protein, in which cysteine is located at position 185), followed by two aliphatic amino acids and any carboxy-terminal amino acid residue. Mutations affecting the basic CAAX box structure of oncogenic ras p21 proteins completely abolish their transforming activity, presumably by impeding their interaction with the inner side of the plasma membrane. Such interaction requires a series of post-translational modifications within the CAAX box motif which include (a) farnesylation of the cys residue of the CAAX box; (b) cleavage of the three carboxy-terminal amino acid residues; and (c) methylation of the free carboxyl group generated in the resulting carboxy-terminal farnesyl-cysteine residue. The interaction of these farnesylated ras p21 proteins with cellular membranes in some cases is further strengthened by palmitoylation of neighboring upstream cysteine residues. See Hancock, et al, June 30, 1989, Cell 57:1167-1177; and Casey, et al, November 1989, Proc. Natl. Acad. Sci. U.S.A. 86:8323-8327.

Recent studies have suggested that the donor of the farnesyl residue present in ras p21 proteins is farnesyl pyrophosphate (FPP), a precursor in the biosynthesis of cholesterol. The transfer of the farnesyl group from FPP, the donor molecule, to ras proteins is mediated by the enzyme, protein-farnesyl transferase (FT).

Treatment of S. cerevisiae cells or Xenopus oocytes with inhibitors of HMG-CoA reductase, the enzyme responsible for the synthesis of mevalonic acid, the precursor of isoprenoid compounds, blocks the function of ras proteins in these cells. These results have raised the possibility of using inhibitors of cholesterol biosynthesis, that is, HMG CoA reductase inhibitors, to block neoplastic transformation induced by ras oncogenes. See, Schafer, et al, July 28, 1989, Science 245:379-385; and Goldstein and Brown, February 1, 1990, Nature 343:425-430.

Rine and Kim, "A Role for Isoprenoid Lipids in the Localization and Function of an Oncoprotein," The New Biologist, Vol. 2, No. 3 (March), 1990: pp 219-236, disclose at pages 222-223 that "lovastatin [also known as Mevacor], compactin, and related drugs that have been developed for the treatment of hypercholesterolemia act by inhibiting 3-hydroxy-3-methylglutaryl coenzyme A reductase (HMG-CoA reductase), the enzyme that catalyzes the rate-limiting step in the synthesis of cholesterol and all other polyisoprenoids.... The drugs were tested in the Xenopus oocyte assay... for their ability to pharmacologically suppress activated H-Ras$^{vall2}$.... These experiments pinpointed farnesyl pyrophosphate as the likely donor molecule for farnesylation of Ras protein, and suggested a rationale for a novel pharmacological route to block the action of this important human oncoprotein."

"Earlier work had already provided evidence that inhibition of isoprenoid synthesis by use of inhibitors of 3-hydroxy-3-methylglutaryl (HMG)-CoA reductase could slow the growth of tumors in animals. In particular, continuous, high levels of lovastatin caused substantial growth inhibition of a mouse neuroblastoma.... Although the oncogene(s) responsible for this tumor has not yet been identified and the dosage required to suppress the tumor was rather high, this study does support the notion that protein prenyl transferase(s) responsible for Ras modification might serve as useful targets for chemotherapy...."

European patent application No. 91107390.6, published as EP-A-456,180 on 13th November 1991, discloses protein-farnesyl transferase (FT) assays for identifying compounds that block the farnesylation of ras oncogene products. The EP-A-456,180 invention is based, in part, on the discovery and identification of the FT enzyme which catalyses the transfer of the farnesyl group from the donor, farnesyl pyrophosphate (FPP), to the ras p21 $Cys^{186}$ residue. Farnesylation of ras proteins is required for their attachment to the inner cell membrane and biological activity. Farnesylation of ras oncogene products is required for ras mediated transforming activity. Because the assays of the EP-A-456,180 invention are designed to target a step subsequent to the synthesis of FPP (in the cholesterol chain), they allow for the identification of

compounds that interfere with farnesylation of the ras oncogene products and inhibit their transforming activity, yet do not interfere with the synthesis of FPP, a precursor in the synthesis of cholesterol, ubiquinones, dolichols and Haem A. Therefore, FT inhibitory compounds that do not disrupt important cellular pathways which require FPP may be identified using the EP-A-456,180 assay.

Squalene synthetase is a microsomal enzyme which catalyses the reductive dimerization of two molecules of farnesyl pyrophosphate (FPP) in the presence of nicotinamide adenine dinucleotide phosphate (reduced form) (NADPH) to form squalene (Poulter, C. D.; Rilling, H. C., in "Biosynthesis of Isoprenoid Compounds," Vol. I, Chapter 8, pp. 413-441, J. Wiley and Sons, 1981, and references therein). This enzyme is the first committed step of the de novo cholesterol biosynthetic pathway.

Squalene synthetase inhibitors which block the action of squalene synthetase (after the formation of farnesyl pyrophosphate) are disclosed in U.S. Patent Nos. 4,871,721 and 5,025,003, European patent application No. 89115400.7 (EP-A-356,866), European patent application No. 90113700.0 (EP-A-409,181), and European patent application No. 92108074.3.

Preferred aspects of the invention will now be described.

In accordance with the present invention, it has been found that post-translational modification of CAAX box containing proteins may be inhibited by administering a protein-prenyl transferase inhibitor which inhibits the transfer of the prenyl group [such as farnesyl (in the case of ras oncogene products), geranyl or geranylgeranyl] to the cysteine of the CAAX box by the protein-prenyl transferase enzyme. The protein-prenyl transferase inhibitor will block the protein-prenyl transferase enzyme from catalyzing the transfer of the prenyl group (for example, farnesyl, geranyl or geranylgeranyl) from the prenyl pyrophosphate to the cys residue of the CAAX box, such as the ras p21 cys, or to the CAAX box cysteine of other CAAX box containing proteins. In the case of ras p21 oncogene products, inasmuch as the cys will not be farnesylated it cannot effect interaction of the ras protein with the membrane so that neoplastic transformation of the cell will be prevented. In this manner protein-prenyl transferase inhibitors prevent neoplastic transformation of the cell, thereby acting as an anti-cancer agent for the treatment of and/or prevention of ras-related tumors.

Examples of CAAX box containing proteins which have been demonstrated or are believed to undergo prenylation include, but are not limited to, nuclear lamins, $\alpha$ or $\gamma$ subunits of heterotrimeric G-proteins, $\gamma$-subunits of retinal transducin, G25K and K-rev p21, and protein families including rho, rap, rac, ral, and rab.

Thus, the present invention resides in a method for blocking or preventing the prenylation of CAAX box containing proteins such as ras oncogene products, and thereby inhibit disease promoting effects of the CAAX box containing protein or more specifically prevent and/or treat ras-related tumors, by administering to a patient in need of treatment a therapeutic amount of a protein-prenyl transferase inhibitor.

The protein-prenyl transferase inhibitors, unlike HMG CoA reductase inhibitors, will interfere with prenylation of the ras oncogene products and inhibit their transforming activity, yet may or may not interfere with the synthesis of FPP, a precursor in the synthesis of ubiquinones, dolichols and Haem A.

The activity of the protein-prenyl transferase inhibitors in blocking the protein-prenyl (e.g. farnesyl, geranyl or geranylgeranyl) transferase from catalyzing the transfer of the prenyl group (e.g. farnesyl, geranyl or geranylgeranyl) from the prenyl pyrophosphate to the cys residue of the CAAX box may be assayed by the procedure described in EP-A-456,180.

The bisphosphonate compounds disclosed in European patent application No. 92108073.5 (EP-A-513,760) include a methylene bridge between the phosphonate moities and includes at least one lipophilic group attached to the methlene bridge.

As will be seen hereinafter, the terms "bisphosphonic," "diphosphonic," "bisphosphonates" and "diphosphonates" are used interchangeably.

The term "lipophilic group" refers to a group which preferably contains at least six carbons (more preferably greater than 10) and preferably less than 2 polar substituents bearing OH, NH or C = O functions.

The bisphosphonates disclosed in Application No. 92108073.5 have the structure

I

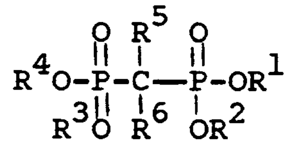

wherein $R^1$, $R^2$, $R^3$ and $R^4$ are the same or different and are H, alkyl, aryl, alkylaryl, arylalkyl, ammonium, alkali metal or a prodrug ester, preferably no more than one of $R^1$, $R^2$, $R^3$ and $R^4$ is alkyl, wherein at least

one of $R^5$ and $R^6$ is a hydrocarbyl group having at least 6 carbons (such as alkyl, alkenyl, alkynyl, cycloalkyl, aryl, alkylaryl, arylalkyl, arylalkenyl); heterocyclic (such as succinimdyl, pyridyl, quinalyl, morpholino, furanyl, indolyl, picolinyl, thiophene, imidazole, oxazole, isoxazole, thiazole, pyridine, 1,2,3-triazole, 1,2,4-triazole, benzimidazole, tetrahydrofuranyl, pyrrolidino, piperidino, 5-membered heteroarylmethyl containing 2 to 4 N atoms or 1-2 N atoms plus an O or S atom); heterocyclicalkyl (wherein heterocyclic is as defined above such as 1-(decahydroquinolin-3-yl)methane); amino; alkylamino; dialkylamino; arylalkylaminoalkyl; ethylcarbonyloxymethylamino; cycloalkyl(alkyl)amino; alkenylamino, cycloalkylamino, aminocycloalkyl; aminocycloalkylalkyl; N-hydroxy-N-ethylamino; acetylamino; aminoalkyloxyalkyl; (benzo- or cyclohexeno-fused) 5 membered heteroaryl containing 2-4 N atoms or 1-2 Natoms plus an O or S atom; $R^8$-X-$(CH_2)_a$- (wherein $R^8$ is H, alkyl, or a nitrogen containing 6-membered aromatic ring such a pyridyl, indanyl, hexahydroindanyl or picolyl; X is O, NH or a single bond and a is 0 to 7);

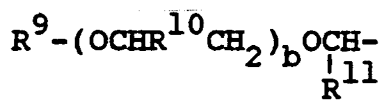

(wherein $R^9$ is $C_1$-$C_{10}$ alkyl, optionally substituted aryl, phenylalkyl or naphthylalkyl),

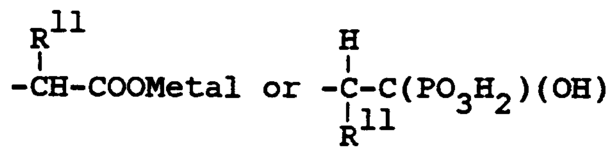

(wherein $R^{10}$ and and $R^{11}$ are the same or different and are H or methyl, b is 1 to 20));

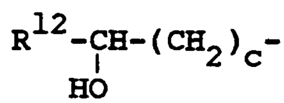

(wherein $R^{12}$ is H, phenyl or phenyl substituted with halogen, alkyl or hydroxy and c is 0 to 9);

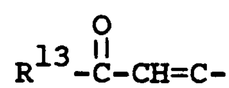

(wherein $R^{13}$ is tert-alkyl ($CR^{14}R^{15}R^{16}$ wherein $R^{14}$ and $R^{15}$ are independently $C_1$-$C_3$ alkyl and $R^{16}$ is $C_1$-$C_{10}$ alkyl), cycloalkyl, aryl or heteroaryl, or substituted cycloalkyl, substituted aryl or substituted heteroaryl wherein the substituent is halogen, $C_1$-$C_4$ alkyl, alkoxy or dialkylamino);

4-Cl-$C_6H_5$-S-$CH_2$; aryloxy;

$R^{17}$-$(QCH_2CH_2)_d$O- (wherein $R^{17}$ is $C_1$-$C_{10}$ alkyl, $C_2$-$C_{10}$ alkenyl, aryl or arylalkyl, or each of the above $R^{17}$ groups optionally substituted with $C_1$-$C_4$ alkyl, amino, alkylamino, carboxyl, alkoxycarbonyl, hydroxy, alkoxy, phenoxy, mercapto, alkylthio, phenylthio, halogen or trifluoromethyl, Q is O or S and d is 0, 1 or 2);

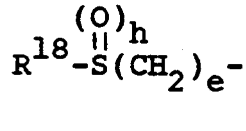

(wherein e is 0 to 10, h is 0, 1 or 2, $R^{18}$ is H, cycloalkyl, aryl, alkyl, each optionally substituted with OH, SH, halogen, alkoxycarbonyl or $NZ_1Z_2$, phenyl optionally substituted with halogen, nitro, lower alkyl, alkoxy, trifluoromethyl, amino, carboxyl, $CO_2$alkyl, -$CONZ_1Z_2$, -$CSNZ_1Z_2$, a 5- or 6-membered heterocyclic radical containing 1 or 2 heteroatoms, which are N or S, which may or may not be fused to a benzene ring, $Z_1$ and $Z_2$ are independently H or lower alkyl);

thiol; phenylthio; chlorophenylthio; 4-thiomorpholinyl;

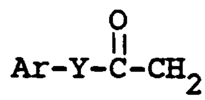

(wherein Ar is aryl, pyrrolyl or aryl optionally substituted with $C_1$-$C_4$alkyl, alkoxy, halo (F, Cl), naphthyl, biphenyl or thienyl and Y is NH or a single bond);

$R^{19}SCH_2$- (wherein $R^{19}$ is alkyl, aryl or arylalkyl);

A-$(CH_2)_r$-NH- (wherein A is $C_5$-$C_8$ cycloalkenyl, bicycloheptyl, bicycloheptenyl, saturated $C_4$-$C_7$ heterocycle containing O,S,SO or $SO_2$);

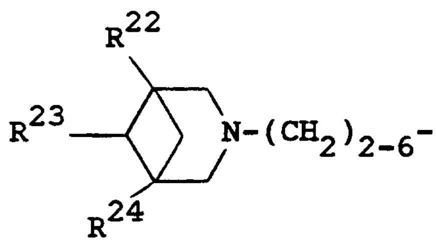

(wherein $R^{22}$ is H, $C_1$-$C_{20}$alkyl, alkoxy, aryl, $R^{23}$ is H, $C_1$-$C_{20}$alkyl, alkoxy, aryl, halo, carboxyl, $R^{24}$ is H, $C_1$-$C_{20}$ alkyl, alkoxy);

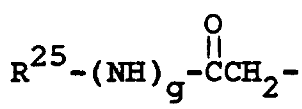

(wherein $R^{25}$ is (alkyl-substituted)pyrrolyl or phenyl and g is 0 or 1);

aromatic-substituted mono- or biazacyclylalkyl (alkyl group bonds with the N in the heterocycle) (such as 3-(4-phenylpiperidino)propyl);

$R^{31}$-Ax-CO-$CH_2$-

(wherein Ax is phenyl, naphthyl, mono- or bicyclic-N-containing heterocycle and $R^{31}$ is H, halo, lower alkyl or lower alkoxy);

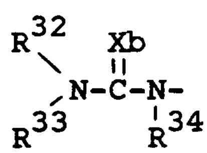

(wherein $R^{32}$ is aryl, aralkyl, alkyl, $R^{33}$ is H or aryl, Xb is O or S, and $R^{34}$ is H or alkyl);

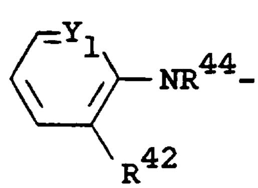

(wherein $R^{42}$ is H, alkyl or halo, $Y_1$ is N, NO, or $NR^{43}Y_2$ wherein $R^{43}$ is alkyl and $Y_2$ is halo; and $R^{44}$ is H or aliphatic acyl);

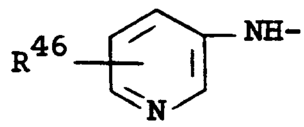

(wherein $R^{46}$ is H, halo or alkyl);

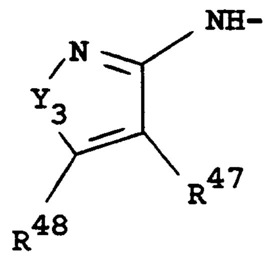

(wherein $Y_3$ is O or NH, $R^{47}$ is H, alkyl or halo, and $R^{48}$ is H or alkyl);

$R^{50}$ -NH-

(wherein $R^{50}$ is

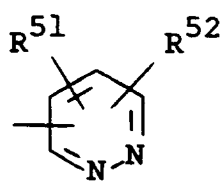

,

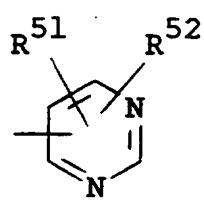

or

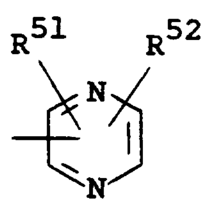

wherein $R^{51}$ and $R^{52}$ are H, halo, alkyl or hydroxy);

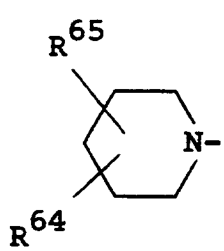

(wherein $R^{64}$ is alkyl and $R^{65}$ is H or alkyl;

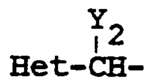

wherein Het is a heteroaromatic 5-membered ring with 2 or 3 heteroatoms, optionally partially hydrogenated and optionally substituted by one or more alkyl, alkoxy, phenyl, cyclohexyl, cyclohexylmethyl, halo or amino, with 2 adjacent alkyl optionally together forming a ring (Het cannot be pyrazole), and $Y_2$ is H or lower alkyl);

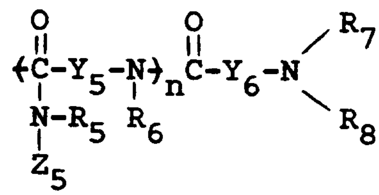

(wherein $Y_4$ is H or OH, $R_5$-$R_8$ are independently H or lower alkyl, whereby $R_7$ and $Y_6$ or $R_6$ and $Y_5$ or $R_5$ and $Z_5$, together with the nitrogen atom to which they are attached can form a 5- or 6-membered ring, $Y_6$ and $Y_5$ which can be the same or different are $C_1$-$C_6$ alkylene chains optionally substituted by aromatic or heteroaromatic radicals, $Z_5$ is $C_1$ to $C_6$ alkylene which can include heteroatoms and optionally substituted by aromatic or heteroaromatic, n is 0, 1 or 2;

$R_{27}$-$Z_9$-

(wherein $R_{27}$ is aryl or heterocyclyl both optionally substituted by one or more of lower alkyl, lower alkoxy, lower alkylthio, halo(lower)alkyl, acyl, acylamino or halo, or $R_{27}$ is lower alkyl substituted by heterocyclyl which is optionally substituted by acyl); $R_{27}$-$Z_9$ is $R_{27}$-NHC(=$X_9$), $R_{27}$-C(=O)NH-, $R_{27}$-$SO_2$-NH- (wherein $X_9$ is O or S);

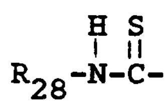

(wherein $R_{28}$ is phenyl, pyridyl or quinolyl substituted by lower alkylsulphonylamino, halo-lower alkylsulphonylamino, arylsulphonylamino and mono- or di-lower alkylamino);

$R_{29}$-CO-[-$R_{30}$($CH_2$)$_o$CO-]$_p$-NH-

(wherein $R_{29}$-CO- is a residue of a pharmaceutically active compound $R_{29}$-COOH, wherein $R_{29}$ is an anti-inflamatory agent, or antioncotic agent or hormone ,

$R_{30}$ is -NH- or -O-

p is 0 or 1;

o is 1-10);

$R_{33}$-($CH_2$)$_q$-

(wherein $R_{33}$ is an N-bonded azabicycloalkyl group with 3 to 8-membered rings and q is 2 to 4);

$R_{34}$-($CH_2$)$_r$-

(wherein $R_{34}$ is an N-bonded, aryl-substituted mono- or diazacycloaliphatic group);

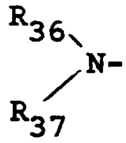

(wherein $R_{36}$ is 5 membered heteroaryl with 2-4 N or with 1-2 N plus an O or S atom, optionally fused to a benzo or cyclohexeno ring;

$R_{36}$ can be C substituted by lower alkyl, phenyl (optionally substituted by lower alkyl, alkoxy and/or halo), lower alkoxy, OH, di(lower alkyl)amino, lower alkylthio and/or halo, and/or N substituted by lower alkyl or phenyl (lower) alkyl (optionally substituted by lower alkyl, lower alkoxy and/or halo);

$R_{37}$ is H or lower alkyl; provided $R_{37}$ is not H if $R_{36}$ is optionally substituted alkyl and/or halo substituted 3-pyrazolyl or 3-isoxazolyl);

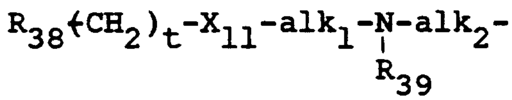

(wherein $R_{38}$ is aromatic residue;

t is 0-3;

$X_{11}$ is 0 S (optionally oxidized) or imino (optionally substituted by aliphatic group);

$alk_1$ and $alk_2$ are divalent aliphatic groups; $R_{39}$ is H or monovalent aliphatic group);

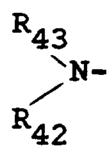

(wherein $R_{42}$ and $R_{43}$ are hydrogen, alkyl having one to 22 carbon atoms, cycloalkyl having five to six carbon atoms, phenyl alkylphenyl having seven to 18 carbon atoms, phenylalkyl having seven to 18 carbon atoms and together with the nitrogen atom, piperidino, pyrrolidino and morpholino);

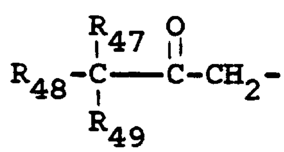

(wherein $R_{47}$ is optionally branched $C_1$-$C_8$alkyl,

$R_{48}$ and $R_{49}$ are each methyl or ethyl, and

M is H or a cation of a water-soluble base);

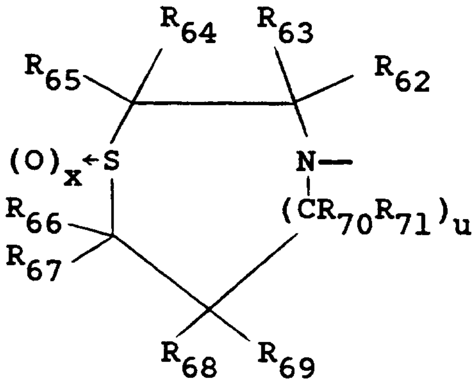

(wherein $R_{62}$-$R_{71}$ is H, straight, branched or alicyclic 1-10C hydrocarbyl, aryl or aryl-(1-4C)-alkyl;

x is 0 or 1;

u is 0, 1 or 2;

or $R_{62}$ and $R_{64}$ may complete a 5- to 7-membered saturated aliphatic ring optionally substituted by 1 or more alkyl groups);

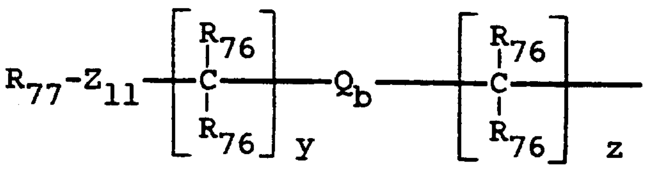

(wherein $Z_{11}$ is an N-containing 6-membered ring heterocycle moiety selected from piperidinyl, diazinyl or triazinyl;

$Q_b$ is a covalent bond, O, S or $NR_{76}$;

y, z, and y + z are integers of 0-10;

$R_{76}$ is H, or $C_1$-$C_3$alkyl;

$R_{77}$ is one or more substituted selected from H, halogen, 1-3C alkyl, unsubstituted amino and its amide derived from a 1-3C carboxylic acid, mono(1-3C alkyl) amino and its amide derived from a 1-3C carboxylic acid, di(1-3C alkyl)amino, tri(1-3C alkyl) ammonium, hydroxy or its ester derived from a 1-3C carboxylic acid, ether having 1-3C, $CO_2H$ and its salts and esters derived from 1-3C alcohols, its amide optionally substituted with one or two 1-3C alkyl groups, and $NO_2$);

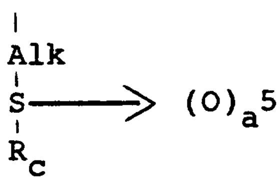

(wherein $R_c$ represents:

$C_1$-$C_6$ alkyl group,

$C_5$-$C_7$ cycloalkyl group,

phenyl group optionally monosubstituted or polysubstituted by a halogen, a $C_1$-$C_6$ alkyl group or a trifluoromethyl group, or

5-membered or 6-membered heterocycle containing 1 or 2 heteroatoms chosen from nitrogen and sulfur,

Alk denoted a linear or branched $C_1$-$C_6$ alkylene group,

$a^5$ represents 0 or the integer 1 or 2);

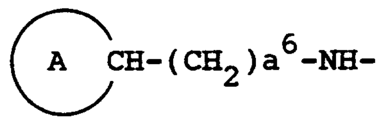

(wherein $a^6$ is 0 to 4 and

Ring A is 5-8C cycloalkenyl, bicycloheptyl, bicycloheptenyl or 4-7C saturated heterocyclyl containing 0, S, SO or $SO_2$);

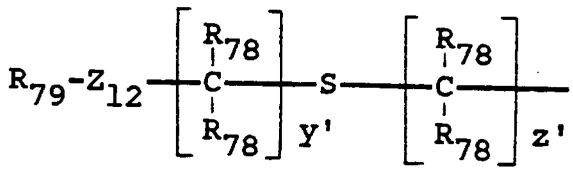

(wherein $Z_{12}$ is a 6-membered aromatic ring containing ≧ 1 N atom(s); where:

the ring is optionally substituted by (optionally substituted, optionally unsaturated) 1-6C alkyl, (optionally substituted) aryl, (optionally substituted) benzyl, OH, halogen, carbonyl, alkoxy, $NO_2$, $CONH_2$, (optionally substituted) $NH_2$ and/or carboxylate, such as pyridine, pyridazine, pyrimidine or pyrazine ring;

$R_{78}$ is H or (optionally substituted, optionally unsubstituted) 1-4C alkyl;

$R_{79}$ is H, (optionally substituted, optionally unsubstituted) 1-6C alkyl, (optionally substituted) aryl, (optionally substituted) benzyl, OH, halogen, carbonyl, alkoxy, $NO_2$, $CONH_2$, (optionally substituted) amino or carboxylate,

y' + z' is 0 to 5);

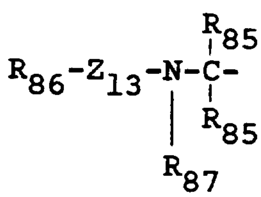

or

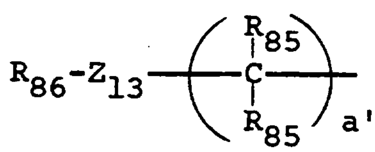

(wherein $Z_{13}$ is a pyridine, pyridazine, pyrimidine or pyrazine ring, optionally substituted by optionally unsaturated 1-6C alkyl, optionally substituted aryl, optionally substituted benzyl, OH, halogen, oxo, alkoxy, $NO_2$, amido, optionally substituted $NH_2$ or carboxylate;

$R_{86}$ is H or optionally substituted, optionally unsaturated 1-4C alkyl;

$R_{86}$ is one or more of H, optionally substituted, optionally unsaturated 1-6C alkyl, optionally substituted aryl, optionally substituted benzyl, OH, halogen, oxo, alkoxy, $NO_2$, amido, optionally substituted $NH_2$ or carboxylate;

$R_{87}$ is H, optionally substituted, optionally unsaturated 1-4C alkyl or acyl;

a' is 1-5);

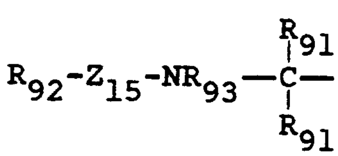

or

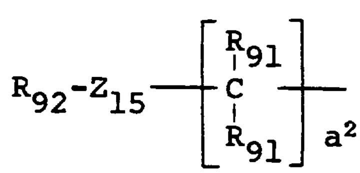

(wherein $Z_{15}$ is a 6 membered aromatic ring containing one or more N atoms such as pyridine, pyridazine, pyrimidine or pyrazine, which ring may be substituted with one or more optionally substituted, optionally unsaturated 1-6C alkyl, optionally substituted aryl, optionally substituted benzyl, OH, halogen, carbonyl, alkoxy, $NO_2$, amido, optionally substituted amino or carboxylate);

$R_{91}$ is H or optionally substituted, optionally unsaturated 1-4C alkyl;

$R_{92}$ is H or one or more substituents selected from optionally substituted, optionally unsaturated 1-6C alkyl, optionally substituted aryl, optionally substituted benzyl, OH, halogen, carbonyl, alkoxy, $NO_2$, amido, optionally substituted amino or carboxylate;

$R_{93}$ is H, optionally substituted, optionally unsaturated 1-4C alkyl or acyl;

$a^2$ is 1 to 5);

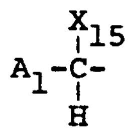

(wherein $X_{15}$ is hydrogen, methyl, or ethyl, and $A_1$ is phenyl substituted in the para-position by isobutyl, cyclohexyl, alkoxy, or 1-pyrrolinyl and, optionally substituted additionally in the meta-position by fluorine or chlorine, or phenyl substituted in the meta-position by benzoyl or phenoxy, or phenyl substituted in the ortho-position by 2,4-dichlorophenoxy or 2,6-dichlorophenylamino);

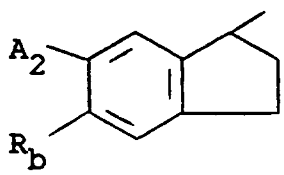

(wherein

$R_b$ is cyclohexyl or cyclophenylmethyl; and

$A_2$ is hydrogen or chlorine);

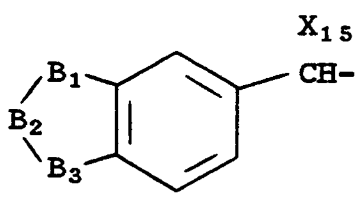

(wherein $X_{15}$ is as defined above, and

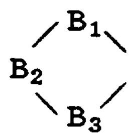 is

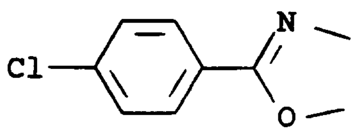

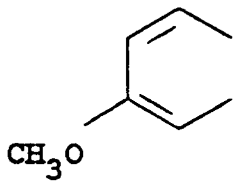 or 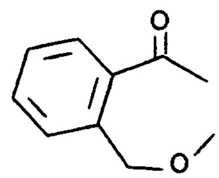 ;

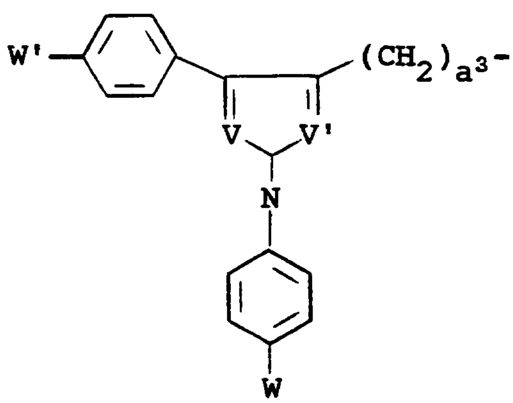

wherein $a^3$ is 1, 2 or 3;

W and W', are identical or different, and each is hydrogen, fluorine or chlorine, and

one of V and V' is nitrogen and the other is a methyne residue optionally substituted by a phenyl group, and

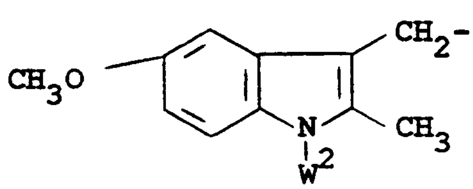

wherein $W^2$ is p-chlorobenzoyl or cinnamoyl);

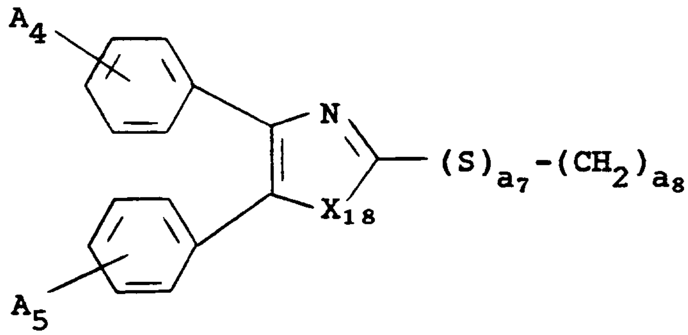

(wherein $A_4$ and $A_5$ are the same or different and are H, OH, lower alkoxy or halogen;
$X_{15}$ is O, S or NH;
$a_7$ is 0 or 1;
$a_8$ is 0 or an integer of 1-6);

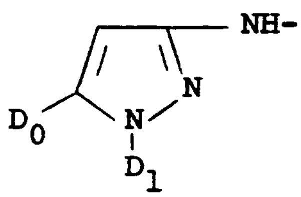

(wherein $D_0$ is H or alkyl;
$D_1$ is H or lower alkyl);

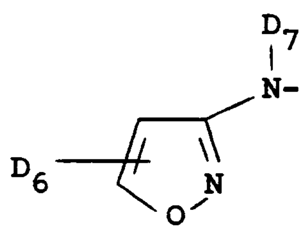

(wherein $D_6$ is H, 1-10C alkyl, 3-10C cycloalkyl, phenyl, 2-10C alkenyl (optionally substituted by phenyl) or phenyl(1-5C)alkyl (optionally ring-substituted by a 1-5C alkoxy);
$D_7$ is H or 2-6C alkanoyl);

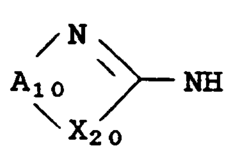

(wherein $A_{10}$ is a group of formula (a)-(c):

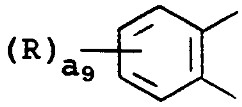

(a)

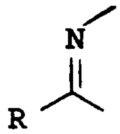

(b)

or

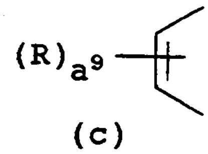

(c)

and $X_{20}$ is O, S or NH);

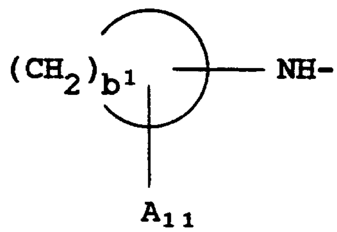

(wherein $A_{11}$ is H or 1-5C alkyl;
$b_1$ is 3-10);

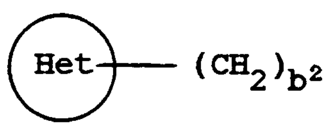

(wherein ring Het is a group of formula (A) or (B):

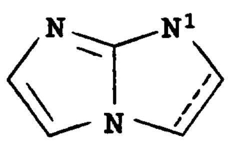

(A)

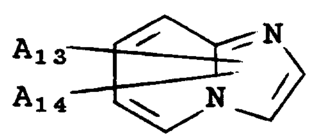

(B)

the dotted line represents an optional double bond; $A_{13}$, $A_{14}$ are H, 1-5C alkyl, halogen or OH);

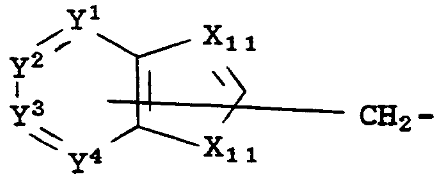

(wherein $X_{11}$ are both N or one is N and the other is CH; one of $Y^1$-$Y^4$ is N and the rest is CH);

and the other of $R^5$ and $R^6$ is H, halogen, $C_1$-$C_{30}$ alkyl, amino, alkylamino, dialkylamino, uriedo ($NH_2CO$-$N(R^{38})$- where $R^{38}$ is H, alkyl, benzyl, phenyl optionally substituted with Cl or $CH_3$); alkenylamino,

cycloalkylamino, aryloxy, pyridinium, guanidinium, ammonium, di-and tri-lower alkanolammonium, hydroxy, arylalkyl, alkoxy, alkylaryloxy, $-CH_2CO_2H$, $-CH_2PO_3H_2$, $-CH(PO_3H_2)(OH)$, $-CH_2CO_2C_2H_5$, $-CH_2CH(PO_3H_2)_2$, a hydrocarbyl radical as defined herein, a heterocyclic radical as defined herein, alkanoyl, an $R^6$ or $R^5$ radical as defined herein, a prodrug ester (such as (1-alkanoyloxy)alkyl, for example $t\text{-}C_4H_9CO_2CH_2$-, $CH_3CO_2CH_2$-);

at least one of $R^5$ and $R^6$ being a lipophilic group, or $R^5$ and $R^6$ can be joined to form a carbocyclic ring containing 3 to 12 carbons or a heterocyclic ring containing N, O and/or S atoms, such as of the formula

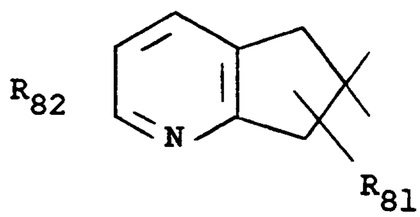

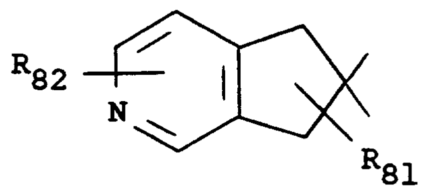

or 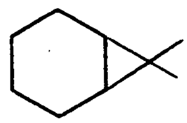

wherein $R_{81}$ and $R_{82}$ are each one or more substituents selected from H, optionally substituted saturated or unsaturated 1-6C alkyl, optionally substituted aryl, optionally substituted benzyl, amido, OH, halogen, optionally substituted amino, amido, COOH, carbonyl, carboxylate, alkoxy and $NO_2$.

The various preferred bisphosphonate compounds of structure I which may be employed in the method of the invention are outlined below.

a) methylene diphosphonic acids and salts and esters as disclosed in U.S. Patent Nos. 3,299,123, 3,414,393 and 3,518,200 all to Fitch et al (all assigned to Monsanto), U.S. Patent Nos. 3,463,835, 3,471,406, 3,892,676 and 4,440,646 all to Budnick (all assigned to Plains Chemical Development Co.) (disclosed for use as surfactants, metal ion sequestering and deflocculating agents for detergents, gasoline additives, dry cleaning agents) having the formula

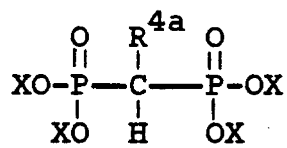

wherein $R^{4a}$ is an aliphatic hydrocarbyl (for example, alkyl, aralkyl), alicyclic (for example, cycloalkyl), aryl, alkylaryl of from 5 to 30 carbon atoms and carbon containing heterocyclics (such as those set out above with respect to $R^4$ and $R^5$) (any of $R^{4a}$ being optionally substituted with OH, halo, alkoxy, ester, ether, nitro, sulfonyl, amido, amino, carboxyl or nitroso); and

X is H, alkali metal, alkaline earth metal, aluminum, ammonium, amine and aliphatic hydrocarbyl, aryl, alkylaryl of from 1 to 30 carbons;

b) alkylenediphosphonic acids and/or salts disclosed in U.S. Patent Nos. 3,297,578 to Crutchfield et al, and 3,346,487 to Irani et al (both assigned to Monsanto) (used in detergents) and having the formula

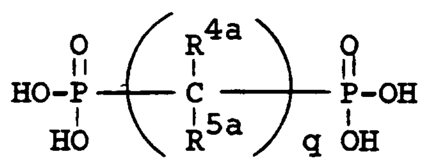

wherein $R^{4a}$ is H or $C_1$-$C_4$ alkyl, and $R^{5a}$ is H, OH or $C_1$-$C_4$ alkyl;

c) substituted methylene diphosphonic acids esters, or salts thereof as disclosed in U.S. Patent Nos. 3,404,178 and 3,422,021 each to Roy (both assigned to Procter & Gamble) (used in detergents) having the formula

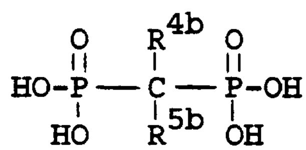

wherein $R^{4b}$ and $R^{5b}$ are each selected from H, methyl, benzyl or carboxymethylene ($CH_2CO_2H$), at least one of $R^{4b}$ and $R^{5b}$ being other than H;

d) alkyldiphosphonic acids, esters or salts as disclosed in U.S. Patent No. 3,609,075 to Barbera (assigned to Procter & Gamble) having the formula

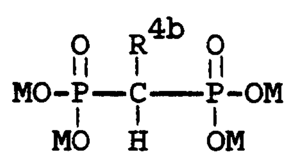

wherein $R^{4b}$ is alkyl of from 12 to 30 carbons and M is H, $C_1$ to $C_8$ alkyl, alkali metal or ammonium;

e) diphosphonates as disclosed in U.S. Patent Nos. 3,488,419 to McCune et al, 3,683,080 to Francis and 3,678,154 to Widder et al, (disclosed for use in compositions for inhibiting deposition and mobilization of calcium phosphate, arthritis, atherosclerosis) (not related to cholesterol biosynthesis inhibition or cholesterol lowering) having the formula

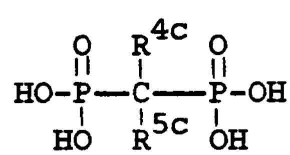

wherein $R^{4c}$ is H, $C_1$ to $C_{20}$ alkyl, $C_2$ to $C_{20}$ alkenyl, aryl, phenylethenyl, benzyl, halo, amino, substituted amino (for example, dimethylamino, diethylamino, N-hydroxy-N-ethylamino, acetylamino), $-CH_2COOH$, $-CH_2PO_3H_2$, $-CH(PO_3H_2)(OH)$ or $-CH_2CH(PO_3H_2)_2$,

$R^{5c}$ is H, lower alkyl, amino, benzyl, halo, OH, $-CH_2CO_2H$, $-CH_2PO_3H_2$, $-CH_2CH_2PO_3H_2$,

at least one of $R^{4c}$ and $R^{5c}$ being a lipophilic group;

[The following additional patents disclose type e) diphosphonate compounds:

U.S. Patent Nos. 4,330,530 to Baker (disclosed for use with gold salts for treating arthritis); 4,067,971 to Francis (disclosed for use in hypoxias and ischemic tissue diseases), 4,254,114 to Triebwasser (disclosed for use in control of pyrophosphate microorganisms), 4,137,309 to Van Duzee (disclosed for use in sickle cell anemia), European Patent Application 88462A2 (disclosed for use with steroids for anti-inflammatory utilities)];

f) methanecycloalkylhydroxydiphosphinates as disclosed in U.S. Patent No. 3,553,314 to Francis (assigned to Procter & Gamble) [disclosed for use in oral compositions for calculus retardation and for treating deposition and mobilization of calcium phosphate including atherosclerosis (unrelated to cholesterol inhibition or lowering)] having the formula

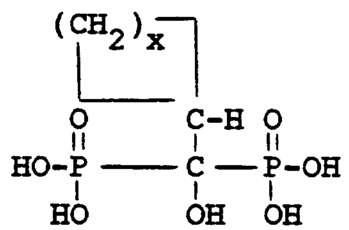

wherein x is 1 to 7 so that the ring may contain 4 to 10 carbons, including salts thereof such as alkali-metal, alkaline earth metal, non-toxic heavy metal, ammonium or low molecular weight substituted ammonium.

[The following additional patents disclose type f) compounds: U.S. Patent Nos. 3,959,458 to Agricola et al, 4,025,616 and 3,934,002 both to Haefele (all relating to therapeutic substances for use in toothpaste compositions), and U.S. Patent No. 3,584,125 to Francis (relating to substances for inhibiting anomalous deposition and mobilization of calcium phosphates in animal tissue, arthritis, atherosclerosis (unrelated to cholesterol inhibition or lowering)); GB 1,453,667 (for containing radio-active P for treatment of tumors) (all assigned to Procter & Gamble)];

g) alkyldiphosphonates as disclosed in U.S. Patent No. 4,113,861 to Fleisch (assigned to Procter & Gamble) (disclosed or use in treatment of diabetes) having the formula

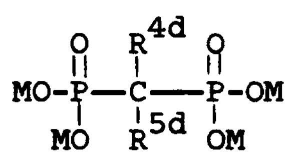

wherein $R^{4d}$ is a $C_2$ or higher hydrocarbyl group (preferably containing 6 to 13 carbons) such as unsubstituted or substituted alkyl, cycloalkyl, alkenyl, alkynyl or carbocyclic group (cycloalkyl)

$R^{5d}$ is H, OH or $NH_2$,

M is H, metal ion (such as alkali metal),

alkyl or aryl;

i) polyphosphonates as disclosed in U.S. Patent No. 4,761,406 to Flora et al (assigned to Procter & Gamble) (disclosed for treating or preventing osteoporosis) having the formula

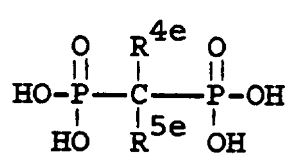

wherein $R^{4e}$ is $R^7$-X-$(CH_2)_a$-

wherein $R^7$ is H or a nitrogen-containing 6-membered aromatic ring (such as pyridyl, indanyl, hexahydroindanyl, picolyl);

X is -NH-, oxygen or a single bond;

"a" is 0 to 7;

$R^{5e}$ is H, Cl, amino or OH;

j) bisphosphonates as disclosed in Dervent No. 85-223756 (Akad Wissenschaft DDR) and DE 3804686 (Henkel) (disclosed for use in anticancer compositions) having the formula

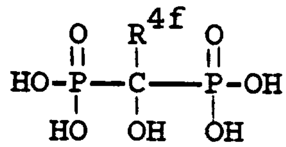

wherein $R^{4f}$ is $C_6$ to $C_{17}$ alkyl (Akad Wissenschaft) and $C_1$ to $C_9$ alkyl (Henkel) containing amino, carboxylate and other substituents);

k) bisphosphonates as disclosed in DE 3,425,746 (Amersham Buchler) (disclosed for use in diagnosis and treatment of bone tumors, and other diseases of the skeletal system) having the formula

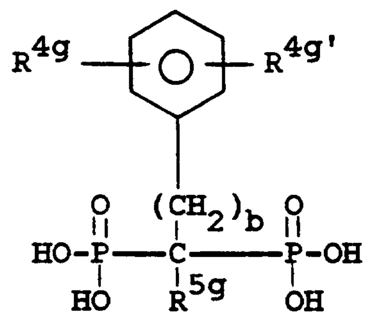

wherein $R^{4g}$ and $R^{4g'}$ are independently H, alkyl, aryl, OH, alkoxy, aryloxy, amino, alkyl- or arylamino, carboxylalkyl, mercapto, alkyl- or arylthio, halo, nitro, cyano, sulfonic or sulfonamide, and $R^{5g}$ is H, alkyl, aryl, OH, halo, amino, $PO_3H_2$ (alkyl has 1 to 6 carbons and is branched or unbranched, aryl has 6 to 14 carbons and either may be substituted);

l) oxa-alkane diphosphonic acids as disclosed in U.S. Patent No. 4,892,679 to Blum et al (assigned to Henkel Kommanditgeselsschaft) (disclosed for use as metal ion complexing agents and in diseases of calcium and phosphate metabolism) having the formula

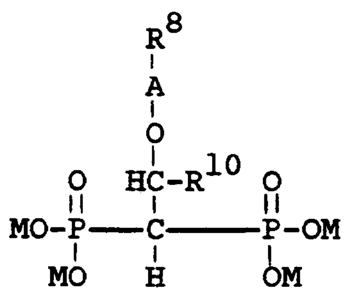

wherein A is

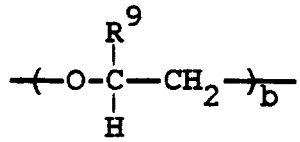

where b is 1 to 20,

$R^8$ is $C_1$-$C_{10}$ alkyl, optionally substituted $C_6$-$C_{10}$ aryl, phenylalkyl, naphthylalkyl,

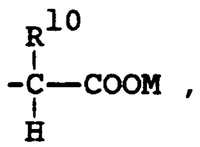 , 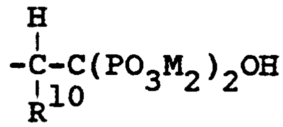

$R^9$ and $R^{10}$ are independently H or $CH_3$; and
M is H or a monovalent cation;

m) dihydroxyalkane diphosphonic acids as disclosed in U.S. Patent No. 4,536,348 to Blum (assigned to Henkel) (disclosed for use as complexing and sequestering agents and in diseases of calcium and phosphate metabolism) having the formula

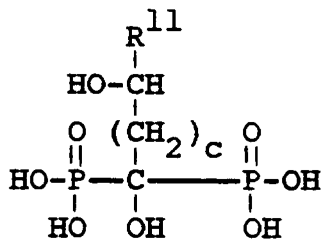

wherein $R^{11}$ is H, phenyl, phenyl substituted with halo, $C_1$ to $C_6$ alkyl or OH and c is 1 to 9;

n) 3-oxo-propene-1,1-diphosphonic acids as disclosed in European Patent 0301352A2 (disclosed for use as a calcium complexing agent and in diseases of calcium and phosphate metabolism and tartar prevention) having the formula

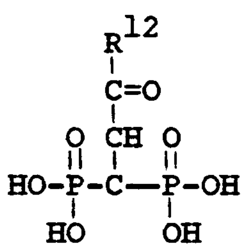

wherein $R^{12}$ is tert-alkyl $CR^{13}$, $R^{14}$, $R^{15}$ (wherein $R^{13}$ and $R^{14}$ are independently $C_1$-$C_3$alkyl and $R^{15}$ is $C_1$-$C_{10}$ alkyl); cycloalkyl; aryl; or heteroaryl; or cycloalkyl, aryl or heteroaryl substituted with halo, $C_1$-$C_4$alkyl, alkoxy or dialkylamino;

o) lipophilic bisphosphonates as disclosed in WO88/00829 (Leo Pharmaceutical Products) (disclosed for use for nasal administration in diseases involving calcium metabolism and arthritis) having the formula

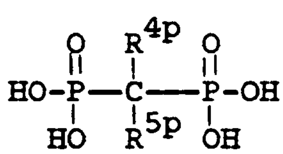

wherein $R^{4p}$ is alkyl, phenoxy, 4-Cl-$C_6H_4$-S-$CH_2$-and $R^{5p}$ is H or OH;

p) methylene bisphosphonic acids as disclosed in WO86/00902 (Leo Pharmaceutical Products) (disclosed for use in various diseases involving calcium phosphate deposition or resorption, including atherosclerosis, and prevention of dental calculus) having the formula

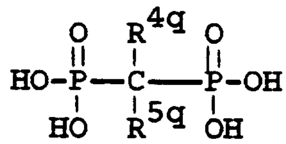

wherein $R^{4q}$ is $R^{16}$-$(QCH_2CH_2)_dO$- wherein $R^{16}$ is a straight or branched, saturated or unsaturated aliphatic or alicyclic $C_1$-$C_{10}$ hydrocarbon radical, an aryl or an aryl-$C_1$-$C_4$-alkyl radical, $R_1$ if desired being unsubstituted or substituted with straight or branched $C_1$-$C_4$-alkyl, amino, $C_1$-$C_4$-alkylamino, di-($C_1$-$C_4$-alkyl)-amino, carboxy, $C_1$-$C_4$-alkoxycarbonyl, hydroxy, $C_1$-$C_4$-alkoxy, phenoxy, mercapto, $C_1$-$C_4$-alkylthio, phenylthio, halogen, trifluoromethyl;

$R^{5q}$ is hydrogen, $C_1$-$C_8$-alkyl, aryl-$C_1$-$C_4$-alkyl or halogen;

Q is O or S, and d is 0, 1 or 2; with the proviso that $R^{5q}$ cannot be hydrogen or methyl if d = O and $R^{1b}$ is methyl,

including double esters thereof;

q) cyclic diphosphonic acids as disclosed in U.S. Patent No. 4,687,768 and European Patent Applications 0304961 and 0304962 all to Benedict et al and assigned to Procter & Gamble (disclosed for treating diseases characterized by abnormal calcium and phosphate metabolism) having the formula

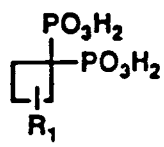

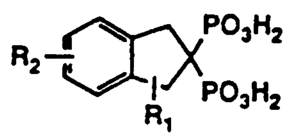

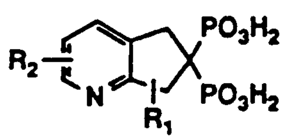

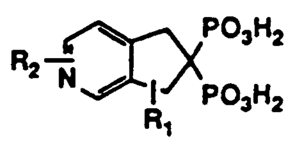

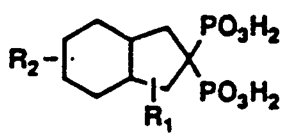

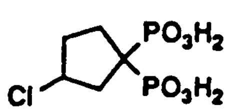

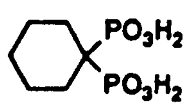

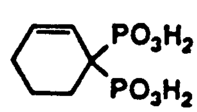

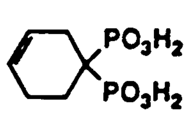

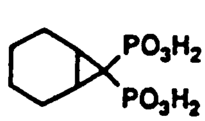

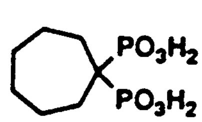

wherein $R_1$ represents one or more substituents including alkyl, alkenyl, aryl, benzyl, hydroxy, halogen, amino, amido, carboxy, carbonyl, carboxylate, alkoxy and combinations thereof, and

$R_2$ represents hydrogen, nitro, or any of the groups defined under $R_1$;

wherein the above $R_1$ and/or $R_2$ groups may optionally include substituents such as alkyl, alkenyl,

aryl, halogen, hydroxy or cycloalkyl;

r) bisphosphonates as disclosed in U.S. Patent No. 4,515,766 to Castronovo et al (assigned to Mass. Gen. Hosp.) (disclosed for use in forming radiolabeled compounds for scanning for calcium deposits) having the formula

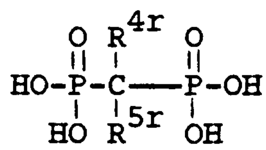

wherein $R^{4r}$ is aryl, and $R^{5r}$ is H, alkyl, alkenyl, amino, benzyl, OH, $-CH_2PO_3H$, $-CH_2CH_2PO_3O_2$.

s) bisphosphonates as disclosed in Derwent No. 79-00757C/01 (Japanese Patent No. 55-4147-925) (disclosed for use as herbicides) having the formula

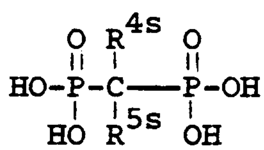

wherein $R^{4s}$ and $R^{5s}$ are independently H, halo, alkyl or cycloalkyl, at least one of $R^{4s}$ and $R^{5s}$ being a lipophilic group;

t) diphosphonic acids as disclosed in U.S. Patent Nos. 4,836,956 and 4,818,774 each to Kem (each assigned to Occidental Chemical Corp) (disclosed for use in extraction of uranium and other metals from water) having the formula

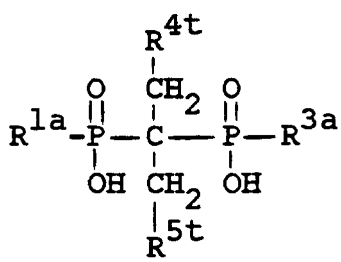

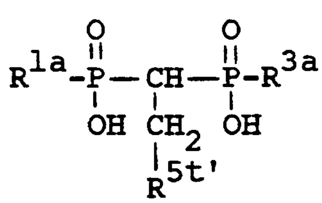

wherein $R^{1a}$ and $R^{3a}$ are the same or different and are alkyl and alkylaryl groups having from 1 to about 18 carbon atoms or hydroxyl;

$R^{4t}$ is independently selected from substituted or unsubstituted alkyl or alkylaryl groups having 1 to about 18 carbon atoms or hydrogen;

$R^{5t}$ is independently selected from alkyl or alkylaryl groups having 1 to about 18 carbon atoms; provided that the sum of carbon atoms of the R groups is at least 15; and

$R^{5t'}$ is independently selected from substituted and unsubstituted alkyl or alkylaryl groups having 1 to about 18 carbon atoms or a polymeric group; provided that the sum of the carbon atoms of the $R^1$, $R^{1a}$, $R^{3a}$ and $R^{5t'}$ groups is a least 16.

Substituted alkyl and alkylaryl groups include alkyl and alkylaryl groups substituted with moieties, such as fluoro, chloro, bromo, iodo and hydroxyl group;

u) bisphosphonate acids as disclosed in European Patent Application 185589A (Rhone-Poulenc) (used for treatment of Paget's disease and osteoporosis) having the formula

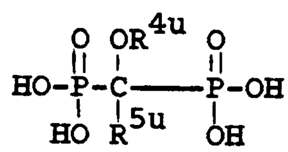

wherein $R^{4u}$ is $C_2$ or $C_3$ alkyl and $R^{5u}$ is H or alkyl; or $R^{4u}$ is $C_{1-3}$ alkyl and $R^{5u}$ is $C_{1-2}$alkyl.

v) methylene diphosphonic acids as disclosed in U.S. Patent Nos. 4,746,654 and 4,876,248 both to Breliere (both assigned to Sanofi) (disclosed for use as anti-inflammatory agents) having the formula

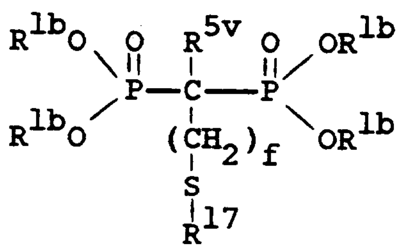

wherein $R^{1b}$ is hydrogen or a straight or branched lower alkyl group having from 1 to 4 carbon atoms;

$R^{17}$ is hydrogen, an alkyl group which is unsubstituted or substituted by a hydroxyl group, a thiol group, one or more halogen atoms, an alkoxycarbonyl group or a

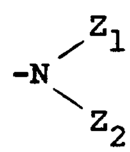 group,

where $Z_1$ and $Z_2$, considered independently of one another, are hydrogen or a lower alkyl group, a phenyl group which is unsubstituted or has one or more halogen, nitro group, lower alkyl group, lower alkoxy group, trifluoromethyl, $NH_2$ group, COOH group or COOalkyl group, a

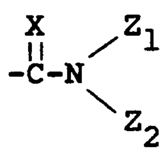 group,

where X is oxygen or sulfur and $Z_1$ and $Z_2$ are as defined above, a heterocyclic radical with 5 or 6 members, containing 1 or 2 heteroatoms chosen from amongst nitrogen and sulfur, or, a heterocyclic radical with 5 members fused to a benzene ring and having the formula

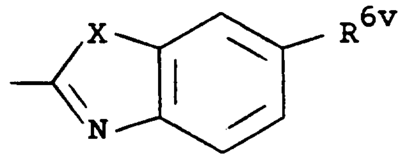

where X is oxygen, an NH group or sulfur and $R^{6v}$ is hydrogen or a halogen atom, preferably chlorine, $R^{5v}$ is hydrogen or a hydroxyl group, and f is an integer between 0 and 10, with the proviso that f cannot be 0 if $R^{5v}$ is OH;

w) bisphosphonic acids as disclosed in European Patent Application 336851A (Sanofi) (disclosed for use with sodium lauryl sulfate (for oral availability) for treating rheumatism and arthritis) having the formula

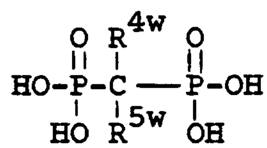

wherein $R^{4w}$ is halo, $C_{1-5}$ alkyl (optionally substituted with Cl, OH, $NH_2$ or dialkylamino), phenoxy, phenyl, thio, phenylthio, chlorophenylthio, pyridyl, 4-thiomorpholinyl and $R^{5w}$ is H, halo, OH, $NH_2$, dialkylamino;

x) diphosphonic acids as disclosed in U.S. Patent No. 4,503,049 to Biere et al (assigned to Schering A.G.) (disclosed for use as anti-inflammatory and antiarthritic agents and for diseases involving calcium) having the structure

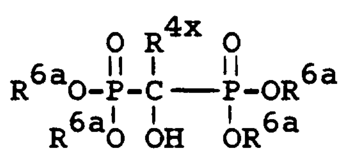

wherein $R^{6a}$ is hydrogen, an alkali metal atom, an alkaline earth metal atom, or alkyl of 1-4 carbon atoms, and $R^{4x}$ is the residue of a carboxylic acid containing an aromatic or heteroaromatic group and being of the formula

arylCOOH,

diphosphonic acid derivatives of the formula

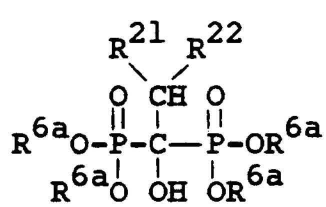

wherein $R^{6a}$ is as defined above;

$R^{21}$ is hydrogen, methyl, or ethyl; and

$R^{22}$ is phenyl substituted in the para-position by isobutyl, cyclohexyl, alkoxy, or 1-pyrrolinyl and, optionally substituted additionally in the meta-position by fluorine or chlorine; or phenyl substituted in the meta-position by benzoyl or phenoxy, or phenyl substituted in the ortho-position by 2,4-dichlorophenoxy or 2,6-dichlorophenylamino;

diphosphonic acid derivatives of the formula

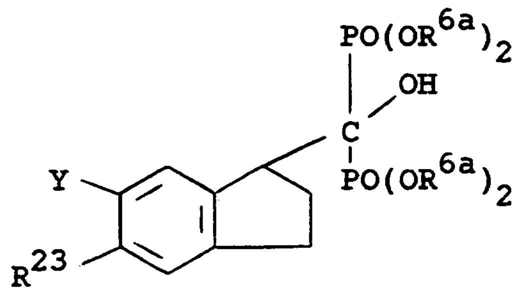

wherein $R^{6a}$ is as defined above;

$R^{23}$ is cyclohexyl or cyclopentylmethyl; and Y is hydrogen or chlorine;

diphosphonic acid derivatives of the formula

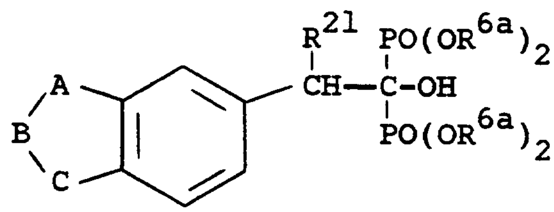

wherein $R^{6a}$ and $R^{21}$ are as defined above, and

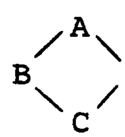

is

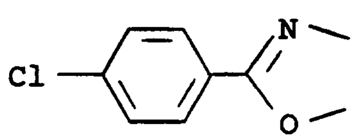

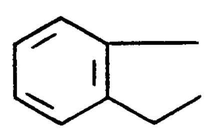

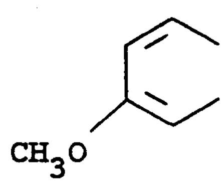

or

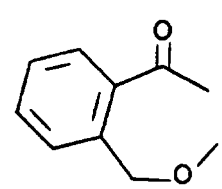

;

diphosphonic acid derivatives of the formula

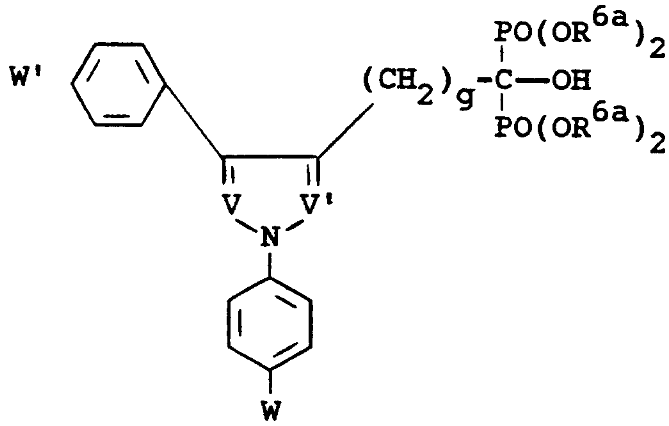

wherein g is 1, 2, or 3;

$R^{6a}$ is as defined above;

W and W', are identical or different, and each is hydrogen, fluorine or chlorine, and

one of V and V' is nitrogen and the other is a methyne residue optionally substituted by a phenyl group; and

diphosphonic acid derivatives of the formula

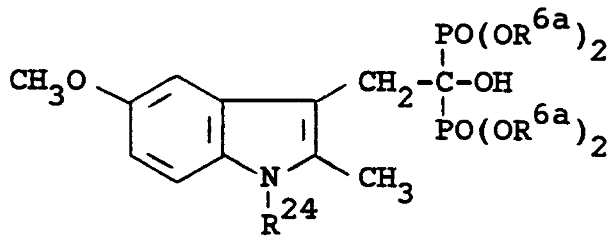

wherein $R^{6a}$ is as defined above, and

$R^{24}$ is p-chlorobenzoyl or cinnamoyl; or, throughout, when $R^{6a}$ is H, a physiologically acceptable salt thereof with an organic base;

y) diphosphonic acids as disclosed in U.S. Patent No. 4,473,560 to Biere et al (assigned to Schering AG) (disclosed for use as anti-inflammatory and antiarthritic agents, and for diseases involving calcium, such as Paget's disease, osteoporosis, and ectopic calcification) having the formula

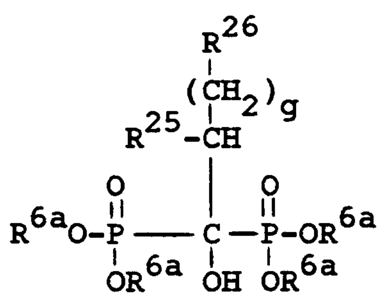

wherein g is 0, 1 or 2;

$R^{25}$ is H or $C_1$-$C_4$ alkyl;

$R^{6a}$ is H, alkali metal, alkaline earth metal, or $C_1$-$C_4$ alkyl; and

$R^{26}$ is phenyl optionally substituted by F atom(s), Cl atom(s), alkyl group(s) of 1-4 carbons, alkoxy

group(s) of 1-4 carbons; naphthyl; biphenyl; or thienyl; or salts thereof;

z) bisphosphonates as disclosed in Japanese Patent JK 73-54,278 (used in peroxide bleaching solutions) having the formula

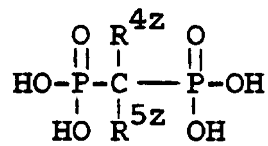

wherein $R^{4z}$ is H or alkyl and

$R^{5z}$ is H, OH or alkyl, at least one of $R^{4z}$ and $R^{5z}$ being a lipophilic group;

aa) alkylenepolyphosphonic acids as disclosed in U.S. Patent No. 4,276,089 to Moran (assigned to Union Chimique et Industrielle de l'Ouest S.A.) (disclosed for use with polyamines as corrosion inhibitors) having the structure

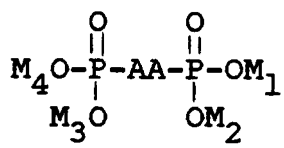

wherein AA represents a bivalent alkylene group which is a straight and saturated $C_1$-$C_{10}$ hydrocarbon chain, each carbon of which may be optionally substituted by at least one of OH, $C_1$-$C_4$ alkyl and phosphonic group

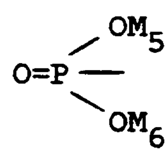

and $M_1$ to $M_6$ may be the same or different and are H, $C_1$-$C_4$ alkyl, $NH_4^+$ or a metal cation;

bb) bisphosphonates as disclosed in DD 237,252A, Derwent No. 86-291914/45 (assigned to Veb Chem Bitterfeld) (disclosed for use as a plant growth regulator) having the formula

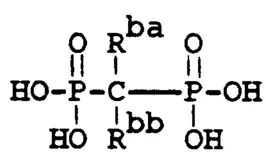

wherein $R^{ba}$ is H, lower alkyl, pyrrolidino or piperidino and

$R^{bb}$ is H, OH or lower alkyl, at least one of $R^{ba}$ and $R^{bb}$ being a lipophilic group;

cc) bisphosphonates as disclosed in Japanese Patent No. J-6-3,295,595A, Derwent No. 89-019688/03 (assigned to Yamanouchi Pharm KK) (disclosed or use as anti-inflammatory, and analgesic agents, and for bone abnormalities due to rheumatism, arthritis and osteoporosis), having the formula

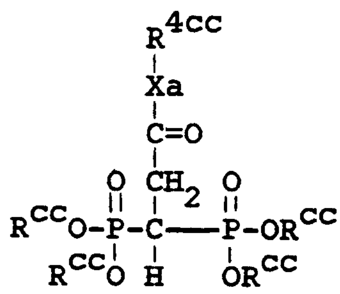

wherein $R^{cc}$ is H or lower alkyl;

Xa is NH or a bond; and

$R^{4cc}$ is phenyl or pyrrolyl each optionally substituted with alkyl;

dd) bisphosphonates as disclosed in USSR SU 862,439 (disclosed for use as collecting agents for the flotation of tin containing ore) having the formula

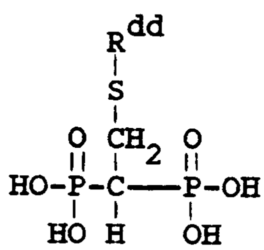

wherein $R^{dd}$ is a $C_3$-$C_{10}$ aliphatic (such as alkyl), aromatic (such as phenyl) or arylaliphatic (such as benzyl) hydrocarbyl group;

ee) substituted aminomethylenebis(phosphonic acid) derivatives as disclosed in European Patent Application 337706 (Yamanouchi Pharmaceutical Co.) (disclosed for use in bone resorption-inhibitory and anti-inflammatory effects) having the formula

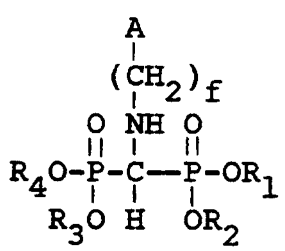

wherein $R_1$-$R_4$ is H or alkyl, f is o to 4 and A is $C_5$-$C_8$ cycloalkenyl, bicycloheptyl, bicycloheptenyl, saturated $C_4$-$C_7$ heterocyclyl containing O, S, SO or $SO_2$;

ff) araliphatyl aminoalkyldiphosphonic acids as disclosed in European Patent Application 320455 (Ciba-Geigy) (disclosed for use in disorders of calcium metabolism) having the formula

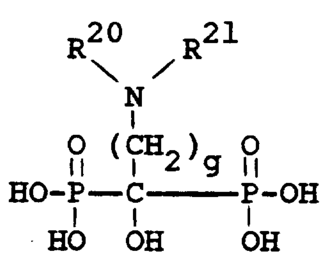

wherein $R^{20}$ is arylaliphatic residue, $R^{21}$ is H or aliphatic residue, and $(CH_2)_g$ is a divalent aliphatic residue where g is 1 to 6;

gg) azabicycloheptanes as disclosed in European Patent Application 317506 (Ciba-Geigy) (disclosed for use as calcium metabolism modulators) having the formula I wherein $R^5$ is

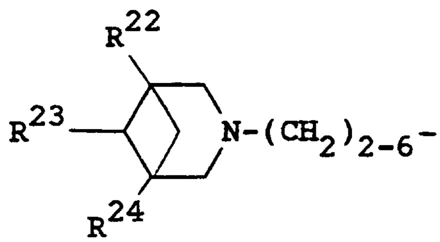

(wherein $R^{22}$ is H, $C_1$-$C_{20}$alkyl, alkoxy, aryl;

$R^{23}$ is H, $C_1$-$C_{20}$alkyl, alkoxy, aryl, halo, carboxyl;

$R^{24}$ is H, $C_1$-$C_{20}$alkyl, alkoxy);

hh) 3-oxopropylidene-1,1-diphosphonates as disclosed in Japanese Patent Application 87-271,433 (Yamanouchi Pharmaceutical Co.) (disclosed for use as inflammation inhibitors, analgesics, antipyretics) having the formula I wherein $R^5$ is

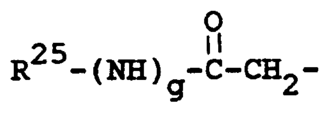

wherein $R^{25}$ is (alkyl-substituted)pyrrolyl or phenyl and g is 0 or 1;

ii) (aminomethylene)diphosphonic acids as disclosed in Japanese Patent 63150290 (Ciba-Geigy) having the structure

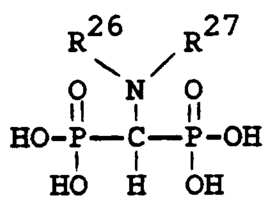

wherein $R^{26}$ is (benzo- or cyclohexeno-fused) 5-membered heteroaryl containing 2 to 4 N atoms or 1-2 N atoms plus an O or S atom with optional substituents such as alkyl or halo;

$R^{27}$ is H or alkyl, but $R^{27}$ is alkyl when $R^{26}$ is (alkyl and/or halo-substituted) pyrazol-3-yl or isoxazol-3-yl;

jj) alkylenediphosphonic acids as disclosed in Japanese Patent 63150291 (Ciba-Geigy) having the formula

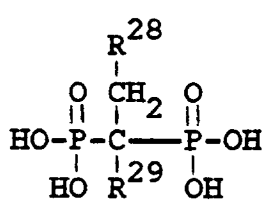

wherein $R^{28}$ is a 5-membered heteroaryl containing 2-4 N atoms or 1-2 N atoms plus an O or S atom

optionally substituted with alkyl or halo, and

$R^{29}$ is H, OH, $NH_2$, alkylthio or halo;

kk) azacycloalkylalkanediphosphonic acids as disclosed in EP 272208 (Ciba-Geigy) (disclosed for use as regulators of calcium metabolism) having the formula

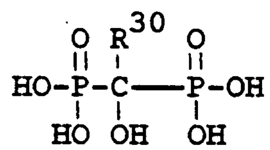

wherein $R^{30}$ is aromatic-substituted mono- or biazacyclylalkyl (alkyl bonds with N in heterocycle);

ll) 2-substituted-2-oxoethylene-1,1-diphosphonic acids as disclosed in Japanese Patent 63185993 (Yamanouchi Pharmaceutical Co.) (disclosed for use in bone disorders, anti-inflammatories) having the structure

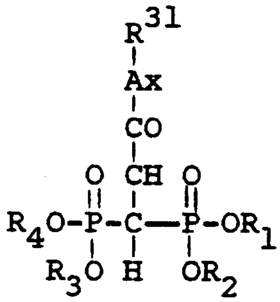

wherein $R_1$-$R_4$ is H or lower alkyl, Ax is phenyl, naphthyl, mono- or bicyclic N-containing heterocycles, and

$R^{31}$ is H, halo, lower alkoxy, lower alkyl;

mm) ureidoalkylbisphosphonic acids as disclosed in Japanese Patent 63088192 (Fujisawa Pharmaceutical Co.) (disclosed for use as bone absorption inhibitors) having the formula

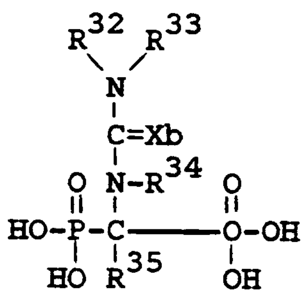

wherein $R^{32}$ is optionally substituted aryl, optionally substituted aralkyl, or optionally substituted alkyl,

$R^{33}$ is H or aryl,

$R^{34}$ and $R^{35}$ are H or alkyl, and

Xb is O or S;

nn) heterocyclicalkyl diphosphonic acids as disclosed in DE 3640938 (Boehringer Mannheim) (for use in treating calcium metabolic disorders) such as 1-(decahydroquinolin-3-yl)methane-1-hydroxy-1,1-diphosphonic acid, di Na salt;

oo) 1-aminoalkyl-1,1-bisphosphonic acids as disclosed in DD 222598 (Akademic der Wissenschaften) having the formula

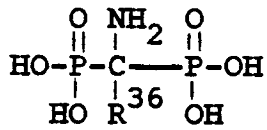

wherein $R^{36}$ is $C_2$-$C_{12}$ alkyl.

pp) methylenediphosphonic acids as disclosed in EP 151072 (Sanofi) (disclosed for use in anti-inflammatory and antiarthritic compositions) having the formula

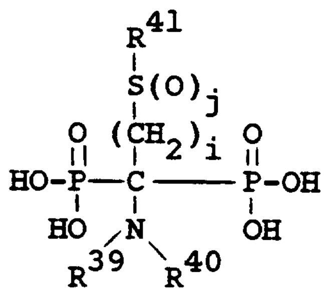

wherein $R^{39}$ is H, alkyl or $CONH_2$,

$R^{40}$ is H, alkyl, benzyl, optionally substituted phenyl (such as with Cl or $CH_3$), or $R^{39}$, $R^{40}$ are $(CH_2)_{4,5}$, j is 0, 1 or 2, i is 1 to 5,

$R^{41}$ is alkyl, cycloalkyl, optionally substituted phenyl or heterocyclyl;

qq) N-(unsubstituted or substituted pyridyl)amino-methylene diphosphonic acids as disclosed in U.S. Patent No. 4,447,256 (Nissan Chemical Industries) (disclosed for use as herbicides) having the formula

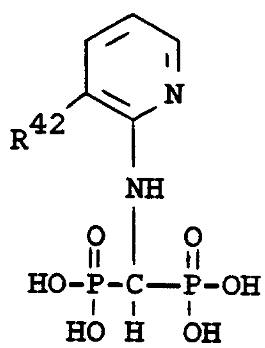

wherein $R^{42}$ is H, alkyl or halo;

rr) pyridylaminomethylenediphosphonates as disclosed in Japanese Patent 55089210 (Nissan Chemical Industries) (disclosed for use as herbicides) having the formula

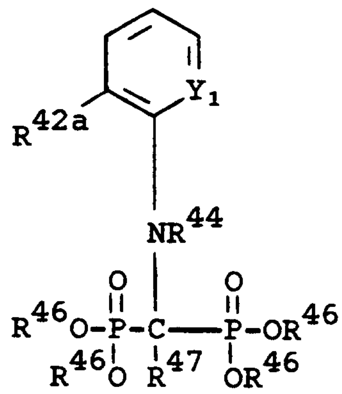

wherein $R^{46}$ is OH, halo, phenoxy or alkylamino,
$R^{47}$ is H or halo,
$R^{44}$ is H or aliphatic acyl (such as alkanoyl or alkenoyl),
$R^{42a}$ is alkyl, and
$Y_1$ is N, NO or $NR^{43}Y_2$, wherein $R^{43}$ is alkyl and $Y_2$ is halo;

ss) pyridylbisphosphonates as disclosed in Japanese Patent 55098105 (Nissan Chemical Industries) (disclosed for use as herbicides) having the formula

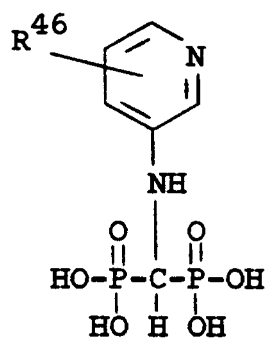

wherein $R^{46}$ is H, halo or alkyl;

tt) bisphosphonates as disclosed in Japanese Patent 55089293 (Nissan Chemical Industries) (disclosed for use as herbicides) having the formula

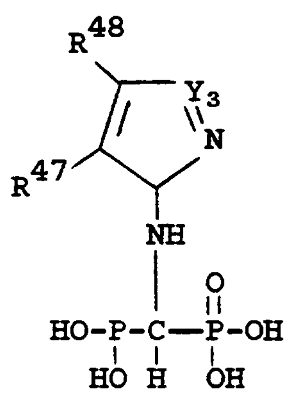

wherein $Y_3$ is O or NH, $R^4$ is H, alkyl or halo, and $R^{48}$ is H or alkyl;

uu) diphosphonic acids as disclosed in Japanese Patent 54144383 (Nissan Chemical Industries) (disclosed for use as herbicides) having the formula

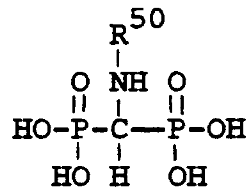

wherein $R^{50}$ is

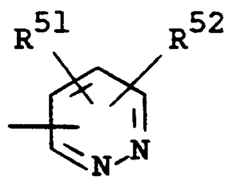

,

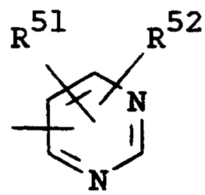

or

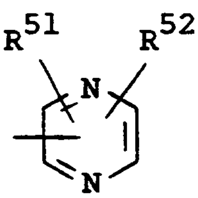

wherein $R^{51}$ and $R^{52}$ are H, halo, alkyl or hydroxy;

vv) diphosphonates as disclosed in Japanese Patent 54037829 (Nissan Chemical Industries) (disclosed for use as herbicides) having the formula

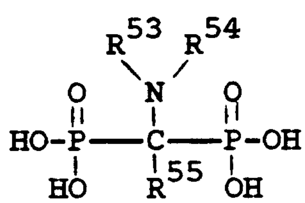

wherein $R^{53}$ is H, alkyl, alkenyl, benzyl or ethylcarbonyloxymethyl,

$R^{54}$ is H, alkyl, alkenyl or cyclohexyl,

$R^{55}$ is H, alkyl, alkenyl or ethylcarbonyloxymethyl;

ww) disphosphonates as disclosed in UK 1508772 and DE 2625767 (Benckiser-Knapsack) (Shell) having the formula

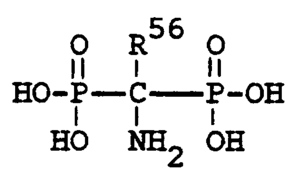

wherein $R^{56}$ is $C_5$-$C_{12}$ alkyl, aryl or aralkyl;

xx) 1-aminoalkylidene-1,1-diphosphonic acids as disclosed in DE 2115737 (Henkel) (disclosed for use as water softeners) having the formula

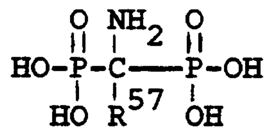

wherein $R^{57}$ is $CH_3$, propyl, n-heptyl, n-$C_{11}H_{23}$, n-$C_{15}H_{31}$, i-propyl;

yy) 1-amino-alkylidenediphosphonic acids as disclosed in DE 2048912 and DE 2048913 (Henkel) (disclosed for use as water softeners) having the formula

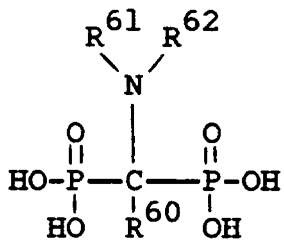

wherein $R^{60}$ is H, $CH_3$, n-nonyl, phenyl, benzyl or $CH_2CO_2H$, $R^{61}$ and $R^{62}$ are H or $CH_3$;

zz) 1-piperidinoalkane-1,1-diphosphonic acids as disclosed in Japanese Patent 53059674 (Nissan chemical Industries) (disclosed for use as herbicides) having the formula

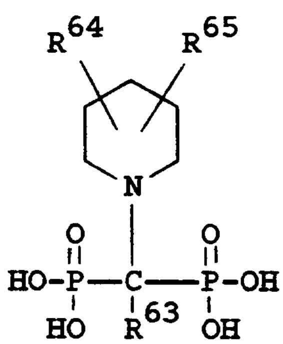

wherein $R^{63}$ is H or $CH_3$,

$R^{64}$ is alkyl, and

$R^{65}$ is H or alkyl;

aaa) heterocyclically substituted alkane-1,1-diphosphonic acids as disclosed in EP 170228A (Boehringer Mannheim) (disclosed for use as anti-inflammatory agents) having the structure

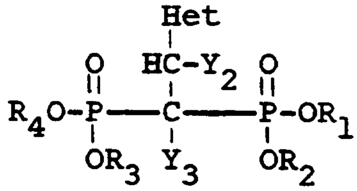

wherein Het is a heteroaromatic 5-membered ring with 2 or 3 heteroatoms, optionally partially hydrogenated and optionally substituted by one or more alkyl, alkoxy, phenyl, cyclohexyl, cyclohexylmethyl, halo or amino, with 2 adjacent alkyl optionally together forming a ring (Het cannot be pyrazole),

$Y_2$ is H or lower alkyl, and

$Y_3$ is H, OH, amino or alkylamino;

bbb) diphosphonic acids as disclosed in U.S. Patent No. 4,687,767 (Boehringer Mannheim) (disclosed for use in calcium metabolism disturbances) having the formula

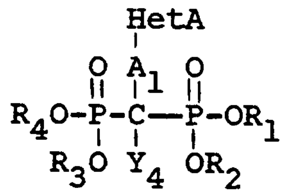

wherein HetA is imidazole, oxazole, isoxazole, thiazole, pyridine, 1,2,3-triazole, 1,2,4-triazole or benzimidazole, the above heterocyclics optionally substituted by alkyl, alkoxy, halo, OH, carboxyl, amino optionally substituted by alkyl or alkanoyl or a benzyl optionally substituted by alkyl, nitro, amino or aminoalkyl, $A_1$ is a straight-chained or branched saturated or unsaturated hydrocarbon chain containing 2 to 8 carbons, $Y_3$ is H, OH, alkanoyl;

bbb') diphosphonic acids as disclosed in U.S. Patent No. 4,666,895 (Boehringer Mannheim) (disclosed for use in calcium metabolism disturbances) having the formula

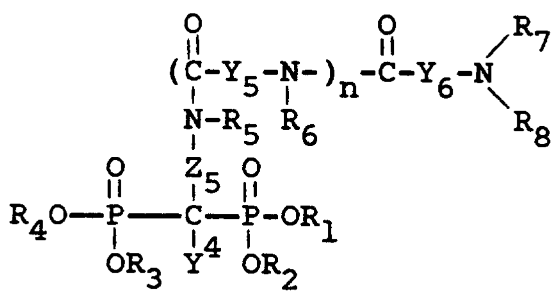

wherein $Y_4$ is H or OH,

$R_1$-$R_8$ are independently H or lower alkyl, whereby $R_7$ and $Y_6$, or $R_6$ and $Y_5$, or $R_5$ and $Z_5$, together with the nitrogen atoms to which they are attached can form a 5- or 6-membered ring,

$Y_6$ and $Y_5$ which can be the same or different are $C_1$-$C_6$ alkylene chains optionally substituted by aromatic or heteroaromatic radicals,

$Z_5$ is $C_1$ to $C_6$ alkylene which can include heteroatoms and optionally substituted by aromatic or heteroaromatic,

n is 0, 1 or 2;

ccc) omega amino-alkane-1,1-diphosphonic acids as disclosed in DE 623397 (Boehringer Mannheim) (disclosed for use in disorders of calcium metabolism) having the formula

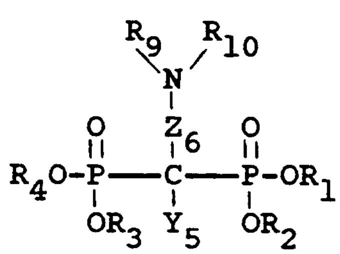

wherein $R_1$-$R_4$ are H or $C_1$-$C_4$ alkyl,

$Z_6$ is $C_1$-$C_6$ alkylene,

$R_9$ is saturated or unsaturated $C_1$-$C_9$ alkyl optionally substituted by phenyl or cyclohexyl,

$R_{10}$ is cyclohexyl, cyclohexylmethyl, benzyl or saturated or unsaturated $C_4$-$C_{18}$ alkyl optionally

substituted by phenyl or optionally esterified or etherified OH, and

$Y_5$ is H, OH, or mono- or di($C_1$-$C_6$alkyl)amino;

ddd) aminocycloalkane diphosphonates as disclosed in DE 3,540,150 (Boehringer Mannheim) (disclosed for use in calcium metabolic disorders) having the structure

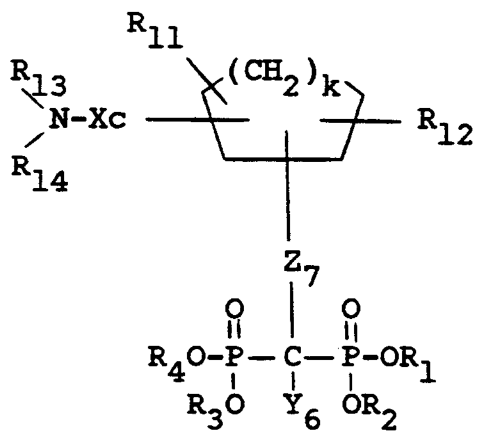

wherein $R_1$-$R_4$ are H or alkyl,

Xc is a bond or alkylene,

$R_{13}$, $R_{14}$ are H, acyl, alkyl or aralkyl,

$R_{11}$, $R_{12}$ are H, alkyl or

$R_{11}$, $R_{12}$ are together ($CH_2$),

$Z_7$ is a bond or (amino)alkylene,

$Y_6$ is H, OH, amino and k is 1 to 3;

eee) amino-oxa-alkane-diphosphonic acids as disclosed in DE 822650 (Boehringer Mannheim) (disclosed for use in calcium metabolism disorders) having the structure

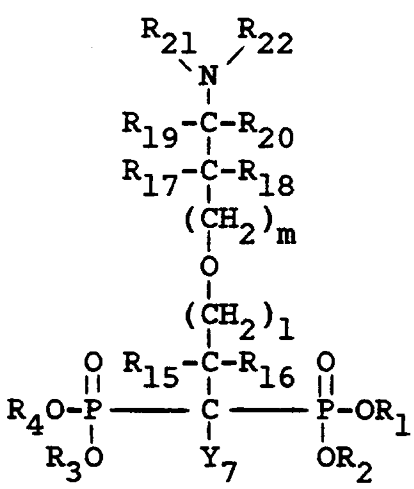

$R_{21}$ and $R_{22}$ are independently H, saturated or unsaturated 1-9C alkyl (optionally substituted by OH, 1-5C alkoxy, 1-5C alkylthio, Aryl or 5-7C cycloalkyl) 5-7C cycloalkyl or phenyl;

Aryl is phenyl optionally substituted by 1-5C alkyl, 1-5C alkoxy, OH or halogen;

$R_{19}$ is H, 1-5C alkyl (optionally substituted by OH, 1-5C alkoxy, 1-5C alkylthio, SH, phenyl, 3-indolyl or 4-imidazolyl), or phenyl optionally substituted by OH or 1-5C alkoxy;

$R_1$-$R_4$, $R_{20}$, $R_{18}$, $R_{16}$ are independently H or 1-5C alkyl;

$R_{15}$ and $R_{17}$ are independently H, 1-5C alkyl, or phenyl optionally substituted by OH or 1-5C alkoxy;

$Y_7$ is H, OH or $NR_{23}R_{24}$;

$R_{23}$ and $R_{24}$ are independently H or 1-5C alkyl;

m and l are independently 0 or 1;

or $NR_{21}R_{22}$ is a 4-9C mono- or bicyclic ring system which is partially or totally hydrogenated and is optionally substituted by OH, 1-5C alkyl or 1-5C alkoxy, where monocyclic rings may also contain an O, N or S atom;

or $R_{21} + R_{20}$ forms a 5- or 6-membered ring, optionally fused with another 6-membered ring;

or $R_{21} + R_{18}$ forms a 5- or 6-membered ring;

or $R_{20} + R_{19}$, $R_{19} + R_{18}$, $R_{18} + R_{17}$ and/or $R_{15} + R_{16}$ forms a 5- or 6-membered ring.

fff) diphosphonic acids as disclosed in DE 640938 (Boehringer Mannheim) (disclosed for use in calcium metabolism disorders) having the structure

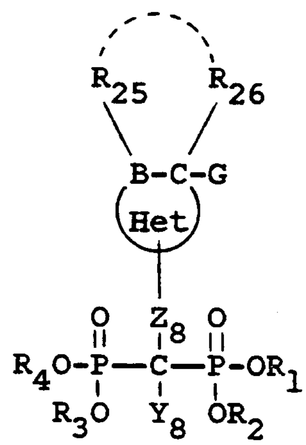

wherein Het is a hydrogenated or partially hydrogenated heterocycle containing 1 or 2 N atoms, each optionally substituted with alkyl, benzyl or cyclohexylmethyl;

B is N or CH and the bond connnecting B to the C atom to which G is attached can be a single or a double bond;

$R_{25}$ and $R_{26}$ are independently H or lower alkyl, or together form a 3-5C alkylene chain, and this ring fused with Het may contain up to 3 double bonds;

$R_1$ to $R_4$ are H or lower alkyl;

$Z_8$ is a single bond or optionally branched 1-6C alkylene which may not be attached to a heteroatom;

$Y_8$ is H, OH or amino;

G is H; and provided that, if $Y_8$ is a single bond, Het is not a pyrrolidine ring which is 2-substituted by $Y_8$;

ggg) methylenediphosphonic acids as disclosed in EP 243173A (Fujisawa Pharm KK) (disclosed for use in abnormal bone metabolism) having the formula

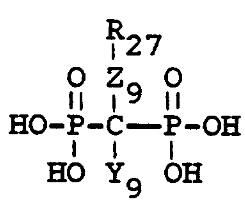

wherein $R_{27}$ is aryl or heterocyclyl both optionally substituted by one or more of lower alkyl, lower alkoxy, lower alkylthio, halo(lower)alkyl, acyl, acylamino or halo or $R_{27}$ is lower alkyl substituted by heterocyclyl which is optionally substituted by acyl;

$R_{27}$-$Z_9$- is $R_{27}$-NHC(=$X_9$)-; $R_{27}$-C(=O)NH-; or $R_{27}$-$SO_2$-NH-;

$X_9$ is O or S;

$Y_9$ is H or lower alkyl, provided that when $R_{27}$ is lower alkyl then $R_{27}$-$Z_9$- is $R_{27}$NHC(X)- or

$R_{27}SO_2NH$-;

hhh) diphosphonic acids as disclosed in Japanese Patent 261275 (Fujisawa Pharm) (disclosed for use in bone disorders) having the formula

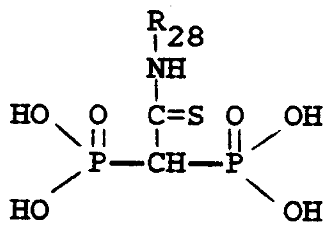

wherein $R_{28}$ is phenyl, pyridyl or quinolyl substituted by lower alkylsulphonylamino halo-lower alkylsulphonylamino and mono- or di-lower alkylamino;

hhh) diphosphonic acids as disclosed in Japanese Patent 259896 (Fujisawa Pharm) (disclosed for use as anti-inflammatory agents) having the formula

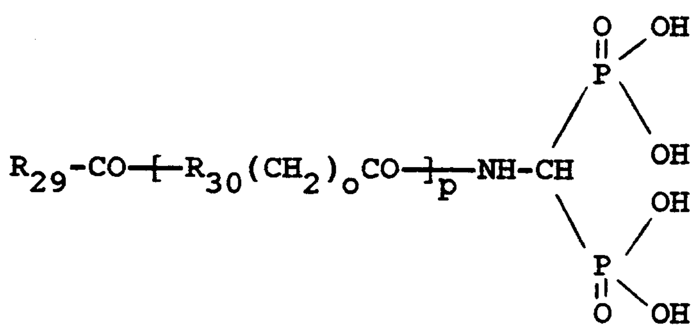

wherein $R_{29}$-CO- is a residue of pharmaceutically active compound $R_{29}$-COOH;

$R_{30}$ = -NH- or -O-;

p = 0 or 1;

o = 1-10;

$R_{29}$-COOH is an anti-inflammatory agent, e.g. diclofenac, ibuprofen, mefenamic acid, aspirin, naproxen, ketoprofen, indomethacin or sulindac; antioncotic, e.g. methotrexate; or hormone, e.g. calcitonin or insulin-like growth factor;

iii) N-aralkyl-amino-alkane-diphosphonic acids as disclosed in EP 371921A (Ciba-Geigy) (disclosed for use in calcium metabolism disorders) having the formula

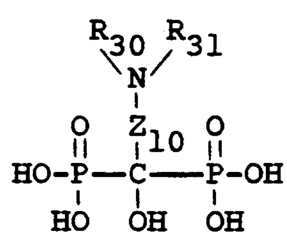

wherein $Z_{10}$ is $C_2$-$C_4$ aliphatic hydrocarbyl (e.g. alkylene),

$R_{30}$ is phenyl substituted $C_4$-$C_7$ aliphatic hydrocarbyl,

$R_{31}$ is $C_1$-$C_4$ aliphatic hydrocarbyl;

jjj) N-aralkylamino-1-hydroxyalkane-1,1-diphosphonic acids as disclosed in EP 320455A (Ciba-Geigy) (disclosed for use in calcium metabolism disorders) having the formula

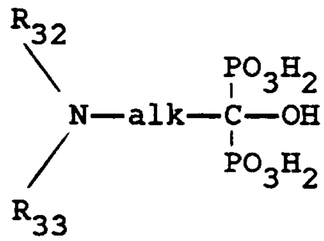

wherein $R_{32}$ ia an aromatically-substituted aliphatic group;

$R_{33}$ is H or monovalent aliphatic group;

alk is a divalent aliphatic group;

provided that when $R_{32}$ is mono-substituted by phenyl, $R_{33}$ is H or when $R_{32}$ has 2 or 3C in the aliphatic portion, $R_{33}$ is an aliphatic group with at most 3C;

kkk) azabicycloalkyl-1-hydroxyalkane-1,1-diphosphonic acids as disclosed in EP 317505A (Ciba-Geigy) (disclosed for use in calcium metabolism disorders) having the structure

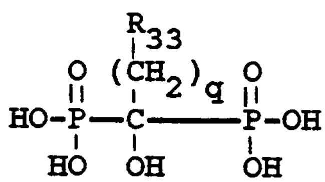

wherein $R_{33}$ is an N-bonded azabicycloalkyl group with 3 to 8-membered rings, such as 3-azabicyclo-(3,1,1,)hept-3-yl, 1,5-dimethyl-3-azabicyclo(3,1,1)-hept-3-yl, 3-azabicyclo(3,2,1)oct-3-yl, 3-azabicyclo-(3,2,2)non-3-yl or 3-azabicyclo(4,2,2)dec-3-yl, and $(CH_2)_q$ is lower alkylene (2 to 4 carbons);

lll) azacycloalkyl-substituted 1-hydroxyalkane-1,1-diphosphonic acids as disclosed in EP 272208A (Ciba-Geigy) (disclosed for use in calcium metabolism disorders) having the formula

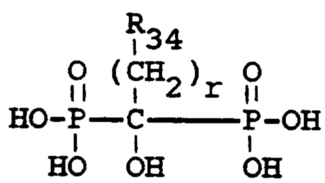

wherein $R_{34}$ is an N-bonded, aryl-substituted mono- or diazacycloaliphatic group, such as 3-$R_{35}$-pyrrolidino, 3- or 4-$R_{35}$-piperidino or 6-$R_{35}$-3-azabicyclo-(3,1,1)hept-3-yl, wherein $R_{35}$ is phenyl optionally substituted by $C_1$-$C_4$ alkyl, $C_1$-$C_4$ alkoxy or halogen;

mmm) heteroarylaminomethane diphosphonic acids as disclosed in EP 274346A (Ciba-Geigy) (disclosed for use in calcium metabolism disorders) having the formula

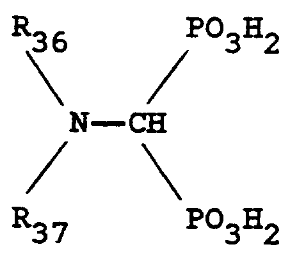

wherein $R_{36}$ is a 5 membered heteroaryl with 2-4 N or with 1-2 N plus an O or S atom, optionally fused to a benzo or cyclohexeno ring;

$R_{36}$ can be C substituted by lower alkyl, phenyl (optionally substituted by lower alkyl, alkoxy and/or halo), lower alkoxy, OH, di(lower alkyl)amino, lower alkylthio and/or halo, and/or N substituted by lower alkyl or phenyl (lower) alkyl (optionally substituted by lower alkyl, lower alkoxy and/or halo);

$R_{37}$ is H or lower alkyl; provided $R_{37}$ is not H if $R_{36}$ is optionally alkyl and/or halo substituted 3-pyrazolyl or 3-isoxazolyl;

examples of $R_{36}$ groups are 2-thiazolyl (optionally substituted by 1 or 2 1-4 C alkyl or phenyl); imidazol-2-yl or benzimidazol-2-yl (optionally 1-substituted by 1-4 C alkyl or phenyl(1-4 C) alkyl); or unsubstituted 2-benzoxazolyl or 2-benzothiazolyl;

nnn) N-substituted aminoalkanediphosphonic acids as disclosed in EP 387194A (Ciba-Geigy) (disclosed for use in calcium metabolism disorders) having the formula

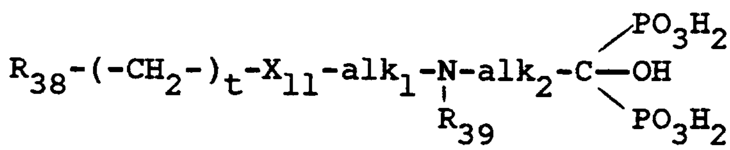

wherein $R_{38}$ is an aromatic residue (such as phenyl, pyridyl or pyrimidyl);

t is 0-3;

$X_{11}$ is O, S (optionally oxidized) or amino (optionally substituted by aliphatic group);

$alk_1$ and $alk_2$ are divalent aliphatic groups;

$R_{39}$ is H or monovalent aliphatic group;

ooo) 2-heteroarylethane-1,1-diphosphonic acids as disclosed in Australian Patent 8781453A (Ciba-Geigy) (disclosed for use in calcium metabolism disorders) having the formula

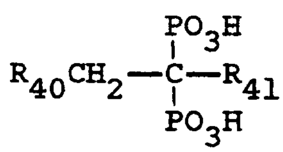

wherein $R_{40}$ is a 5 membered heteroaryl containing either 2-4N atoms or 1-2N atoms and an O or S atom, optionally (a) C-substituted by lower alkyl, aryl, lower alkoxy, OH, di(lower alkyl)amino, lower alkylthio and/or halogen and/or (b) N-substituted by lower alkyl or aryl(lower)alkyl, where aryl in (a) and (b) is phenyl optionally substituted by lower alkyl, lower alkoxy and/or halogen;

$R_{41}$ is H, OH, $NH_2$, lower alkylthio or halogen;

examples of $R_{40}$ groups include imidazolyl, 1,2,4-triazolyl or thiazolyl, optionally C-substituted and/or N-substituted;

ppp) 1-aminoalkane-1,1-diphosphonates as disclosed in U.S. Patent Nos. 3,846,420 and 3,979,385 (Henkel) (disclosed for use as metal ion complexing agents) having the formula

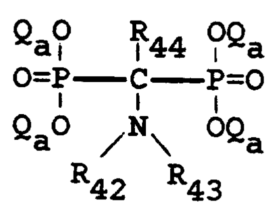

wherein $R_{44}$ is hydrogen, lower alkyl and phenyl; $R_{42}$ and $R_{43}$ are independently hydrogen, alkyl having one to 22 carbon atoms, cycloalkyl having five to six carbon atoms, phenyl, alkylphenyl having seven to 18 carbon atoms, phenylalkyl having seven to 18 carbon atoms and together with the nitrogen atom, piperidino, pyrrolidino and morpholino, and $Q_a$ is hydrogen, alkali metal, ammonium, pyridinium,

guanidinium and mono-, di-, and tri-lower-alkanol-ammonium, with the proviso that at least one of $R_{42}$, $R_{43}$ and $R_{44}$ is other than hydrogen;

qqq) 1-aminoalkane-1,1-diphosphonic acids as disclosed in U.S. Patent No. 3,899,496 (Henkel) having the formula

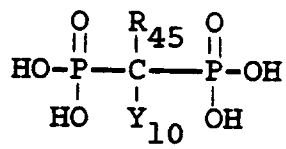

wherein $R_{45}$ represents a hydrogen atom or an alkyl residue with 1 to 12 carbon atoms, a phenyl group, a cyclohexyl group, a phenylalkyl group with 7-12 carbon atoms, a piperidinyl group, a carboxyalkyl group with 2-12 carbon atoms, or a carbalkoxy alkyl group with 3-12 carbon atoms; and

$Y_{10}$ represents an $NH_2$ group, a piperidino group, a morpholino group or a $NR_xR_y$ group, wherein $R_x$ and $R_y$ represent alkyl residues with 1 to 4 carbon atoms;

rrr) aminophosphonic acids as disclosed in U.S. Patent No. 3,303,139 (Henkel) (disclosed for use as metal ion complex formers) having the formula

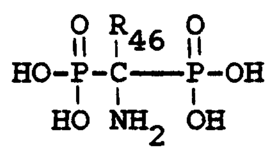

wherein $R_{46}$ is a saturated or unsaturated aliphatic radical having 1-10 carbon atoms or a phenyl- or benzyl-radical;

sss) 3-alkyl-3-oxo-1-amino-propane-1,1-diphosphonic acids as disclosed in DE 3611522A (Henkel) (disclosed for use in inhibiting growth of bacteria) having the formula

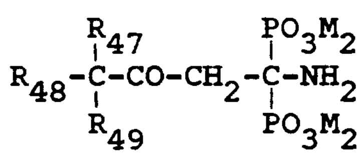

wherein $R_{47}$ is optionally branched 1-8C alkyl;

$R_{48}$ and $R_{49}$ are each methyl or ethyl;

M is H or a cation of a water-soluble base;

ttt) N-heterocyclic propylidene-1,1-bisphosphonic acids as disclosed in WO 89/09775 (PCT/DK89/00071) (Leo Pharmaceutical Products) (disclosed for use in calcium metabolism disorders) having the formula

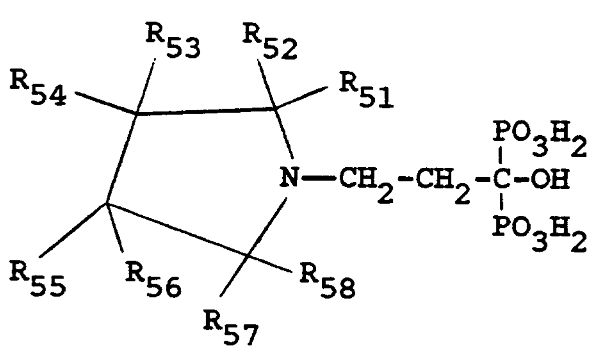

in which $R_{51}$-$R_{58}$ can be the same or different and stand for hydrogen or a straight or branched alphatic $C_1$-$C_{10}$ hydrocarbon radical; and

$R_{53}$ when taken together with either $R_{51}$ or $R_{55}$ can form a saturated aliphatic 5-, 6- or 7-membered ring, which may be substituted with one or more $C_1$-$C_4$-alkyl radicals;

uuu) thiomorpholinylmethylene-bisphosphonic acids as disclosed in WO 8703-598A (Leo Pharmaceuticals) (disclosed for use in reducing bone resorption and stimulating bone alkaline phosphatase) having the formula

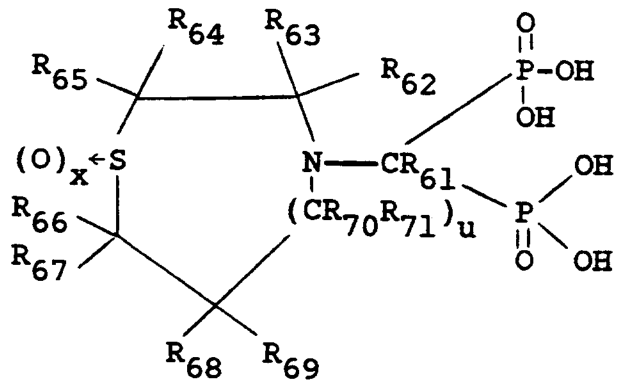

wherein $R_{61}$-$R_{71}$ are independently H, straight, branched or alicyclic 1-10C hydrocarbyl, aryl or aryl-(1-4C)alkyl;

x is 0 or 1;

u is 0, 1 or 2 or

$R_{62}$ + $R_{64}$ may complete a 5- to 7-membered saturated aliphatic ring, optionally substituted by one or more 1-4C alkyl groups;

vvv) 1-aminoalkane-1,1-diphosphonic acids as disclosed in U.S. Patent No. 4,100,167 (Nalco Chemical) (disclosed for use as anti-corrosives) having the formula

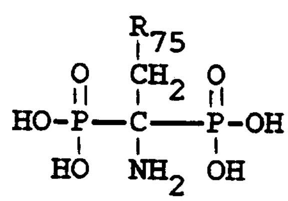

wherein $R_{75}$ is alkyl, aryl, arylalkyl (including phenyl substituted with a sulfonic acid group, halo or pyridyl);

www) heterocycle-substituted diphosphonic acids as disclosed in EP 274158A (Norwich Eaton) (disclosed for use in calcium or phosphate metabolism disorders and as antiplaque agents, herbicides) having the formula

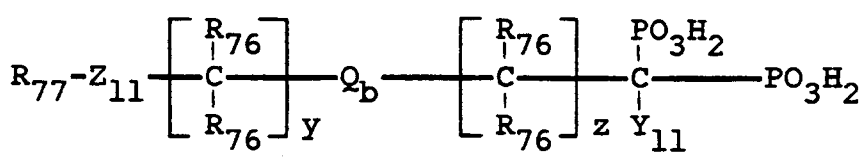

wherein $Z_{11}$ is a N-containing 6-membered ring heterocycle moiety selected from piperidinyl, diazinyl or triazinyl;

$Q_b$ is a covalent bond, O, S or $NR_{76}$;

y, x and y + x are integers of 0-10;

$Y_{11}$ is H, halogen, 1-6C alkyl, phenyl, benzyl, hydroxy or its ester derived from a 1-6C carboxylic

acid, unsubstituted amino or its amide derived from a 1-6C carboxylic acid, amino substituted with one alkyl having 1-6C or its amide derived from a 1-6C carboxylic acid, di(1-6C alkyl)amino, tri(1-6C alkyl)-ammonium, $CO_2H$ or its salts or esters derived from 1-6C alcohols, or its amide optionally substituted with one or two 1-6C alkyl groups, except when n = 0 and $Q_b$ = O, S or N, then $Y_{11}$ is H, 1-6C alkyl, phenyl, benzyl, or $Co_2H$ or its salts or the esters derived from 1-6C alcohols or its amide optionally substituted with one or two 1-6C alkyl groups;

$R_{76}$ is H, methyl, ethyl or propyl;

$R_{77}$ is one or more substituents selected from H, halogen, 1-3C alkyl, unsubstituted amino and its amide derived from a 1-3C carboxylic acid, mono(1-3C alkyl) amino and its amide derived a 1-3C carboxylic acid, di(1-3C alkyl)amino, tri(1-3C alkyl)ammonium, hydroxy or its ester derived from a 1-3C carboxylic acid, ether having 1-3C, $CO_2H$ and its salts and esters derived form 1-3C alcohols, its amide optionally substituted with one or two 1-3C alkyl groups and $NO_2$;

xxx) N-heterocyclyl thio alkane diphosphonic acids as disclosed in EP 230068A (Norwich Eaton) (disclosed for use in calcium and phosphate metabolism disorders) having the structure

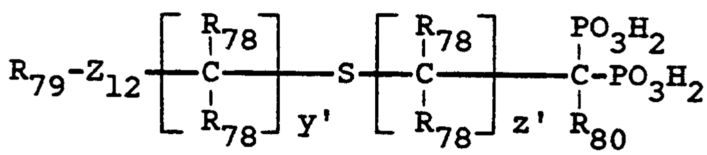

wherein $Z_{12}$ is a 6-membered aromatic ring containing ≧ 1 N atom(s); where:

the ring is optionally substituted by (optionally substituted- optionally unsaturated) 1-6C alkyl, (optionally substituted) aryl, (optionally substituted) benzyl, OH, halogen, carbonyl, alkoxy, $NO_2$, $CONH_2$, (optionally substituted) $NH_2$ and/or carboxylate;

$R_{78}$ is H or (optionally substituted, optionally unsaturated) 1-4C alkyl;

$R_{79}$ is H, (optionally substituted, optionally unsaturated) 1-6C alkyl, (optionally substituted) aryl, (optionally substituted) benzyl, OH, halogen, carbonyl, alkoxy, $NO_2$, $CONH_2$, (optionally substituted) amino or carboxylate;

$R_{80}$ is H, (optionally substituted) $NH_2$, $CONH_2$, OH, alkoxy, halogen, carboxylate, (optionally substituted, optionally unsaturated) 1-6C alkyl;

examples of $Z_{12}$ are pyridine, pyridazine, pyrimidine or pyrazine ring;

y' + z' is 0 to 5;

zzz) cyclic diphosphonic acids as disclosed in EP 304962A (Procter & Gamble) (disclosed for use in calcium and phosphate metabolism disorders) having the formula

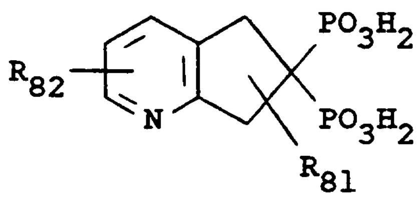

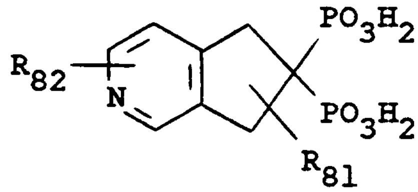

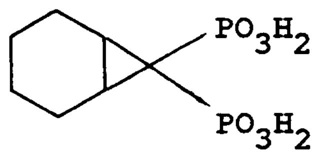

$R_{81}$ and $R_{82}$ are each one or more substituents selected from H, optionally substituted saturated or unsaturated 1-6C alkyl, optionally substituted aryl, optionally substituted benzyl, amimo, OH, halogen, optionally substituted amino, amido, COOH, carbonyl, carboxylate, alkoxy and $NO_2$;

$a^4$) heterocyclyl-alkane-diphosphonic acids as disclosed in Australian Patent 8551534A (Procter & Gamble) (disclosed for use in resorption of bone tissue) having the formula

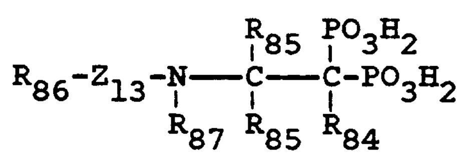

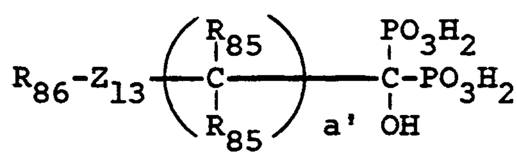

wherein $Z_{13}$ is pyridine, pyridazine, pyrimidine or pyrazine ring; this ring is optionally substituted by optionally unsaturated 1-6C alkyl, optionally substituted aryl, optionally substituted benzyl, OH, halogen, oxo, alkoxy, $NO_2$, amido, optionally substituted $NH_2$ or carboxylate;

$R_{84}$ is H, optionally substituted $NH_2$, amido, OH, alkoxy, halogen, carboxylate, optinally substituted optionally unsaturated 1-6C alkyl, optionally substituted aryl or optionally substituted benzyl;

$R_{85}$ is H or optionally substituted optionally unsaturated 1-4C alkyl;

$R_{86}$ is one or more of H, optionally substituted optionally unsaturated 1-6C alkyl, optionally substituted aryl, optionally substituted benzyl, OH, halogen, oxo, alkoxy, $NO_2$, amido, optionally substituted $NH_2$ or carboxylate;

$R_{87}$ is H, optionally substituted optionally unsaturated 1-4C alkyl or acyl;

a' is 1-5;

$b^4$) geminal diphosphonates as disclosed in EP 186405A (Procter & Gamble) (disclosed for use in calcium and phosphate metabolism disorders) having the strucuture

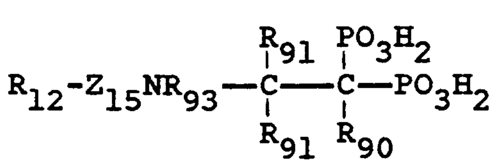

or

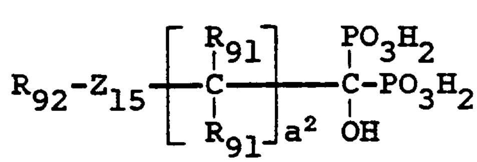

wherein $Z_{15}$ is a 6 membered aromatic ring containing 1 or more N atoms such as pyridine, pyridazine, pyrimidine or pyrazine which ring may be substituted with one or more optionally substituted, optionally unsaturated 1-6C alkyl, optionally substituted aryl, optionally substituted benzyl, OH, halogen, carbonyl, alkoxy, $NO_2$, amido, optionally substituted amino or carboxylate;

$R_{90}$ is H, optionally substituted amino, amido, OH, alkoxy, halogen, carboxylate, optionally substituted, optionally unsaturated 1-6C alkyl, optionally substituted aryl or optionally substituted benzyl;

$R_{91}$ is H or optionally substituted optionally unsaturated 1-4C alkyl;

$R_{92}$ is H or one or more substituents selected from optionally substituted, optionally unsaturated 1-6C alkyl, optionally substituted aryl, optionally substituted benzyl, OH, halogen, carbonyl, alkoxy, $NO_2$, amido, optionally substituted amino or carboxylate;

$R_{93}$ is H, optionally substituted, optionally unsaturated 1-4C alkyl or acyl;

$a^2$ is 1 to 5;

$c^4$) diphosphonic acid derivatives as disclosed in U.S. Patent No. 4,503,049 (Schering A.G.) (disclosed for use as anti-inflammatory agents) having the structure

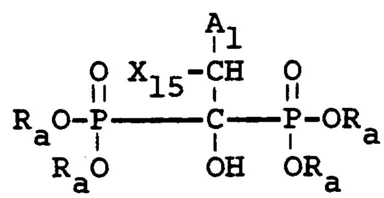

wherein $R_a$ is hydrogen, an alkali metal atom, an alkaline earth metal atom, or alkyl of 1-4 carbon atoms,

$X_{15}$ is hydrogen, methyl, or ethyl, and

$A_1$ is phenyl substituted in the para-position by isobutyl, cyclohexyl, alkoxy, or 1-pyrrolinyl and, optionally substituted additionally in the meta-position by fluorine or chlorine, or phenyl substituted in the meta-position by benzoyl or phenoxy, or phenyl substituted in the ortho-position by 2,4-dichlorophenoxy or 2,6-dichlorophenylamino;

diphosphonic acid derivatives of the sturcture

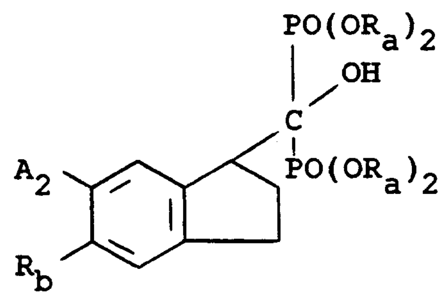

wherein $R_a$ is as defined above;

$R_b$ is cyclohexyl or cyclopentylmethyl; and

$A_2$ is hydrogen or chlorine;

diphosphonic acid derivatives of the formula

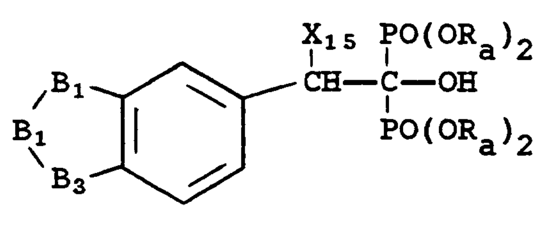

wherein $R_a$ and $X_{15}$ are as defined above, and

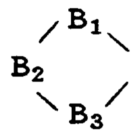

is

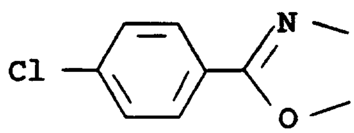 ; 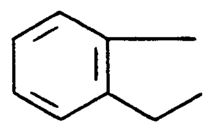

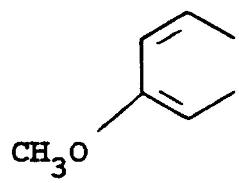 or 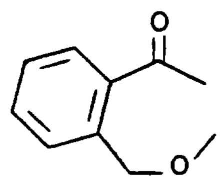 ;

diphosphonic acid derivatives of the formula

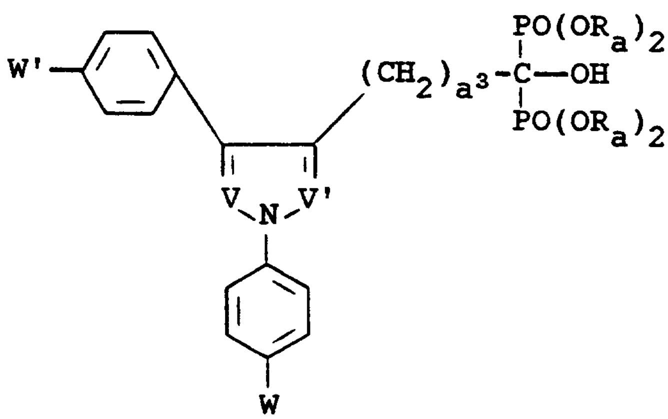

wherein $a^3$ is 1, 2 or 3,

$R_a$ is as defined above,

W and W' are identical or different, and each is hydrogen, fluorine or chlorine, and

one of V and V' is nitrogen and the other is a methyne residue optionally substituted by a phenyl group; and diphosphonic acid derivatives of the formula

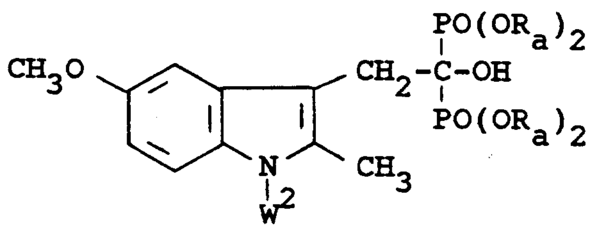

wherein $R_a$ is as defined above, and

$W^2$ is p-chlorobenzoyl or cinnamoyl; or throughout, when $R_a$ is H, a physiologically acceptable salt thereof with an organic base;

$d^4$) methylenediphosphonic acids as disclosed in U.S. Patent No. 4,876,247 (Sanofi) (disclosed for use as an antirheumatic) having the formula

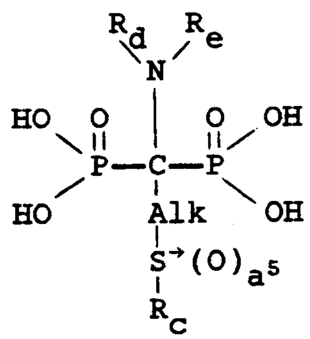

wherein $R_c$ represents:

a $C_1$-$C_6$ alkyl group,

a $C_5$-$C_7$ cycloalkyl group,

a phenyl group optionally monosubstituted or poly-substituted by a halogen, a $C_1$-$C_6$ alkyl group or a trifluoromethyl group, or

a 5-membered or 6-membered heterocycle containing 1 or 2 heteroatoms chosen from nitrogen and sulfur,

Alk denotes a linear or branched $C_1$-$C_6$ alkylene group,

$R_d$ represents hydrogen, a $C_1$-$C_6$ alkyl group or a $-CONH_2$ group,

$R_e$ represents hydrogen, a $C_1$-$C_6$ alkyl group, a benzyl group or a phenyl group optionally substituted by chlorine or methyl groups; or alternatively

$R_d$ and $R_e$ taken together, represent a $(CH_2)a^4$ group, in which $a^4$ is 4 or 5, and

$a^5$ represents 0 or the integer 1 or 2.

$e^4$) aminomethylene-bisphosphonic acids as disclosed in EP 337706A (Yamanouchi Pharm) (disclosed for use in inhibiting bone resportion, and for its anti-inflammatory, antirheumatic and analgesic activities) having the structure

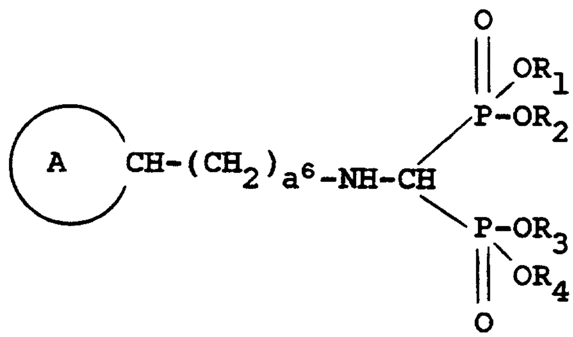

wherein $R_1$-$R_4$ is H or 1-5C alkyl;

$a^6$ is 0-4;

Ring A is 5-8-cycloalkenyl, bicycloheptyl, bicycloheptenyl or 4-7C saturated heterocyclyl containing O, S, SO or $SO_2$;

$f^4$) diphenylazolediphosphonic acids as disclosed in Japanese Patent 210445 (Yamanouchi Pharm) (disclosed for use as anti-inflammatory, antipyretic and analgesic agents) having the structure

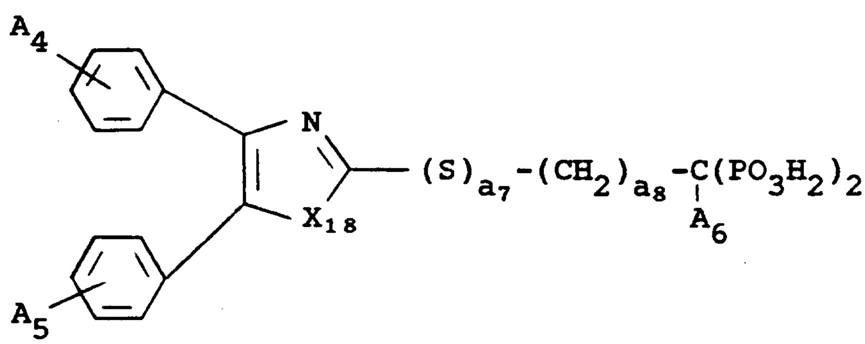

wherein $A_4$ and $A_5$ are the same or different and are H, OH, lower alkoxy or halogen;

$A_5$ is H or OH:

$X_{18}$ is O, S or NH;

$a_7$ is 0 or 1;

$A_5$ is 0 or an integer of 1-6;

$g^4$) (pyrazolylamino)methylene-bis-(phosphonic acids) as disclosed in Japanese Patent 086857 (Yamanouchi Pharm) (disclosed for use as bone resorption inhibitors) having the formula

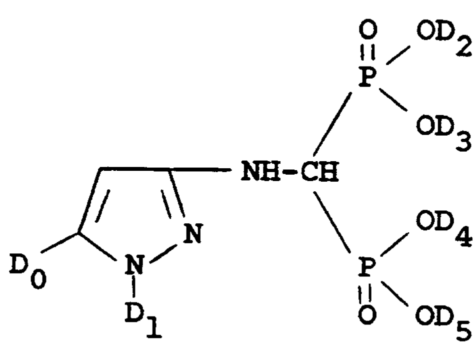

(wherein $D_0$ is H or alkyl;

$D_1$ to $D_5$ is H or lower alkyl);

$h^4$) isoxazolyl-containing bisphosphonic acids as disclosed in EP 282320A (Yamanouchi Pharm) (disclosed for use as bone resorption inhibitors and in arthritis) having the formula

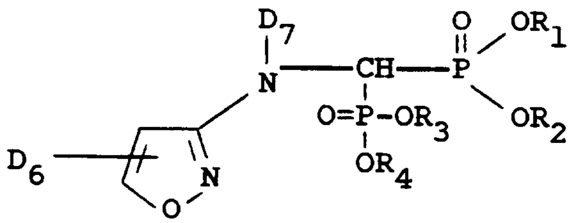

wherein $D_6$ is H, 1-10C alkyl, 3-10C cycloalkyl, phenyl, 2-10C alkenyl (optionally substituted by phenyl) or phenyl(1-5C)alkyl (optionally ring-substituted by a 1-5C alkoxy);

$D_7$ is H or 2-6C alkanoyl, and

$R_1$-$R_4$ is H or 1-5C alkyl;

provided that when $D_6$ is methyl, ethyl, isopropyl or tert-butyl, at least one of $D_7$, $R_1$, $R_2$, $R_3$ or $R_4$ is other than H;

$i^4$) azole-amino methylene bisphosphonic acids as disclosed in EP 282309A (Yamanouchi Pharm) (disclosed for use as bone resorption inhibitors) having the sturcture

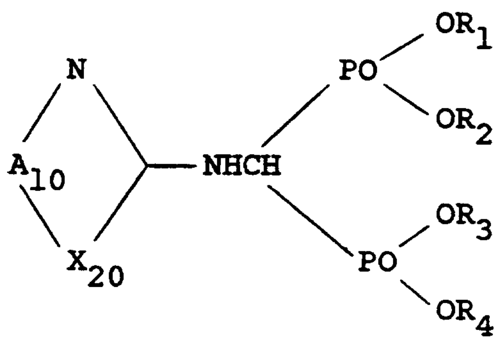

wherein $A_{10}$ = a group of formula (a)-(C):

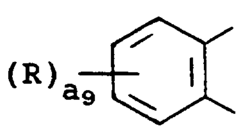

(a)

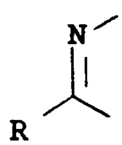

(b)

or

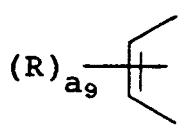

(c)

R is H, halogen, 1-5C alkyl or phenyl;

$a_9$ is 1 or 2;

$X_{20}$ is O, S or NH;

$R_1$-$R_4$ is H or 1-5C alkyl;

j[4]) cycloalkyl:amino-methylene-bis:phosphonic acids having the formula

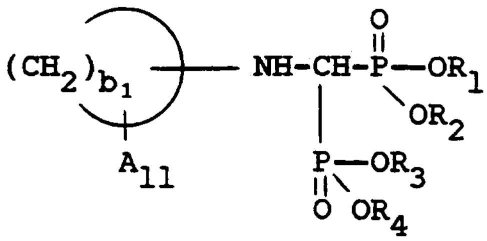

wherein $A_{11}$ and $R_1$ - $R_4$ are H or 1-5C alkyl;

$b_1$ is 3-10; provided that $A_{11}$ is 1-5C alkyl when $R_1$ - $R_4$ is H and $b_1$ is 5 or 6;

k[4]) heterocyclic bisphosphonic acids as disclosed in EP 354806A (Yamanouchi Pharm) (disclosed for use in bone resorption) having the formula

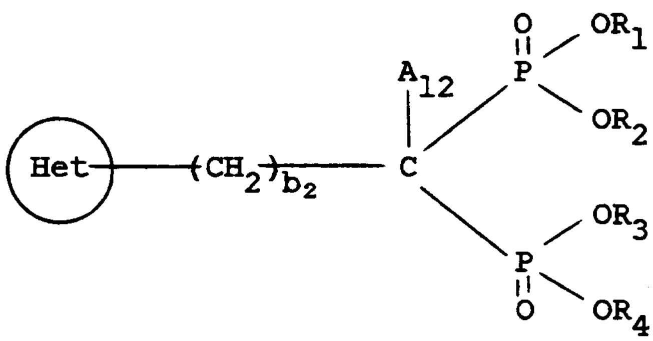

wherein ring Het is a group of formula (A) or (B):

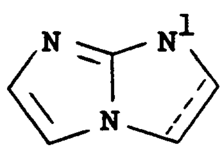

(A)

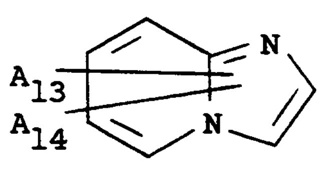

(B)

the dotted line represents optionally double bond;

$A_{13}$, $A_{14}$ are independently H, 1-5C alkyl, halogen or OH;

$A_{12}$ is H or OH;

$R_1$, $R_2$, $R_3$, $R_4$ are H or 1-5C alkyl;

$b_2$ is 0 or 1; provided that $b_2$ is 1 when ring Het is (A); and

$A_{12}$ is OH when ring Het is (B).

l[4]) imidazo- or pyrrolo-pyridine substituted bisphosphonic acids as disclosed in Japanese Patent 200462 (Yamanouchi Pharm) (disclosed for use as bone resorption inhibitors) having the structure

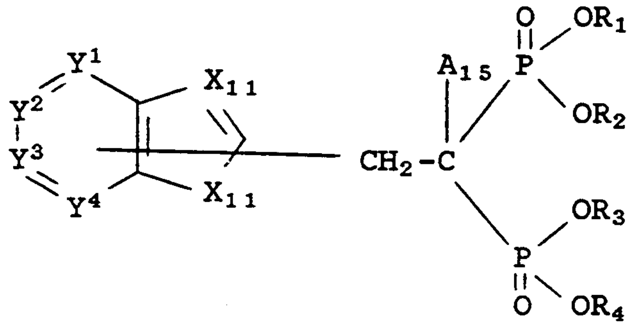

wherein $A_{15}$ is H or OH;

$R_1$-$R_4$ is H or lower alkyl;

$X_{11}$ is both N or one is N and the other is CH;

one of $Y^1$-$Y^4$ is N and the rest are CH.

The methylene phosphonoalkylphosphinate esters also referred to as a phosphonomethylphosphinate and/or salt thereof as disclosed in Application No. 92108073.5 suitable for use herein are described in European Patent Application 0298553A1 (Norwich Eaton Pharmaceuticals, Inc.), published January 11, 1989, (hereinafter referred to as EP 0298553).

The EP 0298553 compounds useful in the method of the invention are methylene phosphonoalkylphosphinic acids, and the pharmaceutically acceptable salts and esters thereof, having the general structure:

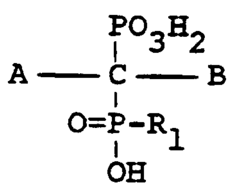

(1)

wherein $R_1$ is selected from hydrogen, substituted alkyl and unsubstituted alkyl. A and B are independent substituent moieties, at least one of which is a lipophilic group.

The term "lipophilic group" is as defined hereinbefore.

The term "alkyl" as used herein, unless otherwise specified, means chemically-stable carbon-containing chains which may be straight, branched, or cyclic; and further which may be saturated, monounsaturated (e.g., one double bond; one triple bond), or polyunsaturated (e.g. two double bonds; two triple bonds; three double bonds; one double and one triple bond). Preferred alkyl have from 1 to 20 carbon atoms. "Cycloalkyls" as used herein, having from 3 to 10 carbon atoms are preferred. Also preferred are straight chain alkyl, saturated alkyl or monounsaturated alkyl.

Alkyl is preferably unsubstituted but may be substituted. Preferred substituent groups for alkyl are as follows: halogen, nitro, cyano, heterocycle, aryl, heteroaryl, unsubstituted amino, and the amide thereof derived from a carboxylic acid of an alkyl, heterocycle, aryl or heteroaryl group, amino substituted with one alkyl, heterocycle, aryl or heteroaryl group and the amide thereof derived from a carboxylic acid of an alkyl group, amino substituted independently with one alkyl group and one alkyl, heterocycle, aryl or heteroaryl group, hydroxy, and the ester thereof derived from a carboxylic acid of an alkyl, heterocycle, aryl or heteroaryl group; ether having an alkyl, heterocycle, aryl or heteroaryl group; thiol, and the thiol ester thereof derived from a carboxylic acid of an alkyl, heterocycle, aryl or heteroaryl group; thioether having an alkyl, heterocycle, aryl or heteroaryl group, and the sulfoxide and sulfone derivatives thereof, $-SO_3H$, the pharmaceutically acceptable salts therof, the ester thereof derived from an alcohol of an alkyl group, the unsubstituted amide thereof, and the amide thereof substituted with one or two alkyl groups, $-CO_2H$, the pharmaceutically acceptable salts thereof, the ester thereof derived from an alcohol of an alkyl group, the unsubstituted amide thereof, and the amide thereof substituted with one or two alkyl groups, $PO_3H_2$, the pharmaceutically acceptable salts thereof, the ester thereof derived from an alcohol of an alkyl group, the unsubstituted amide thereof, and the amide thereof substituted with one or two alkyl groups, $-(R^8)PO_2H$

(where $R^8$ is hydrogen or unsubstituted lower alkyl), the pharmaceutically acceptable salts thereof, the ester thereof derived from an alcohol of an alkyl group, the unsubstituted amide thereof, and the amide thereof substituted with one or two alkyl groups, aldehyde, ketone having an alkyl group, carbamate, unsubstituted or substituted with one or two alkyl groups, peptidyl, and combinations thereof.

The term "lower alkyl" as used herein, unless otherwise specified, means unsubstituted alkyl having from 1 to 6 carbon atoms which may be saturated or unsaturated. Preferred lower alkyl are saturated and have from one to 4 carbon atoms. For lower alkyl groups specified herein as substituted, preferred substituents are the same as for alkyl hereinabove.

The term "heterocycle" as used herein, unless otherwise specified, means chemically-stable non-aromatic rings, including fused non-aromatic rings, having from 5 to 20 atoms, comprising at least one heteroatom selected from nitrogen, sulfur, phosphorus and oxygen. Preferred are 5 and 6 membered ring heterocycles which comprise from 1 to 3 heteroatoms. More preferred are 5 and 6 membered ring heterocycles which comprise one or two heteroatoms (especially nitrogen heteroatoms). Most preferred are the 6 membered ring heterocycles comprising one nitrogen atom, especially piperidinyl and piperidinylidene heterocycles. Heterocycles may be unsubstituted or substituted, saturated or unsaturated. Preferred heterocycles are unsubstituted or substituted with alkyl; halogen; nitro; cyano; heterocycle; aryl; heteroaryl; unsubstituted amino, and the amide thereof derived from a carboxylic acid of an alkyl heterocycle, aryl or heteroaryl group; amino substituted with one alkyl, heterocycle, aryl or heteroaryl group and the amide thereof derived from a carboxylic acid of an alkyl group; amino substituted independently with one alkyl group and one alkyl, heterocycle, aryl or heteroaryl group; hydroxy, and the ester thereof derived from a carboxylic acid of an alkyl, heterocycle, aryl or heteroaryl group; ether having an alkyl, heterocycle, aryl or heteroaryl group; thio, and the thiol ester thereof derived from a carboxylic acid of an alkyl, heterocycle, aryl or heteroaryl group; thioether having an alkyl, heterocycle, aryl or heteroaryl group, and the sulfoxide and sulfone derivatives thereof; $-SO_3H$, the pharmaceutically acceptable salts thereof, the ester thereof derived from an alcohol of an alkyl group, the unsubstituted amide thereof, and the amide thereof substituted with one or two alkyl groups; $-CO_2H$, the pharmaceutically acceptable salts thereof, the ester thereof derived from an alcohol of an alkyl group, the unsubstituted amide thereof, and the amide thereof substituted with one or two alkyl groups; $PO_3H_2$, the pharmaceutically acceptable salts thereof, the ester thereof derived from an alcohol of an alkyl group, the unsubstituted amide thereof, and the amide thereof substituted with one or two alkyl groups; $-(R^8)PO_2H$ (where $R^8$ is hydrogen or unsubstituted lower alkyl), the pharmaceutically acceptable salts thereof, the ester thereof derived from an alcohol of an alkyl group, the unsubstituted amide thereof, and the amide thereof substituted with one or two alkyl groups; aldehyde; ketone having an alkyl group; carbamate, unsubstituted or substituted with one or two alkyl groups; peptidyl, and combinations thereof.

The term "aryl", as used herein, unless otherwise specified, mean chemically-stable aromatic rings, including fused aromatic rings, having from 6 to 20 carbon atoms. Preferred aryl are phenyl or naphthyl, most preferred is phenyl. Aryls may be unsubstituted or substituted. Preferred aryls are unsubstituted or substituted with alkyl; halogen; nitro; cyano; heterocycle; aryl; heteroaryl, unsubstituted amino, and the amide thereof derived from a carboxylic acid of an alkyl, heterocycle, aryl or heteroaryl group; amino substituted with one alkyl, heterocycle, aryl or heteroaryl group and the amide thereof derived from a caroxylic acid of an alkyl group; amino substituted independently with one alkyl group and one alkyl, heterocycle, aryl or heteroaryl group; hydroxy, and the ester thereof derived from a carboxylic acid of an alkyl, heterocycle, aryl or heteroaryl group; ether having an alkyl, heterocycle, aryl or heteroaryl group; thiol, and the thiol ester thereof derived from a carboxylic acid of an alkyl, heterocycle, aryl or heteroaryl group; thioether having an alkyl, heterocycle, aryl or heteroaryl group, and the sulfoxide and sulfone derivatives thereof; $-SO_3H$, the pharmaceutically acceptable salts thereof, the ester thereof derived from an alcohol of an alkyl group, the unsubstituted amide thereof, and the amide thereof substituted with one or two alkyl groups; $-CO_2H$, the pharmaceutically acceptable salts thereof, the ester thereof derived from an alcohol of an alkyl group, the unsubstituted amide thereof, and the amide thereof substituted with one or two alkyl groups; $PO_3H_2$, the pharmaceutically acceptable salts thereof, the ester thereof derived from an alcohol of an alkyl group, the unsubstituted amide thereof, and the amide thereof substituted with one or two alkyl groups; $-(R^8)PO_2H$ (where $R^8$ is hydrogen or unsubstituted lower alkyl), the pharmaceutically acceptable salts thereof, the ester thereof derived from an alcohol of an alkyl group, the unsubstituted amide thereof, and the amide thereof substituted with one or two alkyl groups; aldehyde; ketone having an alkyl group; carbamate, unsubstituted or substituted with one or two alkyl groups; peptidyl; and combinations thereof.

The term "heteroaryl", as used herein, unless otherwise specified, means chemically-stable aromatic rings, including fused aromatic rings and fused aromatic and non-aromatic rings, having from about 5 to about 20 atoms, comprising at least one heteroatom selected from nitrogen, sulfur, phosphorus and oxygen.

Preferred are 5 and 6 membered ring heteroaryls which comprise from 1 to 3 heteroatoms. More preferred are 5 and 6 membered ring heteroaryls which comprise one or two heteroatoms (especially nitrogen heteroatoms). Most preferred heteroaryl is pyridinyl. Heteroaryls may be unsubstituted or substituted. Preferred heteroaryls are unsubstituted or substituted with alkyl; halogen; nitro; cyano; heterocycle; aryl; heteroaryl; unsubstituted amino, and the amide thereof derived from a carboxylic acid of an alkyl, heterocycle, aryl or heteroaryl group; amino substituted with one alkyl, heterocycle, aryl or heteroaryl group and the amide thereof derived from a carboxylic acid of an alkyl group; amino substituted independently with one alkyl group and one alkyl heterocyle, aryl or heteroaryl group; hydroxy, and the ester thereof derived from a carboxylic acid of an alkyl heterocycle, aryl or heteroaryl group; ether having an alkyl, heterocycle, aryl or heteroaryl group; thiol, and the thiol ester thereof derived from a carboxylic acid of an alkyl, heterocycle, aryl or heteroaryl group; thioether having an alkyl, heterocycle, aryl or heteroaryl group, and the sulfoxide and sulfone derivatives thereof; $-SO_3H$, the pharmaceutically acceptable salts thereof, the ester thereof derived from an alcohol of an alkyl group, the unsubstituted amide thereof, and the amide thereof substituted with one or two alkyl groups; $-CO_2H$, the pharmaceutically acceptable salts thereof, the ester thereof derived from an alcohol or an alkyl group, the unsubstituted amide thereof, and the amide thereof substituted with one or two alkyl groups; $PO_3H_2$, the pharmaceutically acceptable salts thereof, the ester thereof derived from an alcohol of an alkyl group, the unsubstituted amide thereof, and the amide thereof substituted with one or two alkyl groups; $-(R^8)PO_2H$ (where $R^8$ is hydrogen or unsubstituted lower alkyl), the pharmaceutically acceptable salts thereof, the ester thereof derived from an alcohol of an alkyl group, the unsubstituted amide thereof, and the amide thereof substituted with one or two alkyl groups; aldehyde; ketone having an alkyl group; carbamate, unsubstituted or substituted with one or two alkyl groups; peptidyl; and combinations threreof.

The term "substituent group", as used herein, means hydrogen or an alkyl, heterocycle, aryl or heteroaryl group, unless otherwise specified.

$R_1$ is a moiety selected from hydrogen, and alkyl. Preferred $R_1$ is unsubstituted alkyl, especially lower alkyl. Preferred substituents on the $R_1$ alkyl, when substituted, include halogen, alkoxy, unsubstituted and substituted phenyl, hydroxy, carboxy, and chemically-stable combinations thereof.

A is a moiety selected from the group consisting of hydrogen; halogen; nitro; alkyl; heterocycle; aryl; heteroaryl; unsubstituted amino, and the amide thereof derived from a carboxylic acid of a substituent group; amino substituted with one substituent group, and the amide thereof derived from a carboxylic acid of a substituent group; amino substituted independently with one alkyl group and one substituent group; hydroxy, and the ester thereof derived from a carboxylic acid of a substituent group; ether having a substituent group; thiol, and the thiol ester thereof derived from a carboxylic acid of a substituent group; thioether having a substituent group, and the sulfoxide and sulfone derivative thereof; $-SO_3H$, the pharmaceutically acceptable salts thereof, the ester thereof derived from an alcohol of a substituent group, the unsubstituted amide thereof, and the amide thereof substituted with one or two alkyl groups; $-CO_2H$, the pharmaceutically acceptable salts thereof, the ester thereof derived from an alcohol of a substitutent group, the unsubstituted amide thereof, and the amide thereof substituted with one or two alkyl groups; aldehyde; ketone having a substituent group; carbamate, unsubstituted or substituted with one or two alkyl groups; pepetides having from about one to 100 amino acid moieties; or the A and B moieties are covalently linked to form a ring having from 3 to 7 atoms with from 0 to 3 heteroatoms selected from the group consisting of nitrogen, sulfur, phosphorus and oxygen, the ring being unsubstituted or substituted with one or more of the above substituents of A; or the A and B moieties are replaced by an unsubstituted or substituted alkyl moiety attached to the geminal carbon by a double bond.

Examples of A moieties include

(1) hydrogen;

(2) halogen; more preferred are F or Cl;

(3) substituted and unsubstituted alkyl having the general structure:

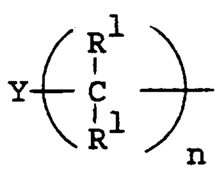

(2)

wherein n is an integer from 1 to 10, preferably from 1 to 5, more preferably n = 1 or 2, and most

preferably n = 1; each $R^1$ is independently selected to achieve chemically-stable moieties from the group consisting of hydrogen, halogen, lower alkyl, unsubstituted amino or the amido thereof derived from a carboxylic acid of a lower alkyl group, amino substituted with one lower alkyl group or the amide thereof derived from a carboxylic acid of a lower alkyl group, amino substituted independently with two lower alkyl groups, hydroxy or the ester thereof derived from a carboxylic acid of a lower alkyl group, $-CO_2H$ or the pharmaceutically acceptable salts thereof or the ester thereof derived from an alcohol of a lower alkyl group or the unsubstituted amide thereof or the amide thereof substituted with one or two lower alkyl groups, ether having a lower alkyl group, $-PO_3H_2$ or the pharmaceutically acceptable salts thereof, and nitro, or two $R^1$'s on the same carbon atom are =O or $=NR^9$ (where $R^9$ is lower alkyl or may be hydrogen when there is another nitrogen atom attached to the same carbon atom as the $=NR^9$ moiety), or two $R^1$'s on adjacent carbon atoms may be replaced by an additional bond between the carbon atoms; or an $R^1$ on the first carbon atom (from the right side of structure (2) hereinabove) and B (see structure (1) hereinabove) may be replaced by an additional bond; and Y is a substituent of alkyl as defined hereinbefore; (for the sake of chemical stability of the compounds used in the present invention, $R^1$ cannot be such that there is a halogen and an oxygen or sulfur or nitrogen singly bonded to the same carbon atom or such that two of an oxygen or sulfur or nitrogen are singly bonded to the same carbon atom);

(4) cycloalkyl having from 4 to 10 carbon atoms; more preferred are cycloalkyl having 5 or 6 carbon atoms;

(5) heterocycle having 5 or 6 atoms in the ring; more preferred are heterocycles having one or two nitrogen atoms in the ring, more preferred still are heterocycles having one nitrogen atom in the ring; most preferred are unsubstituted or substituted piperidinyl, pyrrolidinyl, piperazinyl, morpholinyl;

(6) unsubstituted and substituted phenyl; naphthyl;

(7) unsubstituted and substituted 5 and 6 membered ring heteroaryls having one or two heteroatoms (especially nitrogen heteroatoms); most preferred is pyridinyl;

(8) amine-containing moiety having the general structure:

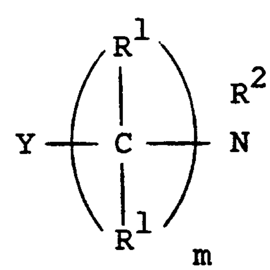

wherein m is an integer from 0 to 10, preferably from 0 to 5, more preferably 0 or 1, and most preferably m = 0; $R^1$ and Y are as described hereinbefore; and $R^2$ is hydrogen, lower alkyl or acyl derived from a carboxylic acid of a lower alkyl;

(9) oxygen-containing moiety having the general structure:

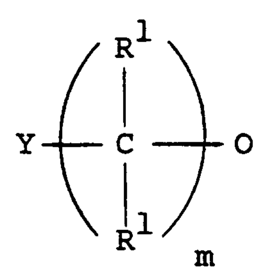

wherein m is an integer from 0 to 10, preferably from 0 to 5, more preferably 0 or 1, and most preferably m = 0; and $R^1$ and Y are as described hereinbefore;

(10) sulfur-containing moiety having the general structure:

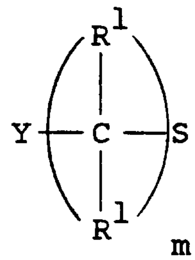

wherein m is an integer from 0 to 10, preferably from 0 to 5, more preferably 0 or 1, and most preferably m = 0; and $R^1$ and Y are as described hereinbefore; and

(11) peptide-containing moiety having the general structure:

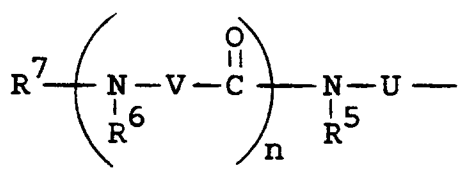

or

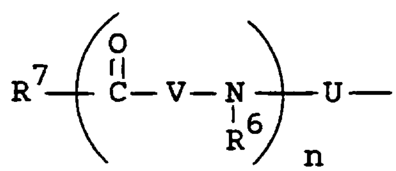

wherein n is an integer from 1 to 100, preferably from 1 to 6; $R^5$, each $R^6$ and $R^7$ are independently hydrogen or lower alkyl, preferably $R^5$, each $R^6$ and $R^7$ are hydrogen; U and each V are independently unsubstituted or substituted lower alkyl (substituted such that moiety is chemically-stable), or $R^5$ and U or each $R^6$ and V, together with the included nitrogen atom to which they are bound, may form a five- or six-membered ring which is unsubstituted or substituted; or U may be nil; preferably U and each V or rings in which they are incorporated are moieties found in naturally-occurring amino acid moieties, i.e., lysine, leucine, isoleucine, valine, phenylalanine, arginine, histidine, methionine, alanine, aspartic acid, threonine, proline, glycine, serine, tyrosine, tryptophan, glutamine and cysteine.

Preferred A moieties of the present invention are optionally substituted alkyl, optionally substituted alkenyl, and optionally substituted aryl.

B is a moiety selected from hydrogen; halogen; unsubstituted and substituted lower alkyl; unsubstituted and substituted cycloalkyl having from 3 to 7 atoms in the ring; unsubstituted and substituted heterocycle having from 3 to 7 atoms in the ring; unsubstituted and substituted phenyl; hydroxy, and the ester thereof derived from a carboxylic acid of a lower alkyl group; thiol; unsubstituted amino, and the amide thereof derived from a carboxylic acid of a lower alkyl group; amino substituted with one lower alkyl group, and the amide thereof derived from a carboxylic acid of a lower alkyl group; amino substituted independently with two lower alkyl groups; $-CO_2H$, the pharmaceutically acceptable salts thereof, the ester thereof derived from an alcohol of a lower alkyl group, the unsubstituted amide thereof, and the amide thereof substituted with one or two lower alkyl groups.

For the sake of chemical stability for the compounds of the present invention, it is preferred that the A and B moieties do not both have heteroatoms (N, O or S), or a heteroatom and a halogen, bonded to the methylene phosphonoalkylphosphinate moiety (i.e., the carbon atom geminally substituted with the phosphorus atoms). Thus, when the A moiety has an oxygen, sulfur, nitrogen, or halogen atom bonded to the phosphorus-substituted methylene carbon, then B is selected from hydrogen; unsubstituted or substituted lower alkyl, cycloalkyl, heterocycle (where a carbon atom of the heterocycle is bonded to the geminal carbon atoms), or phenyl; $-CO_2H$, the pharmaceutically acceptable salts thereof, the ester thereof derived from an alcohol of a lower alkyl group, the unsubstituted amide thereof, and the amide thereof substituted

with one or two lower alkyl groups.

Preferred B is selected from hydrogen, halogen, unsubstituted and substituted lower alkyl, unsubstituted and substituted phenyl, unsubstituted and substituted benzyl, hydroxy and the ester thereof derived from a carboxylic acid of a lower alkyl group, thiol, unsubstituted amino and the amide thereof derived from a carboxylic acid of a lower alkyl group, amino substituted with one lower alkyl group and the amide thereof derived from a carboxylic acid of a lower alkyl group, amino substituted independently with two lower alkyl groups, and $-CO_2H$, and the pharmaceutically acceptable salts thereof and the ester thereof derived from an alcohol of a lower alkyl group and the unsubstituted amide thereof or the amide thereof substituted with one or two lower alkyl groups.

The use of the invention may also be carried out employing the bisphosphonate and/or methylene phosphonoalkylphosphinate in combination with an antihyperlipoproteinemic agent such as probucol and/or with one or more serum cholesterol lowering agents such as Lopid (gemfibrozil), fibric acid derivatives such as bezafibrate, bile acid sequestrants such as cholestyramine, colestipol, polidexide (DEAE-Sephadex) as well as clofibrate, nicotinic acid and its derivatives, neomycin, p-aminosalicyclic acid, bezafibrate and the like and/or one or more HMG CoA reductase inhibitors such as lovastatin, pravastatin, velostatin or simvastatin.

The above compounds to be employed in combination with the protein-prenyl transferase inhibitor will be used in amounts as indicated in the Physicians' Desk Reference (PDR).

The compounds employed in the methods of the invention may also be employed with sodium lauryl sulfate or other pharmaceutically acceptable detergents to enhance oral bioavailability of such compounds.

In carrying out the method of the invention, a pharmaceutical composition will be employed containing at least one bisphosphonate and/or one methylene phosphonoalkylphosphinate protein-prenyl transferase inhibitor in association with a pharmaceutical vehicle or diluent. The pharmaceutical compostion can be formulated employing conventional solid or liquid vehicles or diluents and pharmaceutical additives of a type appropriate to the mode of desired administration. The compounds can be administered to mammalian species including humans, monkeys, dogs, etc. by an oral route, for example, in the form of tablets, capsules, granules or powders, or they can be administered by a parenteral route in the form of injectable preparations. The dose for adults is preferably between 200 and 2,000 mg per day, which can be administered in a single dose or in the form of individual doses from 1-4 times per day.

A typical capsule for oral administration contains protein-prenyl transferase inhibitor (250 mg), lactose (75 mg) and magnesium stearate (15 mg). The mixture is passed through a 60 mesh sieve and packed into a No. 1 gelatin capsule.

A typical injectible preparation is produced by asceptically placing 250 mg of sterile protein-prenyl transferase inhibitor into a vial, asceptically freeze-drying and sealing. For use, the contents of the vial are mixed with 2 ml of physiological saline, to produce an injectible preparation.

## Claims

1. Use of a bisphosphonate protein-prenyl transferase inhibitor wherein the phosphonates are bridged by a methylene group, and which includes at least one lipophilic group attached to the methylene group which contains at least six carbons, for the manufacture of a medicament for treating and/or preventing ras-related tumors by blocking the prenylation of ras oncogene products.

2. Use of a bisphosphonate compound having the structure

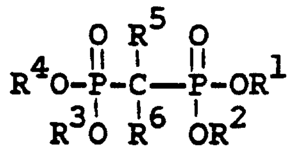

wherein $R^1$, $R^2$, $R^3$ and $R^4$ are the same or different and are H, alkyl, aryl, alkylaryl, arylalkyl, ammonium, alkali metal or a prodrug ester, wherein at least one of $R^5$ and $R^6$ is a hydrocarbyl group having at least 6 carbons (which is alkyl, alkenyl, alkynyl, cycloalkyl, aryl, alkylaryl, arylalkyl, or arylalkenyl); heterocyclic (which is succinimdyl, pyridyl, quinalyl, morpholino, furanyl, indolyl, picolinyl, thiophene, imidazole, oxazole, isoxazole, thiazole, pyridine, 1,2,3-triazole, 1,2,4-triazole, benzimidazole, tetrahydrofuranyl, pyrrolidino, piperidino, 5-membered heteroarylmethyl containing 2 to 4 N atoms or 1-2 N atoms plus an O or S atom); heterocyclicalkyl (wherein heterocyclic is as defined above such as 1-(decahydroquinolin-3-yl)methane); amino; alkylamino; dialkylamino; arylalkylaminoalkyl; ethylcarbonyloxymethylamino; cycloalkyl(alkyl)amino; alkenylamino, cycloalkylamino, aminocycloalkyl; aminocycloalkylalkyl; N-hydroxy-N-ethylamino; acetylamino; aminoalkyloxyalkyl; (benzo- or cyclohexeno-fused) 5 membered heteroaryl containing 2-4 N atoms or 1-2 Natoms plus an O or S atom; $R^8$-X-$(CH_2)_a$-(wherein $R^8$ is H, alkyl, or a nitrogen containing 6-membered aromatic ring which is pyridyl, indanyl, hexahydroindanyl or picolyl; X is O, NH or a single bond and a is 0 to 7);

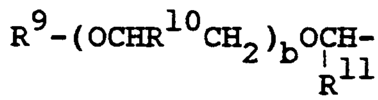

(wherein $R^9$ is $C_1$-$C_{10}$ alkyl, optionally substituted aryl, phenylalkyl or naphthylalkyl),

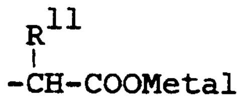 or 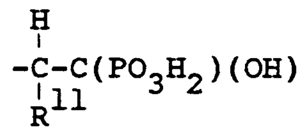

(wherein $R^{10}$ and and $R^{11}$ are the same or different and are H or methyl, b is 1 to 20));

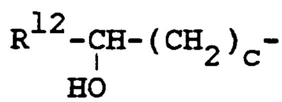

(wherein $R^{12}$ is H, phenyl or phenyl substituted with halogen, alkyl or hydroxy and c is 0 to 9);

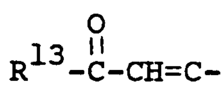

(wherein $R^{13}$ is tert-alkyl ($CR^{14}R^{15}R^{16}$ wherein $R^{14}$ and $R^{15}$ are independently $C_1$-$C_3$ alkyl and $R^{16}$ is $C_1$-$C_{10}$ alkyl), cycloalkyl, aryl or heteroaryl, or substituted cycloalkyl, substituted aryl or substituted heteroaryl wherein the substituent is halogen, $C_1$-$C_4$ alkyl, alkoxy or dialkylamino);

4-Cl-$C_6H_5$-S-$CH_2$; aryloxy;

$R^{17}$-$(QCH_2CH_2)_d$O- (wherein $R^{17}$ is $C_1$-$C_{10}$ alkyl, $C_2$-$C_{10}$ alkenyl, aryl or arylalkyl, or each of the above $R^{17}$ groups optionally substituted with $C_1$-$C_4$ alkyl, amino, alkylamino, carboxyl, alkoxycarbonyl, hydroxy, alkoxy, phenoxy, mercapto, alkylthio, phenylthio, halogen or trifluoromethyl, Q is O or S and d is 0, 1 or 2);

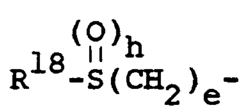

(wherein e is 0 to 10, h is 0, 1 or 2, $R^{18}$ is H, cycloalkyl, aryl, alkyl, each optionally substituted with OH, SH, halogen, alkoxycarbonyl or $NZ_1Z_2$, phenyl optionally substituted with halogen, nitro, $C_1$-$C_6$-alkyl, alkoxy, trifluoromethyl, amino, carboxyl, $CO_2$alkyl, -$CONZ_1Z_2$, -$CSNZ_1Z_2$, a 5- or 6-membered heterocyclic radical containing 1 or 2 heteroatoms, which are N or S, which may or may not be fused to a benzene ring, $Z_1$ and $Z_2$ are independently H or $C_1$-$C_6$-alkyl);

thiol; phenylthio; chlorophenylthio; 4-thiomorpholinyl;

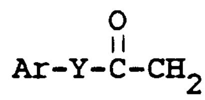

(wherein Ar is aryl, pyrrolyl or aryl optionally substituted with $C_1$-$C_4$alkyl, alkoxy, halo (F, Cl), naphthyl, biphenyl or thienyl and Y is NH or a single bond);

$R^{19}SCH_2$- (wherein $R^{19}$ is alkyl, aryl or arylalkyl);

A-$(CH_2)_f$-NH- (wherein A is $C_5$-$C_8$ cycloalkenyl, bicycloheptyl, bicycloheptenyl, saturated $C_4$-$C_7$ heterocycle containing O,S,SO or $SO_2$);

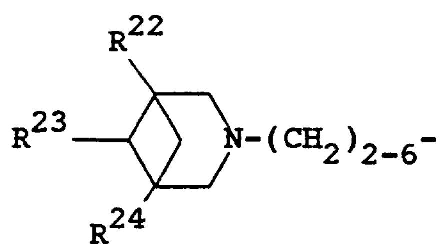

(wherein $R^{22}$ is H, $C_1$-$C_{20}$alkyl, alkoxy, aryl, $R^{23}$ is H, $C_1$-$C_{20}$alkyl, alkoxy, aryl, halo, carboxyl, $R^{24}$ is H, $C_1$-$C_{20}$ alkyl, alkoxy);

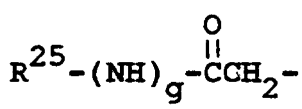

(wherein $R^{25}$ is (alkyl-substituted)pyrrolyl or phenyl and g is 0 or 1);

aromatic-substituted mono- or biazacyclylalkyl (alkyl group bonds with the N in the heterocycle) (such as 3-(4-phenylpiperidino)propyl);

$R^{31}$-Ax-CO-$CH_2$-

(wherein Ax is phenyl, naphthyl, mono- or bicyclic-N-containing heterocycle and $R^{31}$ is H, halo, $C_1$-$C_6$-alkyl or $C_1$-$C_6$alkoxy);

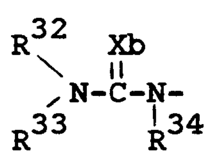

(wherein $R^{32}$ is aryl, aralkyl, alkyl, $R^{33}$ is H or aryl, Xb is O or S, and $R^{34}$ is H or alkyl);

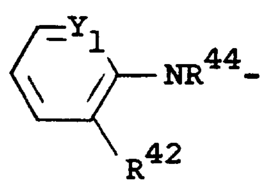

(wherein $R^{42}$ is H, alkyl or halo, $Y_1$ is N, NO, or $NR^{43}Y_2$ wherein $R^{43}$ is alkyl and $Y_2$ is halo; and $R^{44}$ is

H or aliphatic acyl);

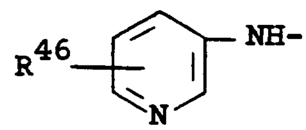

(wherein $R^{46}$ is H, halo or alkyl);

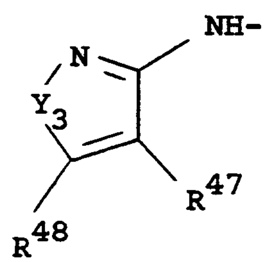

(wherein $Y_3$ is O or NH, $R^{47}$ is H, alkyl or halo, and $R^{48}$ is H or alkyl);

$R^{50}$ -NH-

(wherein $R^{50}$ is

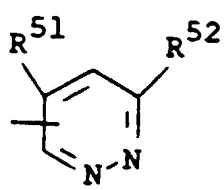

,

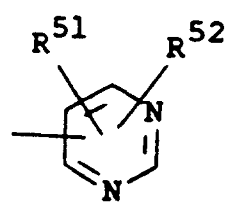

or

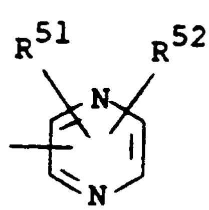

wherein $R^{51}$ and $R^{52}$ are H, halo, alkyl or hydroxy);

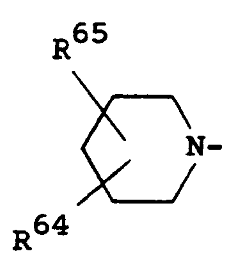

(wherein $R^{64}$ is alkyl and $R^{65}$ is H or alkyl;

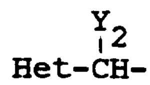

wherein Het is a heteroaromatic 5-membered ring with 2 or 3 heteroatoms, optionally partially hydrogenated and optionally substituted by one or more alkyl, alkoxy, phenyl, cyclohexyl, cyclohexylmethyl, halo or amino, with 2 adjacent alkyl optionally together forming a ring (Het cannot be pyrazole), and $Y_2$ is H or $C_1$-$C_6$-alkyl);

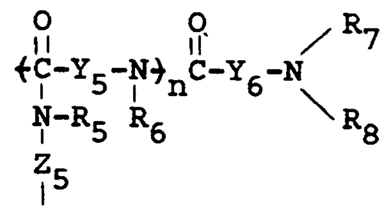

(wherein $Y_4$ is H or OH, $R_5$-$R_8$ are independently H or lower alkyl, whereby $R_7$ and $Y_6$ or $R_6$ and $Y_5$ or $R_5$ and $Z_5$, together with the nitrogen atom to which they are attached can form a 5- or 6-membered ring, $Y_6$ and $Y_5$ which can be the same or different are $C_1$-$C_6$ alkylene chains optionally substituted by aromatic or heteroaromatic radicals, $Z_5$ is $C_1$ to $C_6$ alkylene which can include heteroatoms and optionally substituted by aromatic or heteroaromatic, n is 0, 1 or 2;

$R_{27}$-$Z_9$-

(wherein $R_{27}$ is aryl or heterocyclyl both optionally substituted by one or more of $C_1$-$C_6$-alkyl, $C_1$-$C_6$-alkoxy, $C_1$-$C_6$-alkylthio, halo($C_1$-$C_6$-)alkyl, acyl, acylamino or halo, or $R_{27}$ is $C_1$-$C_6$alkyl substituted by heterocyclyl which is optionally substituted by acyl); $R_{27}$-$Z_9$ is $R_{27}$-NHC(=$X_9$), $R_{27}$-C(=O)NH-, $R_{27}$-$SO_2$-NH- (wherein $X_9$ is O or S);

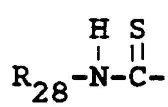

(wherein $R_{28}$ is phenyl, pyridyl or quinolyl substituted by $C_1$-$C_6$alkylsulphonylamino, halo-$C_1$-$C_6$ alkylsulphonylamino, arylsulphonylamino and mono- or di-$C_1$-$C_6$alkylamino);

$R_{29}$-CO-[-$R_{30}$($CH_2$)$_o$CO-]$_p$-NH-

(wherein $R_{29}$-CO- is a residue of a pharmaceutically active compound $R_{29}$-COOH, wherein $R_{29}$ is an anti-inflamatory agent, or antioncotic agent or hormone ,

$R_{30}$ is -NH- or -O-
p is 0 or 1;
o is 1-10);

$R_{33}$-($CH_2$)$_q$-

(wherein $R_{33}$ is an N-bonded azabicycloalkyl group with 3 to 8-membered rings and q is 2 to 4);

$R_{34}$-($CH_2$)$_r$-

(wherein $R_{34}$ is an N-bonded, aryl-substituted mono- or diazacycloaliphatic group);

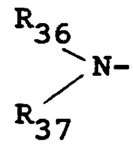

(wherein $R_{36}$ is 5 membered heteroaryl with 2-4 N or with 1-2 N plus an O or S atom, optionally fused to a benzo or cyclohexeno ring;

$R_{36}$ can be C substituted by $C_1$-$C_6$-alkyl, phenyl (optionally substituted by $C_1$-$C_6$-alkyl, alkoxy and/or halo), $C_1$-$C_6$-alkoxy, OH, di(lower alkyl)amino, $C_1$-$C_6$-alkylthio and/or halo, and/or N substituted by $C_1$-$C_6$-alkyl or phenyl ($C_1$-$C_6$-)alkyl (optionally substituted by lower alkyl, lower alkoxy and/or halo);

$R_{37}$ is H or $C_1$-$C_6$-alkyl; provided $R_{37}$ is not H if $R_{36}$ is optionally substituted alkyl and/or halo substituted 3-pyrazolyl or 3-isoxazolyl);

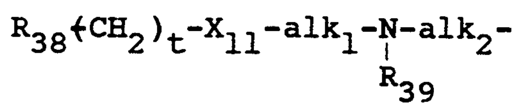

(wherein $R_{38}$ is aromatic residue;

t is 0-3;

$X_{11}$ is 0 S (optionally oxidized) or imino (optionally substituted by aliphatic group);

$alk_1$ and $alk_2$ are divalent aliphatic groups; $R_{39}$ is H or monovalent aliphatic group);

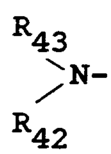

(wherein $R_{42}$ and $R_{43}$ are hydrogen, alkyl having one to 22 carbon atoms, cycloalkyl having five to six carbon atoms, phenyl alkylphenyl having seven to 18 carbon atoms, phenylalkyl having seven to 18 carbon atoms and together with the nitrogen atom, piperidino, pyrrolidino and morpholino);

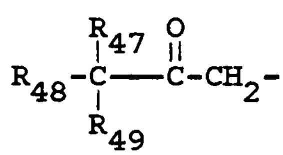

(wherein $R_{47}$ is optionally branched $C_1$-$C_8$alkyl,

$R_{48}$ and $R_{49}$ are each methyl or ethyl, and

M is H or a cation of a water-soluble base);

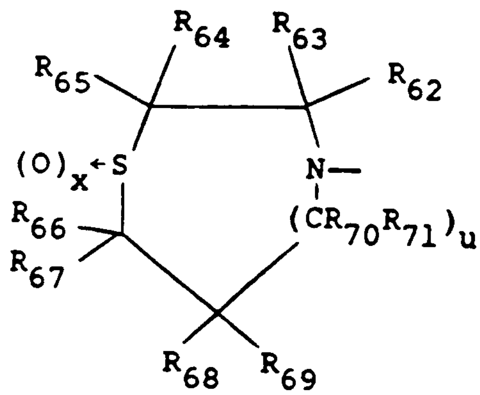

(wherein $R_{62}$-$R_{71}$ is H, straight, branched or alicyclic 1-10C hydrocarbyl, aryl or aryl-(1-4C)-alkyl;

x is 0 or 1;

u is 0, 1 or 2;

or $R_{62}$ and $R_{64}$ may complete a 5- to 7-membered saturated aliphatic ring optionally substituted by 1 or more alkyl groups);

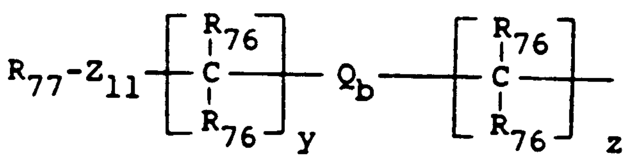

(wherein $Z_{11}$ is an N-containing 6-membered ring heterocycle moiety selected from piperidinyl, diazinyl or triazinyl;

$Q_b$ is a covalent bond, O, S or $NR_{76}$;

y, z, and y + z are integers of 0-10;

$R_{76}$ is H, or $C_1$-$C_3$alkyl;

$R_{77}$ is one or more substituted selected from H, halogen, 1-3C alkyl, unsubstituted amino and its amide derived from a 1-3C carboxylic acid, mono(1-3C alkyl) amino and its amide derived from a 1-3C carboxylic acid, di(1-3C alkyl)amino, tri(1-3C alkyl) ammonium, hydroxy or its ester derived from a 1-3C carboxylic acid, ether having 1-3C, $CO_2H$ and its salts and esters derived from 1-3C alcohols, its amide optionally substituted with one or two 1-3C alkyl groups, and $NO_2$);

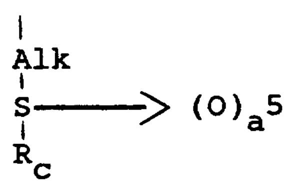

(wherein $R_c$ represents:

$C_1$-$C_6$ alkyl group,

$C_5$-$C_7$ cycloalkyl group,

phenyl group optionally monosubstituted or polysubstituted by a halogen, a $C_1$-$C_6$ alkyl group or a trifluoromethyl group, or

5-membered or 6-membered heterocycle containing 1 or 2 heteroatoms chosen from nitrogen and sulfur,

Alk denoted a linear or branched $C_1$-$C_6$ alkylene group,

$a^5$ represents 0 or the integer 1 or 2);

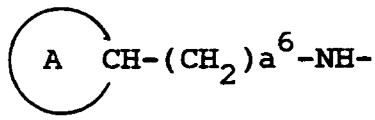

(wherein $a^6$ is 0 to 4 and

Ring A is 5-8C cycloalkenyl, bicycloheptyl, bicycloheptenyl or 4-7C saturated heterocyclyl containing 0, S, SO or $SO_2$);

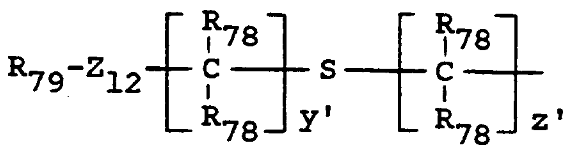

(wherein $Z_{12}$ is a 6-membered aromatic ring containing ≧ 1 N atom(s); where:

the ring is optionally substituted by (optionally substituted, optionally unsaturated) 1-6C alkyl, (optionally substituted) aryl, (optionally substituted) benzyl, OH, halogen, carbonyl, alkoxy, $NO_2$, $CONH_2$, (optionally substituted) $NH_2$ and/or carboxylate, such as pyridine, pyridazine, pyrimidine or pyrazine ring;

$R_{78}$ is H or (optionally substituted, optionally unsubstituted) 1-4C alkyl;

$R_{79}$ is H, (optionally substituted, optionally unsubstituted) 1-6C alkyl, (optionally substituted) aryl, (optionally substituted) benzyl, OH, halogen, carbonyl, alkoxy, $NO_2$, $CONH_2$, (optionally substituted) amino or carboxylate,

y' + z' is 0 to 5);

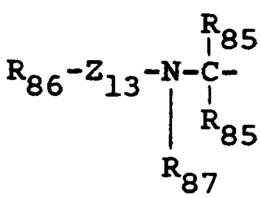

or

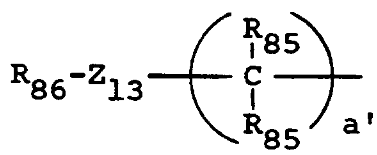

(wherein $Z_{13}$ is a pyridine, pyridazine, pyrimidine or pyrazine ring, optionally substituted by optionally unsaturated 1-6C alkyl, optionally substituted aryl, optionally substituted benzyl, OH, halogen, oxo, alkoxy, $NO_2$, amido, optionally substituted $NH_2$ or carboxylate;

$R_{86}$ is H or optionally substituted, optionally unsaturated 1-4C alkyl;

$R_{86}$ is one or more of H, optionally substituted, optionally unsaturated 1-6C alkyl, optionally substituted aryl, optionally substituted benzyl, OH, halogen, oxo, alkoxy, $NO_2$, amido, optionally substituted $NH_2$ or carboxylate;

$R_{87}$ is H, optionally substituted, optionally unsaturated 1-4C alkyl or acyl;

a' is 1-5);

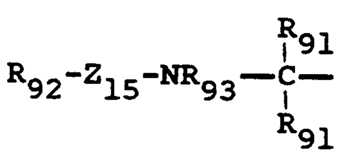

or

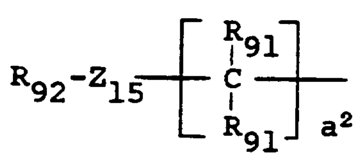

(wherein $Z_{15}$ is a 6 membered aromatic ring containing one or more N atoms such as pyridine, pyridazine, pyrimidine or pyrazine, which ring may be substituted with one or more optionally substituted, optionally unsaturated 1-6C alkyl, optionally substituted aryl, optionally substituted benzyl, OH, halogen, carbonyl, alkoxy, $NO_2$, amido, optionally substituted amino or carboxylate);

$R_{91}$ is H or optionally substituted, optionally unsaturated 1-4C alkyl;

$R_{92}$ is H or one or more substituents selected from optionally substituted, optionally unsaturated 1-6C alkyl, optionally substituted aryl, optionally substituted benzyl, OH, halogen, carbonyl, alkoxy, $NO_2$, amido, optionally substituted amino or carboxylate;

$R_{93}$ is H, optionally substituted, optionally unsaturated 1-4C alkyl or acyl;

$a^2$ is 1 to 5);

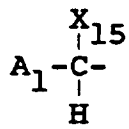

(wherein $X_{15}$ is hydrogen, methyl, or ethyl, and $A_1$ is phenyl substituted in the para-position by isobutyl, cyclohexyl, alkoxy, or 1-pyrrolinyl and, optionally substituted additionally in the meta-position by fluorine or chlorine, or phenyl substituted in the meta-position by benzoyl or phenoxy, or phenyl substituted in the ortho-position by 2,4-dichlorophenoxy or 2,6-dichlorophenylamino);

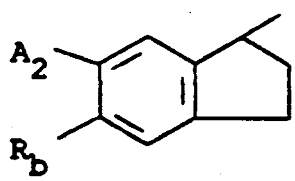

(wherein

$R_b$ is cyclohexyl or cyclophenylmethyl; and

$A_2$ is hydrogen or chlorine);

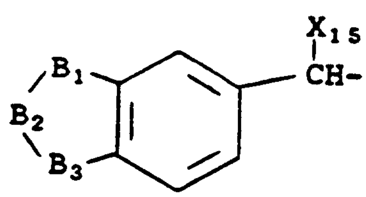

(wherein $X_{15}$ is as defined above, and

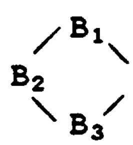

is

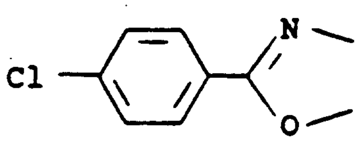

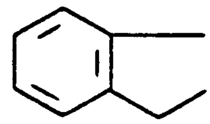

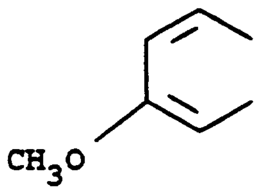

or

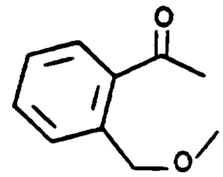

;

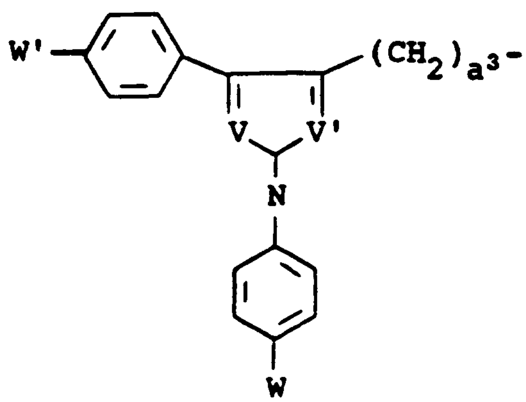

wherein $a^3$ is 1, 2 or 3;

W and W', are identical or different, and each is hydrogen, fluorine or chlorine, and

one of V and V' is nitrogen and the other is a methyne residue optionally substituted by a phenyl group, and

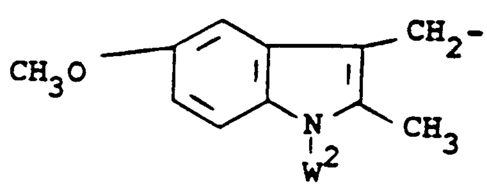

wherein $W^2$ is p-chlorobenzoyl or cinnamoyl);

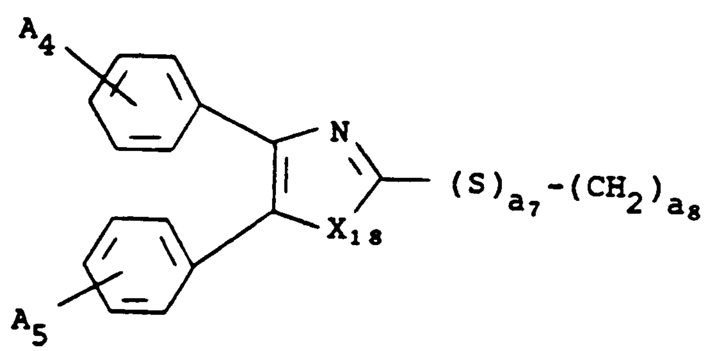

(wherein $A_4$ and $A_5$ are the same or different and are H, OH, lower alkoxy or halogen;
$X_{15}$ is O, S or NH;
$a_7$ is 0 or 1;
$a_8$ is 0 or an integer of 1-6);

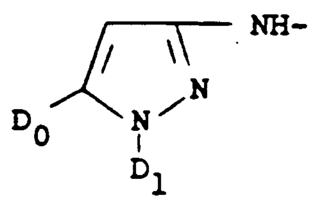

(wherein $D_0$ is H or alkyl;
$D_1$ is H or $C_1$-$C_6$-alkyl);

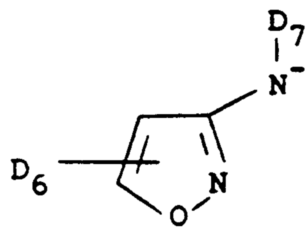

(wherein $D_6$ is H, 1-10C alkyl, 3-10C cycloalkyl, phenyl, 2-10C alkenyl (optionally substituted by phenyl) or phenyl(1-5C)alkyl (optionally ring-substituted by a 1-5C alkoxy);
$D_7$ is H or 2-6C alkanoyl);

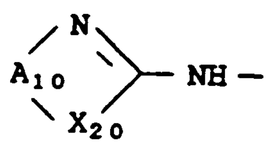

(wherein $A_{10}$ is a group of formula (a)-(c):

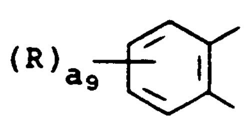
(a)

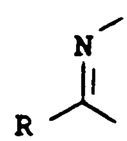
(b)

or

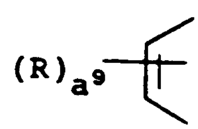
(c)

and $X_{20}$ is O, S or NH);

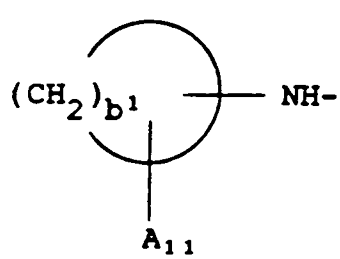

(wherein $A_{11}$ is H or 1-5C alkyl;
$b_1$ is 3-10);

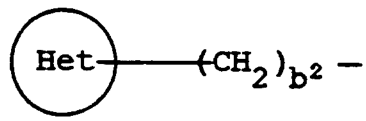

(wherein ring Het is a group of formula (A) or (B):

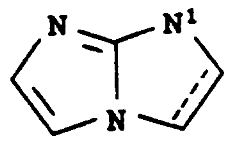

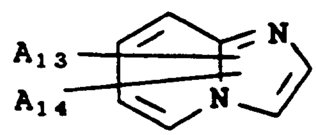

(A) (B)

the dotted line represents an optional double bond; $A_{13}$, $A_{14}$ are H, 1-5C alkyl, halogen or OH);

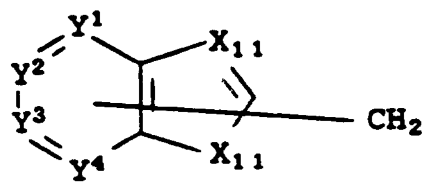

(wherein $X_{11}$ are both N or one is N and the other is CH; one of $Y^1$-$Y^4$ is N and the rest is CH);

and the other of $R^5$ and $R^6$ is H, halogen, $C_1$-$C_{30}$ alkyl, amino, alkylamino, dialkylamino, uriedo ($NH_2CO$-$N(R^{38})$- where $R^{38}$ is H, alkyl, benzyl, phenyl optionally substituted with Cl or $CH_3$); alkenylamino, cycloalkylamino, aryloxy, pyridinium, guanidinium, ammonium, di-and tri-lower alkanolammonium, hydroxy, arylalkyl, alkoxy, alkylaryloxy, $-CH_2CO_2H$, $-CH_2PO_3H_2$, $-CH(PO_3H_2)(OH)$, $-CH_2CO_2C_2H_5$, $-CH_2CH(PO_3H_2)_2$, a hydrocarbyl radical as defined herein, a heterocyclic radical as defined herein, alkanoyl, an $R^6$ or $R^5$ radical as defined herein, a prodrug ester (such as (1-alkanoyloxy)alkyl, for example $t$-$C_4H_9CO_2CH_2$-, $CH_3CO_2CH_2$-);

at least one of $R^5$ and $R^6$ being a lipophilic group, or $R^5$ and $R^6$ can be joined to form a carbocyclic ring containing 3 to 12 carbons or a heterocyclic ring containing N, O and/or S atoms, such as of the formula

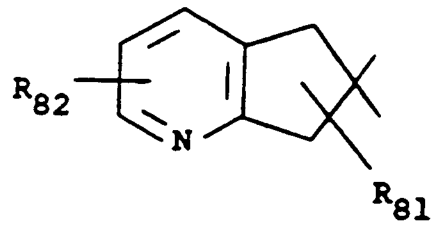

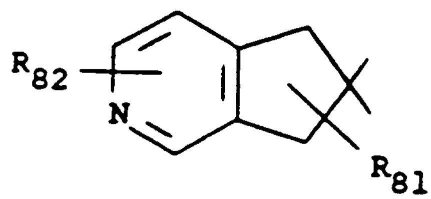

or 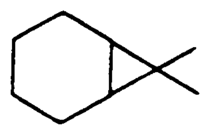

wherein $R_{81}$ and $R_{82}$ are each one or more substituents selected from H, optionally substituted saturated or unsaturated 1-6C alkyl, optionally substituted aryl, optionally substituted benzyl, amido, OH, halogen, optionally substituted amino, amido, COOH, carbonyl, carboxylate, alkoxy and $NO_2$, for the manufacture of a medicament for treating and/or preventing ras-related tumors by blocking the farnesylation of ras oncogene products.

3. Use of a methylene phosphonoalkyl-phosphinate protein-prenyl transferase far the manufacture of a medicament for treating and/or preventing ras-related tumors by blocking the prenylation of ras oncogene products.

4. The use as defined in Claim 3 wherein the methylene phosphonoalkylphosphinate protein-prenyl transferase inhibitor includes at least one lipophilic group which is a group which contains at least 6 carbons and is required for strong enzyme inhibitor binding and inhibition of the enzyme squalene synthetase or other enzymes in the cholesterol biosynthetic pathway.

5. The use as defined in Claim 3 wherein the methylene phosphonoalkylphosphinate compound has the structure

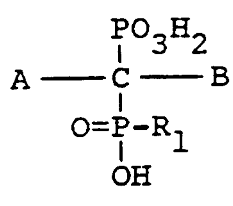 (1)

wherein $R_1$ is selected from hydrogen, substituted alkyl and unsubstituted alkyl, and A and B are independent substituent moieties, wherein at least one of which is a lipophilic group which is a group which contains at least 6 carbons and is required for strong enzyme inhibitor binding and inhibition of the enzyme protein-prenyl transferase(s).

6. The use as defined in Claim 5 wherein A is a moiety selected from hydrogen; halogen; nitro, alkyl; heterocycle; aryl; heteroaryl; unsubstituted amino, and the amide thereof derived from a carboxylic acid of a substituent group; amino substituted with one substituent group, and the amide thereof derived from a carboxylic acid of a substituent group; amino substituted independently with one alkyl group and

one substituent group; hydroxy, and the ester thereof derived from a carboxylic acid of a substituent group; ether having a substituent group; thiol, and the thiol ester thereof derived from a carboxylic acid of a substituent group; thioether having a substituent group, and the sulfoxide and sulfone derivative thereof; $-SO_3H$, the pharmaceutically acceptable salts thereof, the ester thereof derived from an alcohol of a substituent group, the unsubstituted amide thereof, and the amide thereof substituted with one or two alkyl groups; $-CO_2H$, the pharmaceutically acceptable salts thereof, the ester thereof derived from an alcohol of a substitutent group, the unsubstituted amide thereof, and the amide thereof substituted with one or two alkyl groups; aldehyde; ketone having a substituent group; carbamate, unsubstituted or substituted with one or two alkyl groups; pepetides having from about one to 100 amino acid moieties; or the A and B moieties are covalently linked to form a ring having 3 to 7 atoms with from 0 to 3 heteroatoms selected from the group consisting of nitrogen, sulfur, phosphorus and oxygen, the ring being unsubstituted or substituted with one or more of the above substituents of A; or the A and B moieties are replaced by an unsubstituted or substituted alkyl moiety attached to the geminal carbon by a double bond; and

B is a moiety selected from hydrogen; halogen; unsubstituted and substituted lower alkyl; unsubstituted and substituted cycloalkyl having from 3 to 7 atoms in the ring; unsubstituted and substituted heterocycle having from 3 to 7 atoms in the ring; unsubstituted and substituted phenyl; hydroxy, and the ester thereof derived from a carboxylic acid of a $C_1$-$C_6$-alkyl group; thiol; unsubstituted amino, and the amide thereof derived from a carboxylic acid of a $C_1$-$C_6$-alkyl group; amino substituted with one $C_1$-$C_6$-alkyl group, and the amide thereof derived from a carboxylic acid of a $C_1$-$C_6$-alkyl group; amino substituted independently with two $C_1$-$C_6$-alkyl groups; $-CO_2H$, the pharmaceutically acceptable salts thereof, the ester thereof derived from an alcohol of a $C_1$-$C_6$-alkyl group, the unsubstituted amide thereof, and the amide thereof substituted with one or two $C_1$-$C_6$-alkyl groups.

**7.** The use as defined in Claim 6 wherein alkyl is substituted with halogen, nitro, cyano, heterocycle, aryl, heteroaryl, unsubstituted amino, and the amide thereof derived from a carboxylic acid of an alkyl, heterocycle, aryl or heteroaryl group, amino substituted with one alkyl, heterocycle, aryl or heteroaryl group and the amide thereof derived from a carboxylic acid of an alkyl group, amino substituted independently with one alkyl group and one alkyl, heterocycle, aryl or heteroaryl group, hydroxy, and the ester thereof derived from a carboxylic acid of an alkyl, heterocycle, aryl or heteroaryl group; ether having an alkyl, heterocycle, aryl or heteroaryl group; thiol, and the thiol ester thereof derived from a carboxylic acid of an alkyl, heterocycle, aryl or heteroaryl group; thioether having an alkyl, heterocycle, aryl or heteroaryl group, and the sulfoxide and sulfone derivatives thereof, $-SO_3H$, the pharmaceutically acceptable salts therof, the ester thereof derived from an alcohol of an alkyl group, the unsubstituted amide thereof, and the amide thereof substituted with one or two alkyl groups, $-CO_2H$, the pharmaceutically acceptable salts thereof, the ester thereof derived from an alcohol of an alkyl group, the unsubstituted amide thereof, and the amide thereof substituted with one or two alkyl groups, $PO_3H_2$, the pharmaceutically acceptable salts thereof, the ester thereof derived from an alcohol of an alkyl group, the unsubstituted amide thereof, and the amide thereof substituted with one or two alkyl groups, $-(R^8)PO_2H$ (where $R^8$ is hydrogen or unsubstituted lower alkyl), the pharmaceutically acceptable salts thereof, the ester thereof derived from an alcohol of an alkyl group, the unsubstituted amide thereof, and the amide thereof substituted with one or two alkyl groups, aldehyde, ketone having an alkyl group, carbamate, unsubstituted or substituted with one or two alkyl groups, peptidyl, and combinations thereof.

**8.** The use as defined in Claim 7 wherein the term "heterocycle" refers to chemically-stable non-aromatic rings, including fused non-aromatic rings, having from 5 to 20 atoms, comprising at least one heteroatom selected from nitrogen, sulfur, phosphorus and oxygen.

**9.** The use as defined in Claim 8 wherein the term "aryl" refers to chemically-stable aromatic rings, including fused aromatic rings, having from 6 to 20 carbon atoms.

**10.** The use as defined in Claim 6 wherein A is a moiety selected from
(1) hydrogen;
(2) halogen;

(3) substituted and unsubstituted alkyl having the general structure:

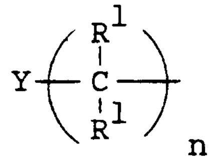

(2)

wherein n is an integer from 1 to 10, preferably from 1 to 5, more preferably n = 1 or 2, and most preferably n = 1; each $R^1$ is independently selected to achieve chemically-stable moieties from hydrogen, halogen, $C_1$-$C_6$-alkyl, unsubstituted amino or the amido thereof derived from a carboxylic acid of a $C_1$-$C_6$-alkyl group, amino substituted with one $C_1$-$C_6$-alkyl group or the amide thereof derived from a carboxylic acid of a lower alkyl group, amino substituted independently with two $C_1$-$C_6$-alkyl groups, hydroxy or the ester thereof derived from a carboxylic acid of a $C_1$-$C_6$-alkyl group, $-CO_2H$ or the pharmaceutically acceptable salts thereof or the ester thereof derived from an alcohol of a $C_1$-$C_6$-alkyl group or the unsubstituted amide thereof or the amide thereof substituted with one or two $C_1$-$C_6$-alkyl groups, ether having a $C_1$-$C_6$-alkyl group, $-PO_3H_2$ or the pharmaceutically acceptable salts thereof, and nitro, or two $R^1$'s on the same carbon atom are =O or $=NR^9$ (where $R^9$ is $C_1$-$C_6$-alkyl or may be hydrogen when there is another nitrogen atom attached to the same carbon atom as the $=NR^9$ moiety), or two $R^1$'s on adjacent carbon atoms may be replaced by an additional bond between the carbon atoms; or an $R^1$ on the first carbon atom (from the right side of structure (2) hereinabove) and B (see structure (1) hereinabove) may be replaced by an additional bond; and Y is a substituent of alkyl as defined hereinbefore; (for the sake of chemical stability of the compounds used in the present invention, $R^1$ cannot be such that there is a halogen and an oxygen or sulfur or nitrogen singly bonded to the same carbon atom or such that two of an oxygen or sulfur or nitrogen are singly bonded to the same carbon atom);

(4) cycloalkyl having from 4 to 10 carbon atoms; more preferred are cycloalkyl having 5 or 6 carbon atoms;

(5) heterocycle having 5 or 6 atoms in the ring; more preferred are heterocycles having one or two nitrogen atoms in the ring, more preferred still are heterocycles having one nitrogen atom in the ring; most preferred are unsubstituted or substituted piperidinyl, pyrrolidinyl, piperazinyl, morpholinyl;

(6) unsubstituted and substituted phenyl; naphthyl;

(7) unsubstituted and substituted 5 and 6 membered ring heteroaryls having one or two heteroatoms (especially nitrogen heteroatoms); most preferred is pyridinyl;

(8) amine-containing moiety having the general structure:

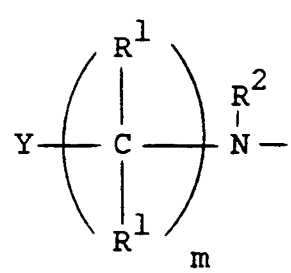

wherein m is an integer from 0 to 10, preferably from 0 to 5, more preferably 0 or 1, and most preferably m = 0; $R^1$ and Y are as described hereinbefore; and $R^2$ is hydrogen, $C_1$-$C_6$-alkyl or acyl derived from a carboxylic acid of a $C_1$-$C_6$-alkyl;

(9) oxygen-containing moiety having the general structure:

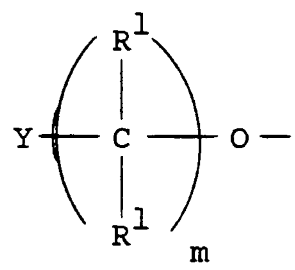

wherein m is an integer from 0 to 10, preferably from 0 to about 5, more preferably 0 or 1, and most preferably m = 0; and $R^1$ and Y are as described hereinbefore;
(10) sulfur-containing moiety having the general strucutre:

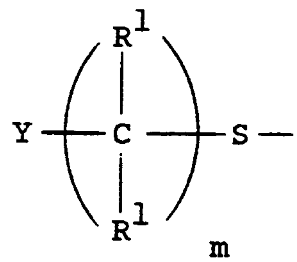

wherein m is an integer from 0 to 10, preferably from 0 to 5, more preferably 0 or 1, and most preferably m = 0; and $R^1$ and Y are as described hereinbefore; and
(11) peptide-containing moiety having the general structure:

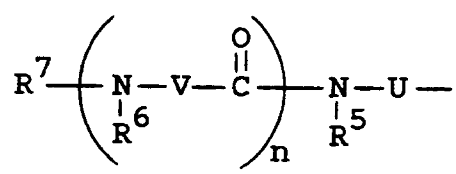

or

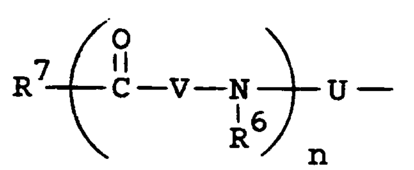

wherein n is an integer from 1 to 100, preferably from 1 to 6; $R^5$, each $R^6$ and $R^7$ are independently hydrogen or $C_1$-$C_6$-alkyl, preferably $R^5$, each $R^6$ and $R^7$ are hydrogen; U and each V are independently unsubstituted or substituted $C_1$-$C_6$-alkyl (substituted such that moiety is chemically-stable), or $R^5$ and U or each $R^6$ and V, together with the included nitrogen atom to which they are bound, may form a five- or six-membered ring which is unsubstituted or substituted; or U may be nil; preferably U and each V or rings in which they are incorporated are moieties found in naturally-occurring amino acid moieties, i.e., lysine, leucine, isoleucine, valine, phenylalanine, arginine, histidine, methionine, alanine, aspartic acid, threonine, proline, glycine, serine, tyrosine, tryptophan, glutamine and cysteine.

## Patentansprüche

1. Verwendung eines Biphosphonat Protein-Prenyl Transferase-Inhibitors, in dem die Phosphonate durch eine Methylengruppe verbrückt sind, und der wenigstens einen an die Methylengruppe gebundenen lipophilen Rest mit wenigstens sechs Kohlenstoffatomen enthält, zur Herstellung eines Arzneimittels zur Behandlung und/oder Vorbeugung von Tumoren, die mit Ras in Verbindung stehen, durch Blockierung der Prenylierung Ras-oncogener Produkte.

2. Verwendung einer Biphosphonat-Verbindung der Struktur

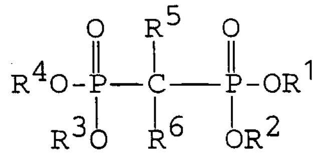

in der $R^1$, $R^2$, $R^3$ und $R^4$ gleich oder verschieden sind und Wasserstoffatome, Alkyl-, Aryl-, Alkylaryl- oder Arylalkylreste, Ammoniumgruppen, Alkalimetallatome oder Prodrug-Esterreste sind, wobei wenigstens einer der Reste $R^5$ und $R^6$ ein Kohlenwasserstoffrest mit wenigstens 6 Kohlenstoffatomen (der ein Alkyl-, Alkenyl-, Alkinyl-, Cycloalkyl-, Aryl-, Alkylaryl-, Arylalkyl- oder Arylalkenylrest ist); ein heterocyclischer Rest (der eine Succinimidyl-, Pyridyl-, Chinalyl-, Morpholin-, Furanyl-, Indolyl-, Picolinyl-, Thiophen-, Imidazol-, Oxazol-, Isoxazol-, Thiazol-, Pyridin-, 1,2,3-Triazol-, 1,2,4-Triazol-, Benzimidazol-, Tetrahydrofuranyl-, Pyrrolidin- oder Piperidingruppe, oder ein Heteroarylmethyl-Fünfring mit 2 bis 4 N-Atomen oder 1-2 N-Atomen plus einem O- oder S-Atom ist); ein Heterocycloalkylrest (wobei heterocyclisch die vorstehend angegebene Bedeutung hat, wie eine 1-(Decahydrochinolin-3-yl)-methangruppe); ein Aminorest; ein Alkylaminorest; ein Dialkylaminorest; ein Arylalkylaminoalkylrest; ein Ethylcarbonyloxymethylaminorest; ein Cycloalkyl(alkyl)aminorest; ein Alkenylaminorest; ein Cycloalkylaminorest; ein Aminocycloalkylrest; ein Aminocycloalkylalkylrest; ein N-Hydroxy-N-ethylaminorest; ein Acetylaminorest; ein Aminoalkyloxyalkylrest; ein mit einer Benzo- oder Cyclohexengruppe kondensierter Heteroaryl-Fünfring mit 2-4 N-Atomen oder 1-2 N-Atomen plus einem O- oder S-Atom; $R^8$-X-$(CH_2)_a$- (wobei $R^8$ ein Wasserstoffatom, ein Alkylrest oder ein Stickstoff enthaltender aromatischer Sechsring ist, der eine Pyridyl-, Indanyl-, Hexahydroindanyl- oder Picolylgruppe ist; X O, NH oder eine Einfachbindung ist, und a 0 bis 7 ist);

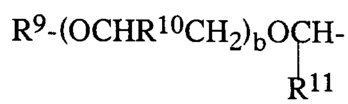

(wobei $R^9$ ein gegebenenfalls mit einem Aryl-, Phenylalkyl- oder Naphthylalkylrest substituierter $C_1$-$C_{10}$-Alkylrest ist),

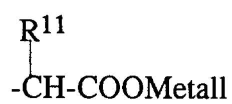 oder 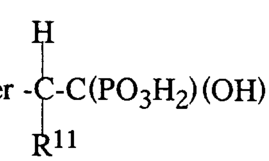

(wobei $R^{10}$ und $R^{11}$ gleich oder verschieden sind, und ein Wasserstoffatom oder eine Methylgruppe sind, b 1 bis 20 ist));

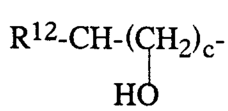

(wobei $R^{12}$ ein Wasserstoffatom, eine Phenylgruppe oder eine mit Halogenatomen, Alkylresten, Hydroxygruppen substituierte Phenylgruppe ist, und c 0 bis 9 ist);

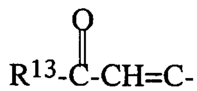

(wobei $R^{13}$ ein tert-Alkyl- ($CR^{14}R^{15}R^{16}$, wobei $R^{14}$ und $R^{15}$ unabhängig voneinander $C_1$-$C_3$-Alkylreste sind, und $R^{16}$ ein $C_1$-$C_{10}$-Alkylrest ist), Cycloalkyl-, Aryl- oder Heteroarylrest oder ein substituierter Cycloalkyl-, oder substituierter Aryl-, oder substituierter Heteroarylrest ist, wobei der Substituent ein Halogenatom, ein $C_1$-$C_4$-Alkyl-, Alkoxy- oder Dialkylaminorest ist);

4-Cl-$C_6H_5$-S-$CH_2$; ein Aryloxyrest;

$R^{17}$-$(QCH_2CH_2)_dO$- (wobei $R^{17}$ ein $C_1$-$C_{10}$-Alkyl-, $C_2$-$C_{10}$-Alkenyl-, Aryl- oder Arylalkylrest ist, oder jeder der vorstehenden $R^{17}$-Reste gegebenenfalls mit einer $C_1$-$C_4$-Alkyl-, Amino-, Alkylamino-, Carboxyl-, Alkoxycarbonyl-, Hydroxy-, Alkoxy-, Phenoxy-, Mercapto-, Alkylthio-, Phenylthio-, Halogen- oder Trifluormethylgruppe substituiert sind, Q O oder S ist, und d 0,1 oder 2 ist);

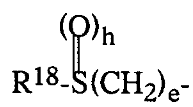

(wobei e 0 bis 10 ist, h 0, 1 oder 2 ist, $R^{18}$ ein Wasserstoffatom, ein Cycloalkyl-, Aryl- oder Alkylrest, von denen jeder gegebenenfalls substituiert ist mit OH, SH, Halogenatomen, Alkoxycarbonylresten oder $NZ_1Z_2$, ein gegebenenfalls mit Halogenatomen, Nitrogruppen, $C_1$-$C_6$-Alkyl-, Alkoxy-, Trifluormethyl-, Amino-, Carboxyl- oder $CO_2$Alkylresten, $CONZ_1Z_2$ oder -$CSNZ_1Z_2$ substituierte Phenylgruppe, ein heterocyclischer 5- oder 6-Ring mit 1 oder 2 Heteroatomen, die N oder S sind, die an den Benzolring kondensiert sein können oder auch nicht, und $Z_1$ und $Z_2$ unabhängig voneinander Wasserstoffatome oder $C_1$-$C_6$-Alkylreste sind);

eine Thiolgruppe; eine Phenylthiogruppe, eine Chlorphenylthiogruppe; eine 4-Thiomorpholinylgruppe;

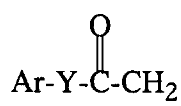

(wobei Ar ein Aryl-, Pyrrolyl- oder Arylrest ist, gegebenenfalls mit $C_1$-$C_4$-Alkyl- oder Alkoxyresten oder Halogenatomen (F, Cl) substituiert, eine Naphthyl-, Biphenyl- oder Thienylgruppe ist, und Y NH oder eine Einfachbindung ist);

$R^{19}SCH_2$- (wobei $R^{19}$ ein Alkyl-, Aryl- oder Arylalkylrest ist);

A-$(CH_2)_f$-NH- (wobei A ein $C_5$-$C_8$-Cycloalkenyl-, Bicycloheptyl-, Bicycloheptenylrest oder ein gesättigter $C_4$-$C_7$ Heterocyclus, der O, S, SO oder $SO_2$ enthält, ist);

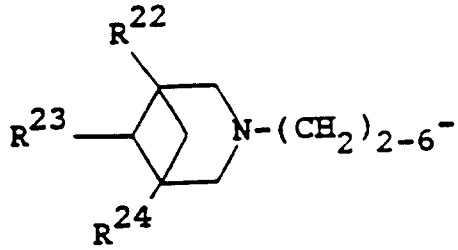

(wobei $R^{22}$ ein Wasserstoffatom, ein $C_1$-$C_{20}$-Alkyl-, Alkoxy- oder Arylrest ist, $R^{23}$ ein Wasserstoffatom, ein $C_1$-$C_{20}$-Alkyl-, Alkoxy-, Aryl-, Halogen- oder Carboxylrest ist, $R^{24}$ ein Wasserstoffatom, ein $C_1$-$C_{20}$-Alkyl- oder Alkoxyrest ist);

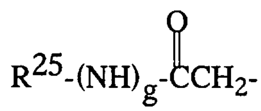

(wobei $R^{25}$ ein alkylsubstituierter Pyrrolyl- oder Phenylrest ist, und g 0 oder 1 ist);

ein aromatisch substituierter mono- oder Biazacyclylalkylrest (der Alkylrest ist mit dem N des Heterocyclus verbunden) (wie die 3-(4-Phenylpiperidino)propylgruppe);

$R^{31}$-Ax-CO-$CH_2$-

(wobei Ax ein Phenyl-, Naphthyl-, mono- oder bicyclischer N-enthaltender Heterocyclus ist, und $R^{31}$ ein Wasserstoffatom, ein Halogenatom, ein $C_1$-$C_6$-Alkyl-oder $C_1$-$C_6$-Alkoxyrest ist);

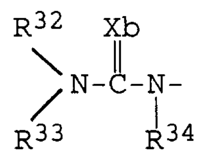

(wobei $R^{32}$ ein Aryl-, Aralkyl- oder Alkylrest ist, $R^{33}$ ein Wasserstoffatom oder ein Arylrest ist, Xb O oder S ist, und $R^{34}$ ein Wasserstoffatom oder ein Alkylrest ist);

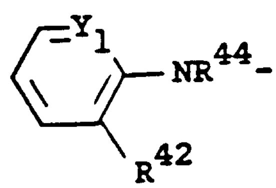

(wobei $R^{42}$ ein Wasserstoffatom, ein Alkylrest oder ein Halogenatom ist, $Y_1$ N, NO oder $NR^{43}Y_2$ ist, wobei $R^{43}$ ein Alkylrest und $Y_2$ ein Halogenatom ist; und $R^{44}$ ein Wasserstoffatom oder ein aliphatischer Acylrest ist);

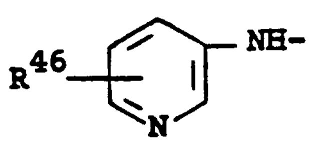

(wobei $R^{46}$ ein Wasserstoffatom, ein Halogenatom oder ein Alkylrest ist);

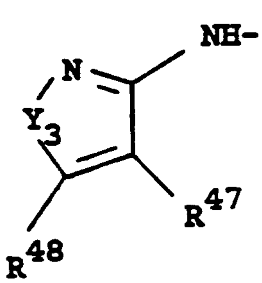

(wobei $Y_3$ O oder NH ist, $R^{47}$ ein Wasserstoffatom, ein Alkylrest oder ein Halogenatom ist, und $R^{48}$ ein Wasserstoffatom oder ein Alkylrest ist);

$R^{50}$-NH-

(wobei $R^{50}$

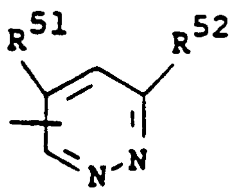

,

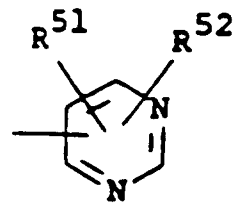

oder

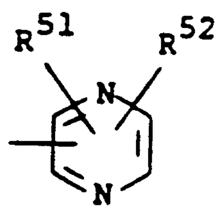

ist, wobei $R^{51}$ und $R^{52}$ ein Wasserstoffatom, ein Halogenatom, ein Alkylrest oder eine Hydroxygruppe sind);

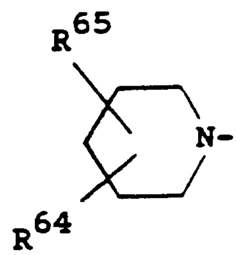

(wobei $R^{64}$ ein Alkylrest und $R^{65}$ ein Wasserstoffatom oder ein Alkylrest ist);

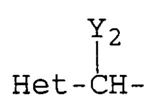

wobei Het ein heteroaromatischer Fünfring mit 2 oder 3 Heteroatomen ist, gegebenenfalls teilweise hydriert und gegebenenfalls mit einem oder mehreren Alkyl-, Alkoxy-, Phenyl-, Cyclohexyl-, Cyclohexylmethyl-, Halogen- oder Aminoresten substituiert, wobei 2 benachbarte Alkylreste gegebenenfalls einen Ring bilden (Het kann nicht Pyrazol sein), und $Y_2$ ein Wasserstoffatom oder ein $C_1$-$C_6$-Alkylrest ist);

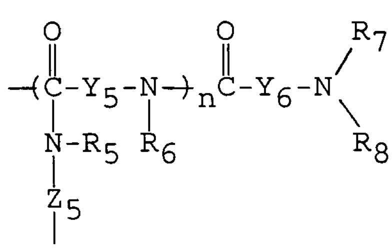

(wobei $Y_4$ ein Wasserstoffatom oder OH ist, $R_5$-$R_8$ unabhängig voneinander Wasserstoffatome oder Niederalkylreste sind, wobei $R_7$ und $Y_6$, oder $R_6$ und $Y_5$, oder $R_5$ und $Z_5$ zusammen mit dem Stickstoffatom, an das sie gebunden sind, einen 5-oder 6-Ring bilden können, $Y_6$ und $Y_5$, die gleich oder verschieden sein können, gegebenenfalls mit aromatischen oder heteroaromatischen Gruppen substituierte $C_1$-$C_6$-Alkylenketten sind, $Z_5$ ein $C_1$ bis $C_6$-Alkylenrest ist, der Heteroatome enthalten kann und gegebenenfalls mit aromatischen oder heteroaromatischen Resten substituiert ist, n 0, 1 oder 2 ist;

$R_{27}$-$Z_9$-

(wobei $R_{27}$ ein Aryl- oder heterocyclischer Rest ist, beide gegebenenfalls mit einer oder mehreren $C_1$-$C_6$-Alkyl-, $C_1$-$C_6$-Alkoxy-, $C_1$-$C_6$-Alkylthio-, halogenierten $C_1$-$C_6$-Alkyl-, Acyl-, Acylaminoresten oder Halogenatomen substituiert, oder $R_{27}$ ein mit einem Heterocyclus, der gegebenenfalls mit einem Acylrest substituiert ist, substituierter $C_1$-$C_6$-Alkylrest ist), $R_{27}$-$Z_9$ $R_{27}$-NHC(=$X_9$), $R_{27}$-C(=O)NH- oder $R_{27}$-$SO_2$-NH- ist (wobei $X_9$ O oder S ist);

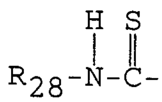

(wobei $R_{28}$ eine Phenyl-, Pyridyl- oder Chinolylgruppe ist, substituiert mit $C_1$-$C_6$-Alkylsulfonylamino-, halogenierten $C_1$-$C_6$-Alkylsulfonylamino-, Arylsulfonylamino- und mono- oder di-$C_1$-$C_6$-Alkylaminoresten);

$R_{29}$-CO-[-$R_{30}$($CH_2$)$_o$CO]$_p$-NH-

(wobei $R_{29}$-CO- ein Rest eines pharmazeutischen Wirkstoffs $R_{29}$-COOH ist, wobei $R_{29}$ ein entzündungshemmendes Mittel oder ein antioncotisches Mittel oder Hormon ist,

$R_{30}$ -NH- oder -O-ist

p 0 oder 1 ist;

o 1-10 ist);

$R_{33}$ -($CH_2$)$_q$-

(wobei $R_{33}$ ein an N gebundener Azabicycloalkylrest mit 3- bis 8-Ringen ist, und q 2 bis 4 ist);

$R_{34}$-($CH_2$)$_r$-

(wobei $R_{34}$ ein an N gebundener arylsubstituierter mono- oder diazacycloaliphatischer Rest ist);

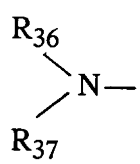

(wobei $R_{36}$ ein Heteroaryl-5-Ring mit 2-4 N-Atomen oder mit 1-2 N-Atomen plus einem O- oder S-Atom ist, der gegebenenfalls an einen Benzo- oder Cyclohexenring kondensiert ist;

$R_{36}$ am C-Atom mit $C_1$-$C_6$-Alkyl-, Phenyl- (gegebenenfalls mit $C_1$-$C_6$-Alkyl-, Alkoxyresten und/oder Halogenatomen substituiert), $C_1$-$C_6$-Alkoxy-, OH, di(Niederalkyl)amino-, $C_1$-$C_6$-Alkylthioresten und/oder Halogenatomen substituiert, und/oder an den N-Atomen mit $C_1$-$C_6$-Alkyl- oder Phenyl-$C_1$-$C_6$-Alkylresten (gegebenenfalls mit Niederalkyl-, Niederalkoxyresten und/oder Halogenatomen substituiert) substituiert sein kann);

$R_{37}$ ein Wasserstoffatom oder ein $C_1$-$C_6$-Alkylrest ist; mit der Maßgabe, daß $R_{37}$ kein Wasserstoffatom ist, wenn $R_{36}$ ein gegebenenfalls substituierter Alkylrest und/oder mit Halogenatomen substituierter 3-Pyrazolyl- oder 3-Isoxazolylrest ist);

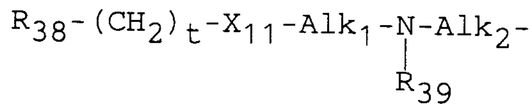

(wobei $R_{38}$ ein aromatischer Rest ist;

t 0-3 ist;

$X_{11}$ O, S (gegebenenfalls oxidiert) oder ein Iminorest ist (gegebenenfalls substituiert mit einem aliphatischen Rest);

$Alk_1$ und $Alk_2$ zweiwertige aliphatische Reste sind; $R_{39}$ ein Wasserstoffatom oder ein einwertiger aliphatischer Rest ist);

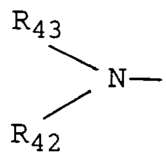

(wobei $R_{42}$ und $R_{43}$ Wasserstoffatome, Alkylreste mit einem bis 22 Kohlenstoffatomen, Cycloalkylreste mit fünf bis sechs Kohlenstoffatomen, Phenylgruppen, Alkylphenylreste mit sieben bis 18 Kohlenstoffatomen, Phenylalkylreste mit sieben bis 18 Kohlenstoffatomen, und zusammen mit dem Stickstoffatom, Piperidin-, Pyrrolidin- und Morpholinreste sind);

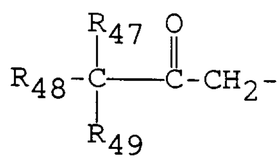

(wobei $R_{47}$ ein gegebenenfalls verzweigter $C_1$-$C_8$-Alkylrest ist, $R_{48}$ und $R_{49}$ jeweils eine Methyl- oder Ethylgruppe sind, und M ein Wasserstoffatom oder ein Kation einer wasserlöslichen Base ist);

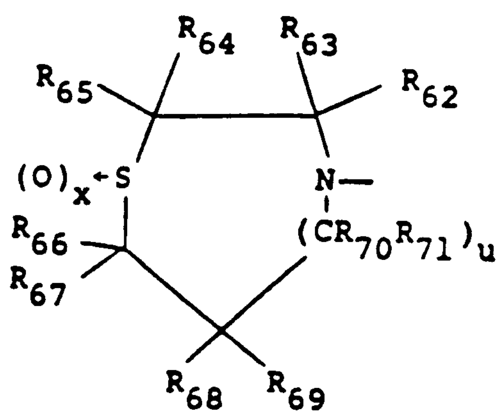

(wobei $R_{62}$-$R_{71}$ Wasserstoffatome, geradkettige, verzweigte oder alicyclische $C_1$-$C_{10}$-Kohlenwasserstoff-, Aryl- oder Aryl-(1-4C)-Alkylreste sind;

x 0 oder 1 ist;

u 0,1 oder 2 ist;

oder $R_{62}$ und $R_{64}$ einen gesättigten aliphatischen 5- bis 7-Ring vervollständigen, der gegebenenfalls mit 1 oder mehreren Alkylresten substituiert ist);

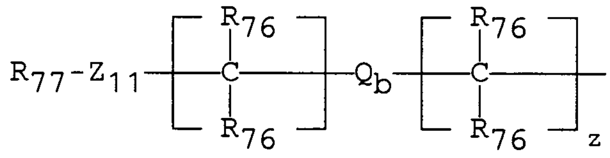

(wobei $Z_{11}$ eine heterocyclische N-haltige 6-Ring-Einheit ist, die ausgewählt ist aus Piperidyl-, Diazinyl- oder Triazinylgruppen;

$Q_b$ eine kovalente Bindung, O, S oder $NR_{76}$ ist;

y, z und y + z ganze Zahlen von 0-10 sind;

$R_{76}$ ein Wasserstoffatom oder ein $C_1$-$C_3$ Alkylrest ist;

$R_{77}$ ein oder mehrere Substituenten ist, ausgewählt aus Wasserstoff-oder Halogenatomen, 1-3C Alkylresten, unsubstituierten Aminogruppen und ihren Amiden, abgeleitet von einer 1-3C Carbonsäure, mono(1-3C Alkyl)aminoresten und ihren Amiden, abgeleitet von einer 1-3C Carbonsäure, di(1-3C Alkyl)-aminoresten, tri(1-3C Alkyl)ammoniumresten, Hydroxygruppen oder ihren Estern, abgeleitet von einer 1-3C Carbonsäure, Ethern mit 1-3C, $CO_2H$ und seine Salze und Ester, abgeleitet von 1-3C Alkoholen, seine Amide, gegebenenfalls mit einem oder zwei 1-3C Alkylresten substituiert, und $NO_2$);

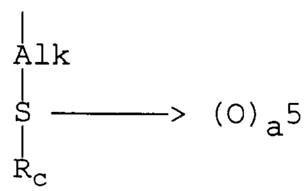

(wobei $R_c$ folgendes darstellt:

einen $C_1$-$C_6$ Alkylrest,

einen $C_5$-$C_7$ Cycloalkylrest,

einen Phenylrest, gegebenenfalls monosubstituiert oder polysubstituiert mit einem Halogenatom, einem $C_1$-$C_6$ Alkylrest oder einer Trifluormethylgruppe, oder

einen 5-Ring- oder 6-Ring-Heterocyclus, der 1 oder 2 Heteroatome, ausgewählt aus Stickstoff und Schwefel, enthält,

Alk einen linearen oder verzweigten $C_1$-$C_6$ Alkylenrest angibt,

$a^5$ 0 oder die ganze Zahl 1 oder 2 darstellt);

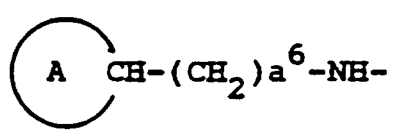

(wobei $a^6$ 0 bis 4 ist, und

Ring A ein 5-8C Cycloalkenyl-, Bicycloheptyl-, Bicycloheptenyl- oder ein gesättigter 4-7C Heterocyclylrest ist, der O, S, SO oder $SO_2$ enthält);

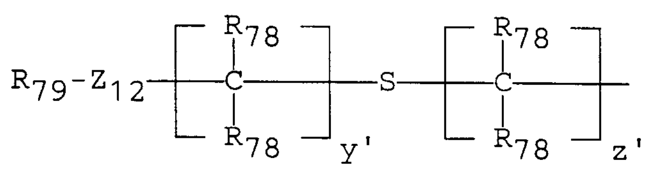

(wobei $Z_{12}$ ein aromatischer 6-Ring ist, der ≧ 1 N Atom(e) enthält; wobei

der Ring gegebenenfalls substituiert ist mit (gegebenenfalls substituierten, gegebenenfalls ungesättigten) 1-6C Alkyl-, (gegebenenfalls substituierten) Aryl-, (gegebenenfalls substituierten) Benzylresten, OH, Halogenatomen, Carbonylresten, Alkoxyresten, $NO_2$, $CONH_2$, (gegebenenfalls substituiertem) $NH_2$ und/oder Carboxylatgruppen, wie ein Pyridin-, Pyridazin-, Pyrimidin- oder Pyrazin-Ring;

$R_{78}$ ein Wasserstoffatom oder ein (gegebenenfalls substituierter, gegebenenfalls unsubstituierter) 1-4C Alkylrest ist;

$R_{79}$ ein Wasserstoffatom, ein (gegebenenfalls substituierter, gegebenenfalls unsubstituierter) 1-6C Alkyl-, (gegebenenfalls substituierter) Aryl-, (gegebenenfalls substituierter) Benzylrest, OH, Halogenatom, Carbonylrest, Alkoxyrest, $NO_2$, $CONH_2$, (gegebenenfalls substituierter) Amino- oder Carboxylatrest ist,

$y' + z^1$ 0 bis 5 ist);

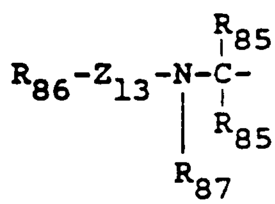

oder

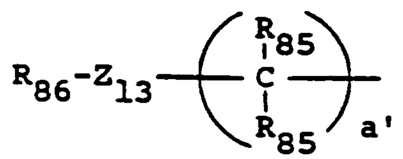

(wobei $Z_{13}$ ein Pyridin-, Pyridazin-, Pyrimidin- oder Pyrazinring ist, gegebenenfalls substituiert mit gegebenenfalls ungesättigten 1-6C Alkyl-, gegebenenfalls substituierten Aryl-, gegebenenfalls substituierten Benzylresten, OH, Halogenatomen, Oxogruppen, Alkoxyresten, $NO_2$, Amidoresten, gegebenenfalls substituiertem $NH_2$ oder Carboxylatgruppen;

$R_{86}$ ein Wasserstoffatom oder ein gegebenenfalls substituierter, gegebenenfalls ungesättigter 1-4C Alkylrest ist;

$R_{86}$ ein oder mehrere Wasserstoffatome, gegebenenfalls substituierte, gegebenenfalls ungesättigte 1-6C Alkyl-, gegebenenfalls substituierte Aryl-, gegebenenfalls substituierte Benzylreste, OH, Halogenatome, Oxogruppen, Alkoxyreste, $NO_2$, Amidoreste, gegebenenfalls substituiertes $NH_2$ oder Carboxylatgruppen ist;

$R_{87}$ ein Wasserstoffatom, ein gegebenenfalls substituierter, gegebenenfalls ungesättigter 1-4C Alkyl- oder Acylrest ist;

a' 1-5 ist);

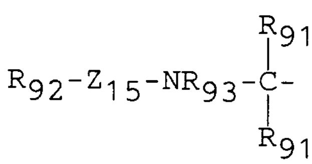

oder

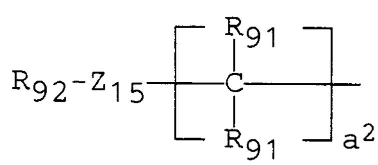

wobei $Z_{15}$ ein aromatischer 6-Ring mit einem oder mehreren N-Atomen ist, wie eine Pyridin-, Pyridazin-, Pyrimidin- oder Pyrazingruppe, wobei der Ring mit einer oder mehreren gegebenenfalls substituierten, gegebenenfalls ungesättigten 1-6C Alkyl-, gegebenenfalls substituierten Aryl-, gegebenenfalls substituierten Benzylresten, OH, Halogenatomen, Carbonyl-, Alkoxyresten, $NO_2$, Amido-, gegebenenfalls substituierten Aminoresten oder Carboxylatgruppen substituiert sein kann);

$R_{91}$ ein Wasserstoffatom oder ein gegebenenfalls substituierter, gegebenenfalls ungesättigter 1-4C Alkylrest ist;

$R_{92}$ ein Wasserstoffatom oder ein oder mehrere Substituenten ist, die aus gegebenenfalls substituierten, gegebenenfalls ungesättigten 1-6C Alkyl-, gegebenenfalls substituierten Aryl-, gegebenenfalls substituierten Benzylresten, OH, Halogenatomen, Carbonyl-, Alkoxyresten, $NO_2$, Amido-, gegebenenfalls substituierten Aminoresten oder Carboxylatgruppen ausgewählt sind;

$R_{93}$ ein Wasserstoffatom, ein gegebenenfalls substituierter, gegebenenfalls ungesättigter 1-4C Alkyl- oder Acylrest ist;

$a^2$ 1 bis 5 ist);

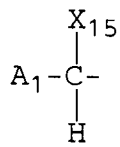

(wobei $X_{15}$ ein Wasserstoffatom, eine Methyl- oder Ethylgruppe ist, und $A_1$ ein Phenylrest, der in para-Stellung mit einer Isobutyl-, Cyclohexyl-, Alkoxy- oder 1-Pyrrolinylgruppe substituiert ist, und gegebenenfalls zusätzlich in meta-Stellung durch Fluor oder Chlor substituiert ist, oder ein Phenylrest, der in meta-Stellung mit Benzoyl-oder Phenoxygruppen substituiert ist, oder ein Phenylrest ist, der in ortho-Stellung mit 2,4-Dichlorphenoxy- oder 2,6-Dichlorphenylaminogruppen substituiert ist);

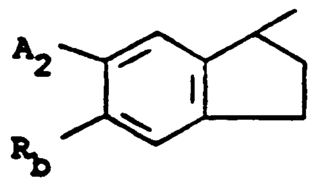

(wobei

$R_b$ eine Cyclohexyl- oder Cyclophenylmethylgruppe ist; und

$A_2$ ein Wasserstoff- oder Chloratom ist);

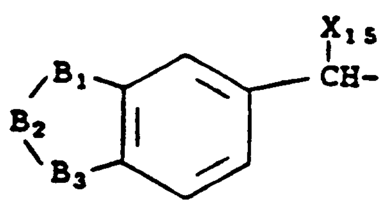

(wobei $X_{15}$ die vorstehend angegebene Bedeutung hat, und

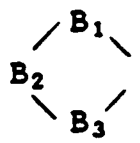

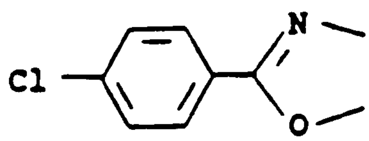

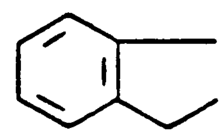

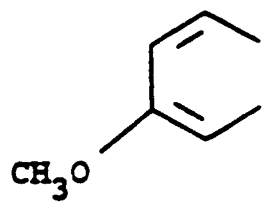

oder

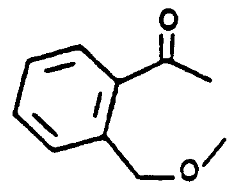

ist

;

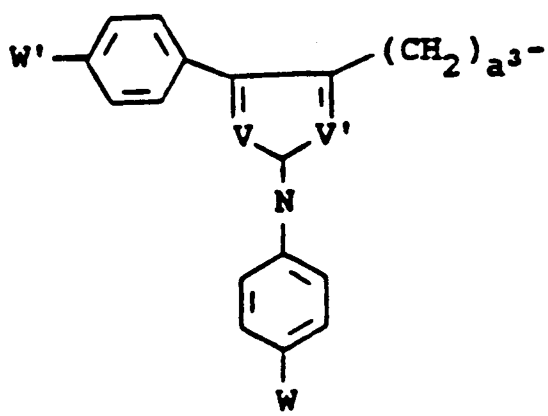

ist, wobei $a^3$ 1, 2 oder 3 ist;

W oder W' gleich oder verschieden sind, und jedes ein Wasserstoff-, Fluor- oder Chloratom ist, und

eines von V und V' ein Stickstoffatom und das andere ein Methinrest ist, gegebenenfalls mit einer Phenylgruppe substituiert, und

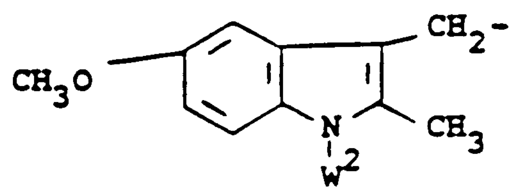

wobei $W^2$ eine p-Chlorbenzoyl- oder Cinnamoylgruppe ist);

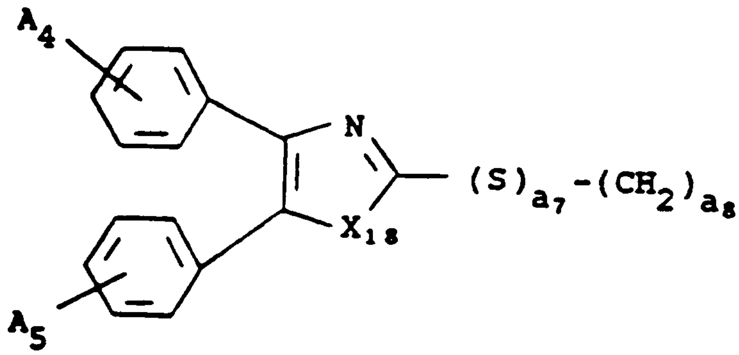

(wobei $A_4$ und $A_5$ gleich oder verschieden sind und ein Wasserstoffatom, OH, ein Niederalkoxyrest oder Halogenatom sind;

$X_{15}$ O, S oder NH ist;

$a_7$ 0 oder 1 ist;

$a_8$ 0 oder eine ganze Zahl von 1-6 ist);

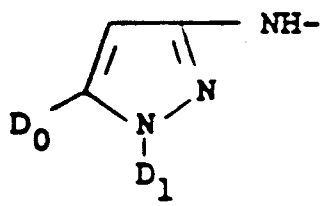

(wobei $D_o$ ein Wasserstoffatom oder ein Alkylrest ist;

$D_1$ ein Wasserstoffatom oder ein $C_1$-$C_6$ Alkylrest ist);

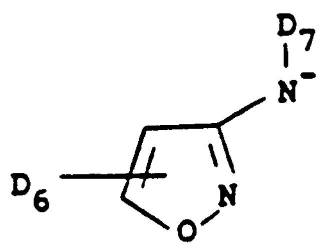

(wobei $D_6$ ein Wasserstoffatom, ein 1-10C Alkyl-, 3-10C Cycloalkyl-, Phenyl-, 2-10C Alkenyl- (gegebenenfalls substituiert mit einer Phenylgruppe) oder Phenyl(1-5C)alkylrest (gegebenenfalls am Ring mit einem 1-5C Alkoxyrest substituiert) ist;

$D_7$ ein Wasserstoffatom oder ein 2-6C Alkanoylrest ist);

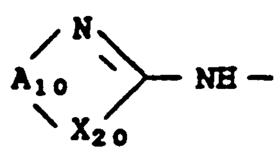

(wobei $A_{10}$ ein Rest der Formel (a)-(c) ist:

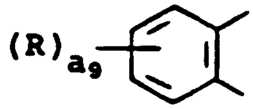 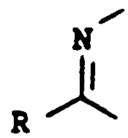 oder 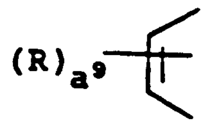

(a) (b) (c)

und $X_{20}$ O, S oder NH ist);

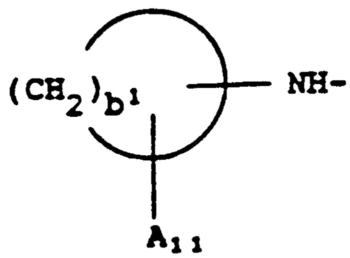

(wobei $A_{11}$ ein Wasserstoffatom oder ein 1-5C Alkylrest ist;
$b_1$ 3-10 ist);

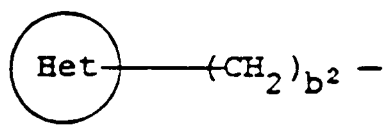

(wobei der Het-Ring ein Rest der Formel (A) oder (B) ist:

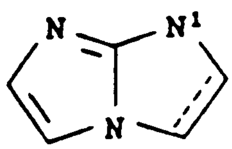 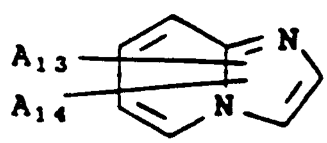

(A) (B)

die gepunktete Linie eine gegebenenfalls vorhandene Doppelbindung darstellt; $A_{13}$, $A_{14}$ Wasserstoffatome, 1-5C Alkylreste, Halogenatome oder OH sind);

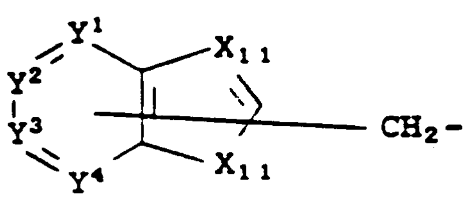

(wobei beide $X_{11}$ N sind oder eines N und das andere CH ist; eines von $Y^1$-$Y^4$ N ist und die übrigen CH sind);

und der andere der Reste $R^5$ und $R^6$ ein Wasserstoff- oder Halogenatom, ein $C_1$-$C_{30}$-Alkyl-, Amino-, Alkylamino-, Dialkylamino-, Uriedo- ($NH_2CO$-$N(R^{38})$)-, wobei $R^{38}$ ein Wasserstoffatom, ein Alkyl-, Benzyl- oder Phenylrest, gegebenenfalls substituiert mit Cl oder $CH_3$ ist; Alkenylamino-, Cycloalkylamino-, Aryloxy-, Pyridinium-, Guanidinium-, Ammonium-, di- und tri-Niederalkanolammonium; Hydroxy-, Arylalkyl-, Alkoxy- oder Alkylaryloxyrest, $-CH_2CO_2H$, $-CH_2PO_3H_2$, $-CH(PO_3H_2)OH$, - $CH_2CO_2C_2H_5$, $-CH_2CH(PO_3H_2)_2$, ein Kohlenwasserstoffrest, der die hier angegebene Bedeutung hat, ein heterocyclischer Rest, der die hier angegebene Bedeutung hat, ein Alkanoylrest, ein $R^6$- oder $R^5$-Rest, die die hier angegebene Bedeutung haben, ein Prodrug-Ester (wie ein 1-Alkanoyloxy)alkylrest, zum Beispiel die Gruppen t-$C_4H_9CO_2CH_2$- und $CH_3CO_2CH_2$-) ist;

wobei wenigstens einer der Reste $R^5$ oder $R^6$ ein lipophiler Rest ist, oder $R^5$ und $R^6$ zu einem carbocyclischen Ring mit 3 bis 12 Kohlenstoffatomen oder einem heterocyclischen Ring mit N-, O- und/oder S-Atomen verbunden sind, wie solche der Formel

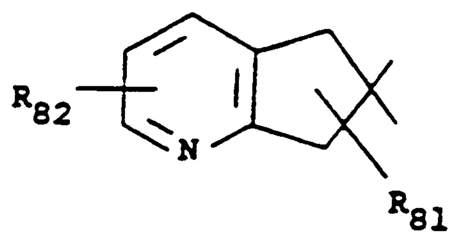

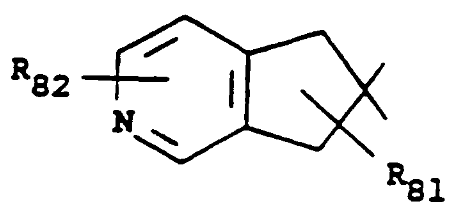

oder 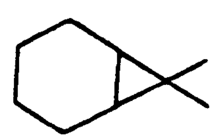

wobei $R_{81}$ und $R_{82}$ ein oder mehrere Substituenten sind, die ausgewählt sind aus Wasserstoffatomen, gegebenenfalls substituierten gesättigten oder ungesättigten 1-6C Alkyl-, gegebenenfalls substituierten Aryl-, gegebenenfalls substituierten Benzyl-, Amidoresten, OH, Halogenatomen, gegebenenfalls substituierten Amino-, Amidoresten, COOH, Carbonyl-, Carboxylat-, Alkoxyresten und $NO_2$,
zur Herstellung eines Arzneimittels zur Behandlung und/oder Vorbeugung von Tumoren, die mit Ras in Verbindung stehen, durch Blockieren der Farnesylierung Ras-oncogener Produkte.

3. Verwendung eines Methylen Phosphonalkyl-Phosphinat Protein-Prenyl Transferase-Inhibitors zur Herstellung eines Arzneimittels zur Behandlung und/oder Vorbeugung von Tumoren, die mit Ras in Verbindung stehen, durch Blockierung der Prenylierung Ras-oncogener Produkte.

4. Verwendung nach Anspruch 3, wobei der Methylen Phosphonalkylphosphinat Protein-Prenyl Transferase-Inhibitor wenigstens einen lipophilen Rest enthält, der ein Rest ist, der wenigstens 6 Kohlenstoffatome enthält und für eine starke Enzym-Inhibitor Bindung und Inhibierung der Enzym-Squalen-Synthetase oder anderer Enzyme auf dem Biosyntheseweg des Cholesterins erforderlich ist.

**5.** Verwendung nach Anspruch 3, wobei die Methylen Phosphonalkylphosphinat-Verbindung die Struktur

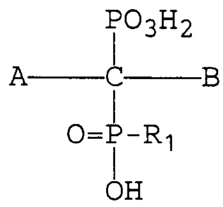

(1)

hat, wobei $R_1$ ausgewählt ist aus einem Wasserstoffatom, einem substituierten Alkyl- und unsubstituierten Alkylrest, und A und B unabhängige Substituenten-Einheiten sind, wobei wenigstens eine von ihnen ein lipophiler Rest ist, der ein Rest ist, der wenigstens 6 Kohlenstoffatome enthält und für eine starke Enzym-Inhibitor Bindung und Inhibierung von Enzym-Protein-Prenyl Transferase(n) erforderlich ist.

**6.** Verwendung nach Anspruch 5, wobei A eine Einheit ist, die ausgewählt ist aus: Wasserstoffatom; Halogenatom; Nitrogruppe; Alkylrest; Heterocyclus; Arylrest; Heteroarylrest; unsubstituierte Aminogruppe und dem Amid davon, abgeleitet von einer Carbonsäure eines Substituenten; Aminogruppe, substituiert mit einem Substituenten, und das Amid davon, abgeleitet von einer Carbonsäure eines Substituenten; Aminorest, unabhängig substituiert mit einem Alkylrest und einem Substituenten; Hydroxygruppe und die Ester davon, abgeleitet von einer Carbonsäure eines Substituenten; Ether mit einem Substituenten; Thiolgruppe und der Thioester davon, abgeleitet von einer Carbonsäure eines Substituenten; Thioether mit einem Substituenten, und das Sulfoxid-und Sulfon-Derivat davon; $-SO_3H$, die pharmazeutisch verträglichen Salze davon, die Ester davon, abgeleitet von einem Alkohol eines Substituenten, das unsubstituierte Amid davon, und das Amid davon, das mit einem oder zwei Alkylresten substituiert ist; $-CO_2H$, die pharmazeutisch verträglichen Salze davon, die Ester davon, abgeleitet von einem Alkohol eines Substituenten, das unsubstituierte Amid davon, und das Amid davon, das mit einem oder zwei Alkylresten substituiert ist; Aldehyd; Keton mit einem Substituenten; Carbamat, unsubstituiert oder substituiert mit einem oder zwei Alkylresten; Peptide, die etwa eine bis 100 Aminosäure-Einheiten haben; oder die A- und B-Einheiten kovalent zu einem Ring mit 3 bis 7 Atomen mit 0 bis 3 Heteroatomen verbunden sind, die ausgewählt sind aus Stickstoff, Schwefel, Phosphor und Sauerstoff, wobei der Ring unsubstituiert oder mit einem oder mehreren der vorstehenden Substituenten A substituiert ist; oder die A-und B-Einheiten durch eine unsubstituierte oder substituierte Alkyleinheit ersetzt sind, die durch eine Doppelbindung an das geminale Kohlenstoffatom gebunden ist; und

B eine Einheit ist, die ausgewählt ist aus: Wasserstoffatom; Halogenatom; unsubstituierte und substituierte Niederalkylreste; unsubstituierte und substituierte Cycloalkylreste mit 3 bis 7 Atomen im Ring; unsubstituierte und substituierte Heterocyclen mit 3 bis 7 Atomen im Ring; unsubstituierte und substituierte Phenylreste; Hydroxygruppe und die Ester davon, abgeleitet von einer Carbonsäure eines $C_1$-$C_6$-Alkylrestes; Thiolgruppe; unsubstituierte Aminogruppe, und das Amid davon, abgeleitet von einer Carbonsäure eines $C_1$-$C_6$-Alkylrestes; Aminorest, substituiert mit einem $C_1$-$C_6$-Alkylrest, und das Amid davon, abgeleitet von einer Carbonsäure eines $C_1$-$C_6$-Alkylrestes; Aminorest, unabhängig substituiert mit zwei $C_1$-$C_6$-Alkylresten ; $-CO_2H$, die pharmazeutisch verträglichen Salze davon, die Ester davon, abgeleitet von einem Alkohol eines $C_1$-$C_6$-Alkylrestes, das unsubstituierte Amid davon, und das substituierte Amid davon, substituiert mit einem oder zwei $C_1$-$C_6$-Alkylresten.

**7.** Verwendung nach Anspruch 6, wobei der Alkylrest substituiert ist mit: Halogenatom, Nitrogruppe, Cyanogruppe, Heterocyclus, Aryl-, Heterarylrest, unsubstituierte Aminogruppe und dem Amid davon, abgeleitet von einer Carbonsäure eines Alkyl-, heterocyclischen, Aryl- oder Heteroarylrest, Aminorest, substituiert mit einem Alkylrest, Heterocyclus, Aryl- oder Heteroarylrest, und das Amid davon, abgeleitet von einer Carbonsäure eines Alkylrestes, Aminorest, unabhängig substituiert mit einem Alkylrest und einem Alkyl-, heterocyclischen, Aryl- oder Heteroarylrest, Hydroxygruppe und die Ester davon, abgeleitet von einer Carbonsäure eines Alkyl-, heterocyclischen, Aryl- oder Heteroarylrestes; Ether mit einem Alkyl-, heterocyclischen, Aryl- oder Heteroarylrest; Thiolgruppe und der Thiolester davon, abgeleitet von einer Carbonsäure eines Alkyl-, heterocyclischen, Aryl- oder Heteroarylreste; Thioether mit einem Alkyl-, heterocyclischen, Aryl- oder Heteroarylrest, und die Sulfoxid- und Sulfon-Derivate

davon, - $SO_3H$, die pharmazeutisch verträglichen Salze davon, die Ester davon, abgeleitet von einem Alkohol eines Alkylrestes, das unsubstituierte Amid davon, und das Amid davon, das mit einem oder zwei Alkylresten substituiert ist, -$CO_2H$, die pharmazeutisch verträglichen Salze davon, die Ester davon, abgeleitet einem Alkohol eines Alkylrestes, das unsubstituierte Amid davon, und das Amid davon, das mit einem oder zwei Alkylresten substituiert ist, $PO_3H_2$, die pharmazeutisch verträglichen Salze davon, die Ester davon, abgeleitet von einem Alkohol eines Alkylrestes, das unsubstituierte Amid davon, und das Amid davon, das mit einem oder zwei Alkylresten substituiert ist, -($R^8$)$PO_2H$ (wobei $R^8$ ein Wasserstoffatom oder unsubstituierter Niederalkylrest ist), die pharmazeutisch verträglichen Salze davon, die Ester davon, abgeleitet von einem Alkohol eines Alkylrestes, das unsubstituierte Amid davon, und das Amid davon, das mit einem oder zwei Alkylresten substituiert ist, Aldehyd, Keton mit einem Alkylrest, Carbamat, unsubstituiert oder substituiert mit einem oder zwei Alkylresten, Peptidylgruppe und Kombinationen davon.

**8.** Verwendung nach Anspruch 7, wobei der Begriff "Heterocyclus" chemisch stabile nichtaromatische Ringe, einschließlich nichtaromatische kondensierte Ringe, mit 5 bis 20 Atomen betrifft, umfassend wenigstens ein Heteroatom, ausgewählt aus Stickstoff, Schwefel, Phosphor und Sauerstoff.

**9.** Verwendung nach Anspruch 8, wobei der Begriff "Aryl" chemisch stabile aromatische Ringe, einschließlich kondensierter aromatischer Ringe, mit 6 bis 20 Kohlenstoffatomen betrifft.

**10.** Verwendung nach Anspruch 6, wobei A eine Einheit ist, die ausgewählt ist aus

(1) Wasserstoffatom;

(2) Halogenatom;

(3) substituierten und unsubstituierten Alkylresten der allgemeinen Struktur:

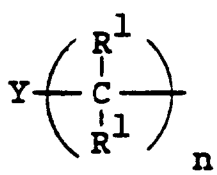

(2)

wobei n eine ganze Zahl von 1 bis 10 ist, vorzugsweise von 1 bis 5, stärker bevorzugt n = 1 oder 2, und am meisten bevorzugt n = 1; alle Reste $R^1$ unabhängig auswählt sind, um chemisch stabile Einheiten ausgewählt aus: Wasserstoffatom, Halogenatom, $C_1$-$C_6$-Alkylrest, unsubstituierte Aminogruppe oder das Amid davon, abgeleitet von einer Carbonsäure eines $C_1$-$C_6$-Alkylrestes, Aminogruppe, substituiert mit einem $C_1$-$C_6$-Alkylrest, oder das Amid davon, abgeleitet von einer Carbonsäure eines Niederalkylrestes, Aminogruppe unabhängig substituiert mit zwei $C_1$-$C_6$-Alkylresten, Hydroxygruppe oder die Ester davon, abgeleitet von einer Carbonsäure eines $C_1$-$C_6$-Alkylrestes, -$CO_2H$ oder die pharmazeutisch verträglichen Salze davon, oder die Ester davon, abgeleitet von einem Alkohol eines $C_1$-$C_6$-Alkylrestes, oder das unsubstituierte Amid davon, oder das Amid davon, substituiert mit einem oder zwei $C_1$-$C_6$-Alkylresten, Ether mit einem $C_1$-$C_6$-Alkylrest, -$PO_3H_2$ oder die pharmazeutisch verträglichen Salze davon, und Nitrogruppe, zu erhalten, oder zwei Reste $R^1$ am gleichen Kohlenstoffatom sind =O oder =$NR^9$ sind (wobei $R^9$ ein $C_1$-$C_6$-Alkylrest ist, oder ein Wasserstoffatom sein kann, wenn ein weiteres Stickstoffatom an das gleiche Kohlenstoffatom wie die =$NR^9$-Einheit gebunden ist), oder zwei Reste $R^1$ an benachbarten Kohlenstoffatomen durch eine zusätzliche Bindung zwischen den Kohlenstoffatomen ersetzt sein können; oder ein Rest $R^1$ am ersten Kohlenstoffatom (von der rechten Seite der vorstehenden Struktur (2)) und B (siehe vorstehende Struktur (1)) durch eine zusätzliche Bindung ersetzt sein können; und Y ein Alkylsubstituent gemäß vorstehender Bedeutung ist; (der chemischen Stabilität der Verbindungen halber, die in der vorliegenden Erfindung verwendet werden, kann $R^1$ nicht so sein, daß ein Halogenatom und ein Sauerstoff-oder Schwefel- oder Stickstoffatom mit einer Einfachbindung an das gleiche Kohlenstoffatom gebunden sind, oder so, daß zwei Sauerstoff- oder Schwefel- oder Stickstoffatome mit einer Einfachbindung an das gleiche Kohlenstoffatom gebunden sind);

(4) Cycloalkylresten mit 4 bis 10 Kohlenstoffatomen; stärker bevorzugt sind Cycloalkylreste mit 5 bis 6 Kohlenstoffatomen;

(5) Heterocyclen mit 5 oder 6 Atomen im Ring; stärker bevorzugt sind Heterocyclen mit einem oder zwei Stickstoffatomen im Ring, noch stärker bevorzugt sind Heterocyclen mit einem Stickstoffatom im Ring; am meisten bevorzugt sind substituierte oder unsubstituierte Piperidinyl-, Pyrrolidinyl-, Piperazinyl- und Morpholinreste;
(6) unsubstituierten und substituierten Phenyl- und Naphtylresten;
(7) unsubstituierten und substituierten Heteroaryl 5- und 6-Ringen mit einem oder zwei Heteroatomen (insbesondere Stickstoff-Heteroatomen); am stärksten bevorzugt ist Pyridinyl;
(8) Amin-haltigen Einheiten der allgemeinen Struktur

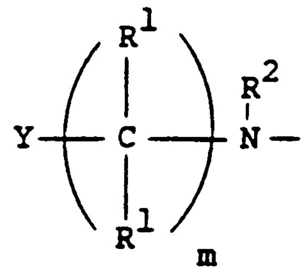

wobei m eine ganze Zahl von 0 bis 10 ist, vorzugsweise von 0 bis 5, stärker bevorzugt 0 oder 1, und am meisten bevorzugt m = 0; $R^1$ und Y wie vorstehend beschrieben sind; und $R^2$ ein Wasserstoffatom, ein $C_1$-$C_6$-Alkyl- oder Acylrest ist, abgeleitet von einer Carbonsäure eines $C_1$-$C_6$-Alkylrestes;
(9) Sauerstoff enthaltenden Einheiten der allgemeinen Sturktur:

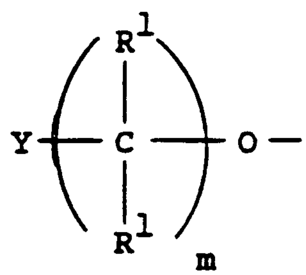

wobei m eine ganze Zahl von 0 bis 10 ist, vorzugsweise von 0 bis etwa 5, stärker bevorzugt 0 oder 1, und am meisten bevorzugt m = 0; und $R^1$ und Y wie vorstehend beschrieben sind;
(10) Schwefel enthaltenden Einheiten der allgemeinen Sturktur:

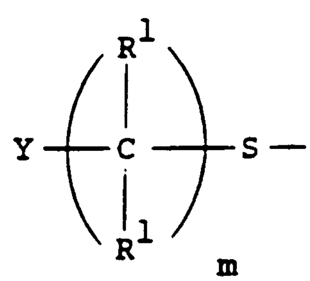

wobei m eine ganze Zahl von 0 bis 10 ist, vorzugsweise von 0 bis 5, stärker bevorzugt 0 oder 1, und am meisten bevorzugt m = 0; und $R^1$ und Y wie vorstehend beschrieben sind; und

(11) Peptid enthaltenden Einheiten der allgemeinen Struktur:

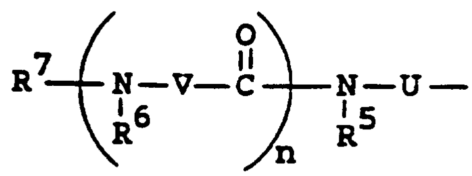

oder

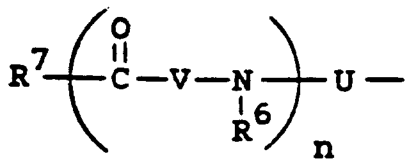

wobei n eine ganze Zahl von 1 bis 100 ist, vorzugsweise von 1 bis 6; $R^5$, alle Reste $R^6$ und $R^7$ unabhängig voneinander Wasserstoffatome oder $C_1$-$C_6$-Alkylreste sind, wobei vorzugsweise $R^5$, alle Reste $R^6$ und $R^7$ Wasserstoffatome sind; U und alle Reste V unabhängig voneinander unsubstituierte oder substituierte $C_1$-$C_6$-Alkylreste (so substituiert, daß die Einheit chemisch stabil ist) sind, oder $R^5$ und U, oder alle Reste $R^6$ und V mit dem enthaltenen Stickstoffatom, an das sie gebunden sind, einen Fünf-oder Sechsring bilden können, der unsubstituiert oder substituiert ist; oder U nicht vorhanden sein kann; vorzugsweise U und alle Reste V, oder Ringe, in denen sie eingebaut sind, Einheiten sind, die in natürlich vorkommenden Aminosäure-Einheiten gefunden werden, d.h. Lysin, Leucin, Isoleucin, Valin, Phenylalanin, Arginin, Histidin, Methionin, Alanin, Asparaginsäure, Threonin, Prolin, Glycin, Serin, Tyrosin, Tryptophan, Glutamin und Cystein.

## Revendications

1. Utilisation d'un bisphosphonate, en tant qu'inhibiteur de protéine-prényl transférase, dans la formule duquel les phosphonates sont réunis par un pont méthylène, et qui comporte au moins un groupe lipophile, fixé au groupe méthylène, qui contient au moins six atomes de carbone, pour la fabrication d'un médicament permettant de traiter et/ou de prévenir les tumeurs liées aux ras en bloquant la prénylation des produits oncogènes ras.

2. Utilisation d'un bisphosphonate de formule

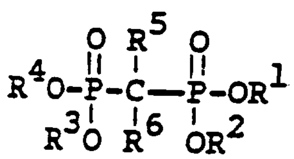

dans laquelle $R^1$, $R^2$, $R^3$ et $R^4$ sont identiques ou différents et sont l'hydrogène, un groupe alkyle; aryle, alkylaryle, arylalkyle ou ammonium, un métal alcalin ou un ester de pro-médicament, dans laquelle au moins l'un de $R^5$ et $R^6$ est un groupe hydrocarbyle ayant au moins 6 atomes de carbone (qui est un groupe alkyle, alcényle, alcynyle, cycloalkyle, aryle, alkylaryle, arylalkyle ou arylalcényle); un groupe hétérocyclique (qui est un groupe succinimidyle, pyridyle, quinalyle, morpholine, furanyle, indolyle, picolinyle, thiophène, imidazole, oxazole, isoxazole, thiazole, pyridine, 1,2,3-triazole, 1,2,4-triazole, benzimidazole, tétrahydrofuranyle, pyrrolidine, pipéridine, hétéroarylméthyle à cycle pentagonal contenant de 2 à 4 atomes d'azote ou bien 1 ou 2 atomes d'azote plus un atome d'oxygène ou de soufre); un groupe alkyle hétérocyclique (dans lequel le groupe hétérocyclique est comme défini ci-dessus, tel que 1-(décahydroquinoléin-3-yl)méthane); amine; alkylamine; dialkylamine; arylalkylaminoalkyle; éthylcarbonyloxyméthylamine; cycloalkyl(alkyl)amine; alcénylamine, cyclcalkylamine, aminocycloalkyle; aminocycloalkylalkyle; N-hydroxy-N-éthylamine; acétylamine; aminoalkyloxyalkyle; un groupe hétéroaryle

pentagonal (benzo- ou cyclohexéno-soudé) contenant 2 à 4 atomes d'azote ou bien 1 ou 2 atomes d'azote plus un atome d'oxygène ou de soufre; $R^8$-X-$(CH_2)_a$- (où $R^8$ est l'hydrogène, un groupe alkyle ou un cycle aromatique hexagonal contenant de l'azote qui est le groupe pyridyle, indanyle, hexahydroindanyle ou picolyle; X est O, NH ou une liaison simple et a est un nombre de 0 à 7);

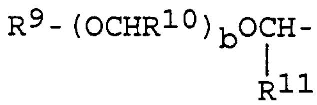

(où $R^9$ est un groupe alkyle en $C_1$-$C_{10}$, un groupe aryle facultativement substitué ou un groupe phénylalkyle ou naphtylalkyle),

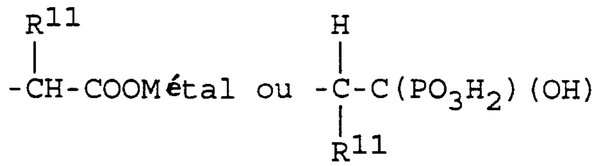

(où $R^{10}$ et $R^{11}$ sont identiques ou différents et sont l'hydrogène ou le groupe méthyle, b est un nombre de 1 à 20));

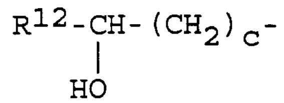

(où $R^{12}$ est l'hydrogène ou un groupe phényle éventuellement substitué par halogène, alkyle ou hydroxy et c est un nombre de 0 à 9);

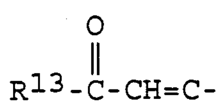

(où $R^{13}$ est un groupe tert-alkyle ($CR^{14}R^{15}R^{16}$ où $R^{14}$ et $R^{15}$ sont indépendamment un groupe alkyle en $C_1$-$C_3$ et $R^{16}$ est un groupe alkyle en $C_1$-$C_{10}$), cycloalkyle, aryle ou hétéroaryle, ou cycloalkyle substitué, aryle substitué ou hétéroaryle substitué, le substituant étant un atome d'halogène ou un groupe alkyle en $C_1$-$C_4$, alcoxy ou dialkylamine);

4-Cl-$C_6H_5$-S-$CH_2$; aryloxy;

$R^{17}$-$(QCH_2CH_2)_d$O- (où $R^{17}$ est un groupe alkyle en $C_1$-$C_{10}$, alcényle en $C_2$-$C_{10}$, aryle ou arylalkyle, ou bien chacun des groupes $R^{17}$ ci-dessus, facultativement substitué par un groupe alkyle en $C_1$-$C_4$, amine, alkylamine, carboxyle, alcoxycarbonyle, hydroxy, alcoxy, phénoxy, mercapto, alkylthio, phénylthio, par un atome d'halogène ou par le groupe trifluorométhyle, Q est O ou S et d est égal à 0, 1 ou 2);

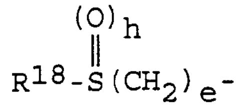

(où e est un nombre de 0 à 10, h est égal à 0, 1 ou 2, $R^{18}$ est l'hydrogène ou un groupe cycloalkyle, aryle ou alkyle, chacun facultativement substitué par OH, SH, halogène, alcoxycarbonyle ou $NZ_1Z_2$, phényle facultativement substitué par halogène, nitro, alkyle en $C_1$-$C_6$, alcoxy, trifluorométhyle, amine, carboxyle, $CO_2$alkyle, -$CONZ_1Z_2$, -$CSNZ_1Z_2$, un radical hétérocyclique pentagonal ou hexagonal contenant 1 ou 2 hétéroatomes qui sont l'azote ou le soufre, ce radical pouvant être ou ne pas être soudé à un noyau benzénique, $Z_1$ et $Z_2$ sont indépendamment l'hydrogène où un groupe alkyle en $C_1$-

$C_6$) ;

thiol; phénylthio; chlorophénylthio; 4-thiomorpholinyle;

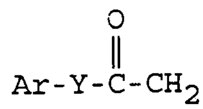

(où Ar est un groupe aryle, pyrrolyle ou aryle facultativement substitué par un groupe alkyle en $C_1$-$C_4$, alcoxy, par un atome d'halogène (fluor, chlore), ou par un groupe naphtyle, biphényle ou thiényle, et Y est NH ou une liaison simple);

$R^{19}SCH_2$- (où $R^{19}$ est un groupe alkyle, aryle ou arylalkyle);

A-$(CH_2)_r$-NH- (où A est un groupe cycloalcényle en $C_5$-$C_8$, bicycloheptyle, bicycloheptényle, un hétérocycle en $C_4$-$C_7$ saturé contenant O, S, SO ou $SO_2$);

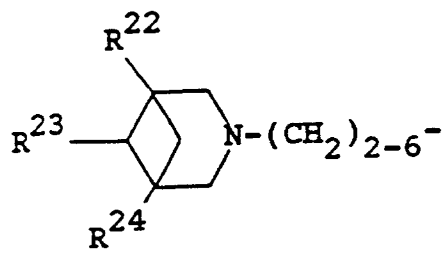

(où $R^{22}$ est l'hydrogène ou un groupe alkyle en $C_1$-$C_{20}$,alcoxy ou aryle, $R^{23}$ est l'hydrogène ou un groupe alkyle en $C_1$-$C_{20}$, alcoxy, aryle, un atome d'halogène ou un groupe carboxyle, $R^{24}$ est l'hydrogène ou un groupe alkyle en $C_1$-$C_{20}$ ou alcoxy);

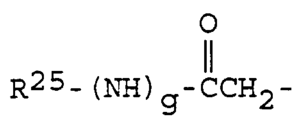

(où $R^{25}$ est un groupe pyrrolyle (alkyl-substitué) ou phényle et g est égal à 0 ou 1);

un groupe mono- ou biazacyclylalkyle substitué par aromatique (le groupe alkyle est lié à l'atome d'azote dans l'hétérocycle) (tel que le groupe 3-(4-phénylpipéridino)propyle);

$R^{31}$-Ax-CO-$CH_2$-

(où Ax est un groupe phényle ou naphtyle ou un hétérocycle azoté mono- ou bicyclique et $R^{31}$ est l'hydrogène, un halogène ou un groupe alkyle en $C_1$-$C_6$ ou alcoxy en $C_1$-$C_6$);

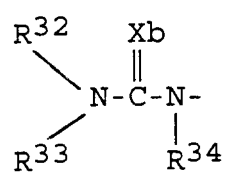

(où $R^{32}$ est un groupe aryle, aralkyle ou alkyle, $R^{33}$ est l'hydrogène ou un groupe aryle, Xb est O ou S, et $R^{34}$ est l'hydrogène ou un groupe alkyle);

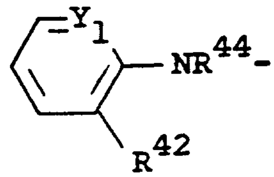

(où $R^{42}$ est l'hydrogène, un groupe alkyle ou un atome d'halogène, $Y_1$ est N, NO, ou $NR^{43}Y_2$ où $R^{43}$ est un groupe alkyle et $Y_2$ est un halogène; et $R^{44}$ est l'hydrogène ou un groupe acyle aliphatique);

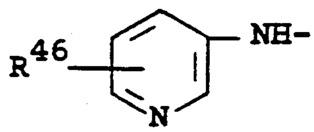

(où $R^{46}$ est l'hydrogène, un halogène ou un groupe alkyle);

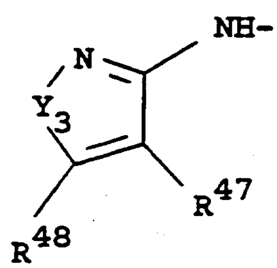

(où $Y_3$ est O ou NH, $R^{47}$ est l'hydrogène, un groupe alkyle ou un halogène, et $R^{48}$ est l'hydrogène ou un groupe alkyle);

$R^{50}$-NH-

(où $R^{50}$ est

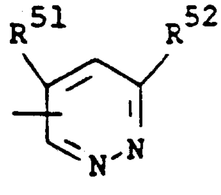

,

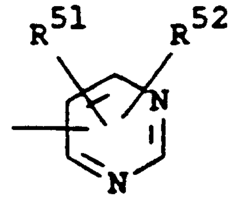

ou

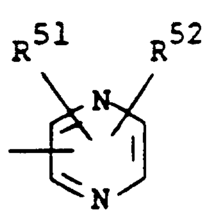

où $R^{51}$ et $R^{52}$ sont l'hydrogène, un halogène, ou un groupe alkyle ou hydroxy);

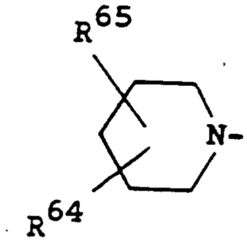

(où $R^{64}$ est un groupe alkyle et $R^{65}$ est l'hydrogène ou un groupe alkyle;

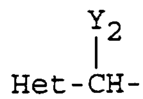

où Het est un cycle pentagonal hétéroaromatique à 2 ou 3 hétéroatomes, qui est facultativement partiellement hydrogéné et facultativement substitué par un ou plusieurs groupes alkyle, alcoxy, phényle, cyclohexyle, cyclohexylméthyle, atomes d'halogène ou groupes amine, 2 groupes alkyle adjacents formant facultativement ensemble un cycle (Het ne peut être le pyrazole), et $Y_2$ est l'hydrogène ou un groupe alkyle en $C_1$-$C_6$);

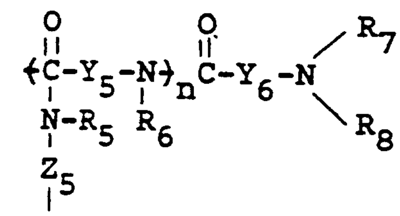

(où $Y_4$ est H ou OH, $R_5$ à $R_8$ sont indépendamment l'hydrogène ou des groupes alkyle inférieur, de sorte que $R_7$ et $Y_6$ ou $R_6$ et $Y_5$ ou $R_5$ et $Z_5$ peuvent former, avec l'atome d'azote auquel ils sont fixés, un cycle pentagonal ou hexagonal, $Y_6$ et $Y_5$, qui peuvent être identiques ou différents, sont des chaînes alkylène en $C_1$-$C_6$ facultativement substituées par des radicaux aromatiques ou hétéroaromatiques, $Z_5$ est un groupe alkylène en $C_1$ à $C_6$ qui peut comporter des hétéroatomes et qui est facultativement substitué par des groupes aromatiques ou hétéroaromatiques, n est égal à 0, 1 ou 2;

$R_{27}$-$Z_9$-

(où $R_{27}$ est un groupe aryle ou hétérocyclyle, qui sont tous deux facultativement substitués par un ou plusieurs groupes alkyle en $C_1$-$C_6$, alcoxy en $C_1$-$C_6$, alkylthio en $C_1$-$C_6$, halo-alkyle en $C_1$-$C_6$, acyle, acylamine ou atomes d'halogène, ou bien $R_{27}$ est un groupe alkyle en $C_1$-$C_6$ substitué par un groupe hétérocyclyle qui est facultativement substitué par un groupe acyle); $R_{27}$-$Z_9$ est $R_{27}$-NHC(=$X_9$), $R_{27}$-C(=O)NH-, $R_{27}$-$SO_2$-NH- (où $X_9$ est O ou S);

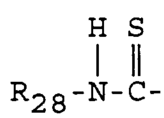

(où $R_{28}$ est un groupe phényle, pyridyle ou quinolyle substitué par un groupe alkylsulfonylamine en $C_1$-$C_6$, halo-alkylsulfonylamine en $C_1$-$C_6$, arylsulfonylamine ou mono- ou di-alkylamine en $C_1$-$C_6$);

$R_{29}$-CO-[-$R_{30}$($CH_2$)$_o$CO-]$_p$-NH-

(où $R_{29}$-CO- est un reste de composé à action pharmaceutique $R_{29}$-COOH, où $R_{29}$ est un anti-inflammatoire, un anti-oncotique ou une hormone,

$R_{30}$ est -NH- ou -O-

p est égal à 0 ou 1;

o est un nombre de 1 à 10);

$R_{33}$-($CH_2$)$_q$-

(où $R_{33}$ est un groupe azabicycloalkyle lié à N avec des cycles de 3 à 8 chaînons et q est un nombre de 2 à 4);

$R_{34}$-($CH_2$)$_r$-

(où $R_{34}$ est un groupe mono- ou diazacycloaliphatique aryl-substitué lié à N);

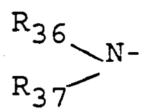

(où $R_{36}$ est un groupe hétéroaryle pentagonal avec 2 à 4 atomes d'azote ou bien avec 1 ou 2 atomes d'azote plus un atome d'oxygène ou de soufre, facultativement soudé à un noyau benzène ou cyclohexène;

$R_{36}$ peut être un atome C substitué par un groupe alkyle en $C_1$-$C_6$, phényle (facultativement substitué par alkyle en $C_1$-$C_6$, alcoxy et/ou halogène), alcoxy en $C_1$-$C_6$, OH, di(alkyl inférieur)amine, alkylthio en $C_1$-$C_6$ et/ou halogène, et/ou un atome N substitué par un groupe alkyle en $C_1$-$C_6$ ou phényl-alkyle en $C_1$-$C_6$ (facultativement substitué par alkyle inférieur, alcoxy inférieur et/ou halogène);

$R_{37}$ est l'hydrogène ou un groupe alkyle en $C_1$-$C_6$; sous réserve que $R_{37}$ ne soit pas l'hydrogène si $R_{36}$ est un groupe alkyle facultativement substitué et/ou un groupe 3-pyrazolyle ou 3-isoxazolyle halo-substitué);

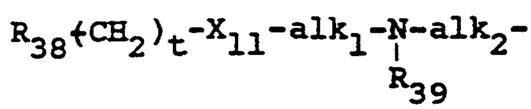

(où $R_{38}$ est un reste aromatique;

t est un nombre de 0 à 3;

$X_{11}$ est O S (facultativement oxydé) ou un groupe imine (facultativement substitué par un groupe aliphatique);

$alk_1$ et $alk_2$ sont des groupes aliphatiques divalents; $R_{39}$ est l'hydrogène ou un groupe aliphatique monovalent);

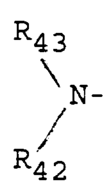

(où $R_{42}$ et $R_{43}$ sont l'hydrogène, un groupe alkyle ayant de 1 à 22 atomes de carbone, un groupe cycloalkyle ayant 5 ou 6 atomes de carbone, un groupe phényl alkylphényle ayant de 7 à 18 atomes de carbone, un groupe phénylalkyle ayant de 7 à 18 atomes de carbone ou bien forment, avec l'atome d'azote, un groupe pipéridine, pyrrolidine ou morpholine);

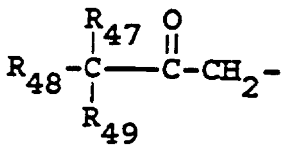

(où $R_{47}$ est un groupe alkyle en $C_1$-$C_8$ facultativement ramifié,

$R_{48}$ et $R_{49}$ sont chacun le groupe méthyle ou éthyle, et

M est l'hydrogène ou un cation d'une base soluble dans l'eau);

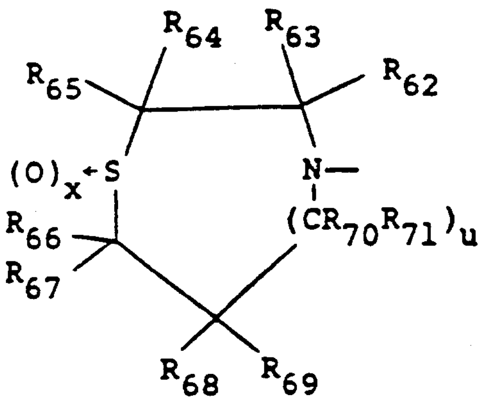

(où $R_{62}$-$R_{71}$ sont des atomes d'hydrogène, des groupes hydrocarbyle de 1 à 10 atomes de carbone, à chaîne droite, ramifiée ou alicyclique, ou des groupes aryle ou aryl-alkyle en $C_1$-$C_4$;

x est égal à 0 ou 1;

u est égal à 0, 1 ou 2;

ou bien $R_{62}$ et $R_{64}$ peuvent compléter un cycle aliphatique saturé de 5 à 7 chaînons, facultativement substitué par un ou plusieurs groupes alkyle);

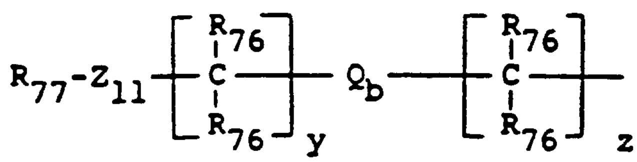

(où $Z_{11}$ est un hétérocycle à 6 chaînons contenant de l'azote, choisi entre pipéridinyle, diazinyle et triazinyle);

$Q_b$ est une liaison covalente, O, S ou $NR_{76}$;

y, z, et y + z sont des nombres entiers de 0 à 10;

$R_{76}$ est l'hydrogène ou un groupe alkyle en $C_1$-$C_3$;

$R_{77}$ est formé par un ou plusieurs substituants choisis entre l'hydrogène, les halogènes, les groupes alkyle de 1 à 3 atomes de carbone, le groupe amine non substitué et son amide dérivé d'un acide carboxylique en $C_1$-$C_3$, un groupe mono(alkyl en $C_1$-$C_3$)amine et son amide dérivé d'un acide carboxylique en $C_1$-$C_3$, un groupe di(alkyl en $C_1$-$C_3$)amine, tri(alkyl en $C_1$-$C_3$)ammonium, hydroxy, ou son ester dérivé d'un acide carboxylique en $C_1$-$C_3$, éther ayant de 1 à 3 atomes de carbone, $CO_2H$ et ses sels et esters dérivés d'alcools en $C_1$-$C_3$, son amide facultativement substitué par un ou deux groupes alkyle en $C_1$-$C_3$, et $NO_2$);

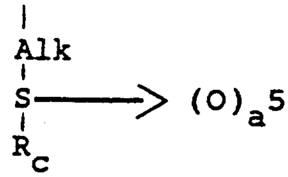

(où $R_c$ représente:

un groupe alkyle en $C_1$-$C_6$,

un groupe cycloalkyle en $C_5$-$C_7$,

un groupe phényle facultativement monosubstitué ou polysubstitué par un halogène, un groupe alkyle en $C_1$-$C_6$ ou un groupe trifluorométhyle, ou bien

un hétérocycle à 5 ou 6 chaînons contenant 1 ou 2 hétéroatomes choisis entre l'azote et le soufre,

Alk désigne un groupe alkylène en $C_1$-$C_6$ linéaire ou ramifié,

$a^5$ représente 0 ou le nombre entier 1 ou 2);

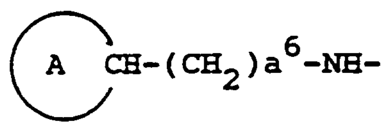

(où $a^6$ est un nombre de 0 à 4 et le cycle A est un groupe cycloalcényle de 5 à 8 atomes de carbone, bicycloheptyle, bicycloheptényle ou hétérocyclyle saturé de 4 à 7 atomes de carbone contenant O, S, SO ou $SO_2$);

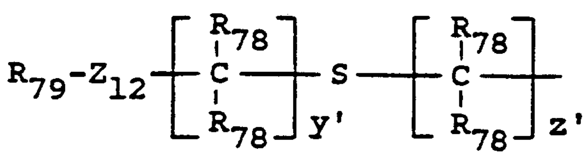

(où $Z_{12}$ est un noyau aromatique à 6 chaînons contenant au moins un atome d'azote); où:

le noyau est facultativement substitué par un groupe alkyle de 1 à 6 atomes de carbone (facultativement substitué, facultativement insaturé), un groupe aryle facultativement substitué, un groupe benzyle facultativement substitué, OH, halogène, carbonyle, alcoxy, $NO_2$, $CONH_2$, $NH_2$ facultativement substitué et/ou carboxylate, tel que le noyau pyridine, pyridazine, pyrimidine ou pyrazine;

$R_{78}$ est l'hydrogène ou un groupe alkyle de 1 à 4 atomes de carbone facultativement substitué et facultativement insaturé;

$R_{79}$ est l'hydrogène ou un groupe alkyle de 1 à 6 atomes de carbone facultativement substitué, facultativement insaturé, un groupe aryle facultativement substitué, un groupe benzyle facultativement substitué, OH, un halogène, carbonyle, alcoxy, $NO_2$, $CONH_2$, amine facultativement substitué ou carboxylate,

y' + z' est un nombre de 0 à 5);

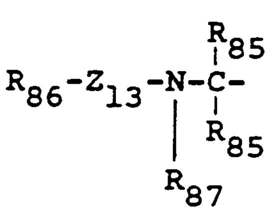

ou

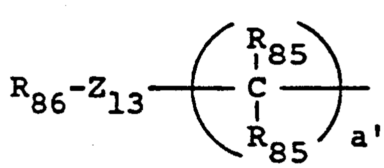

(où $Z_{13}$ est un noyau pyridine, pyridazine, pyrimidine ou pyrazine, facultativement substitué par un groupe alkyle de 1 à 6 atomes de carbone facultativement insaturé, un groupe aryle facultativement substitué, un groupe benzyle facultativement substitué, OH, halogène, oxo, alcoxy, $NO_2$, amide, $NH_2$ facultativement substitué ou carboxylate;

$R_{85}$ est l'hydrogène ou un groupe alkyle de 1 à 4 atomes de carbone, facultativement substitué, et facultativement insaturé;

$R_{86}$ est formé par un ou plusieurs des atomes et groupes suivants: hydrogène, alkyle de 1 à 6 atomes de carbone facultativement substitué et facultativement insaturé, aryle facultativement substitué, benzyle facultativement substitué, OH, halogène, oxo, alcoxy, $NO_2$, amide, $NH_2$ facultativement substitué ou carboxylate;

$R_{87}$ est l'hydrogène, un groupe alkyle de 1 à 4 atomes de carbone facultativement substitué et facultativement insaturé, ou un groupe acyle;

a' est un nombre de 1 à 5);

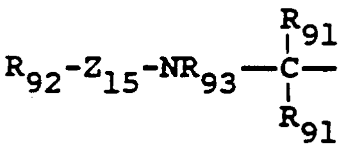

ou

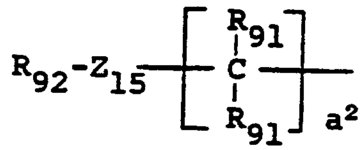

(où $Z_{15}$ est un noyau aromatique à 6 chaînons contenant un ou plusieurs atomes d'azote, tel que pyridine, pyridazine, pyrimidine ou pyrazine, lequel noyau est éventuellement substitué par un ou plusieurs groupes alkyle de 1 à 6 atomes de carbone facultativement substitués et facultativement insaturés, un groupe aryle facultativement substitué, un groupe benzyle facultativement substitué, OH, halogène, carbonyle, alcoxy, $NO_2$, amide, amine facultativement substitué ou carboxylate;

$R_{91}$ est l'hydrogène ou un groupe alkyle de 1 à 4 atomes de carbone facultativement substitué et facultativement insaturé;

$R_{92}$ est l'hydrogène ou est formé par un ou plusieurs substituants choisis entre un groupe alkyle de 1 à 6 atomes facultativement substitué et facultativement insaturé, un groupe aryle facultativement substitué, un groupe benzyle facultativement substitué, OH, halogène, carbonyle, alcoxy, $NO_2$, amide, amine facultativement substitué ou carboxylate;

$R_{93}$ est l'hydrogène, un groupe alkyle de 1 à 4 atomes de carbone facultativement substitué et facultativement insaturé, ou un groupe acyle;

$a^2$ est un nombre de 1 à 5);

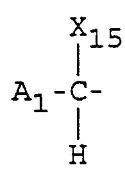

(où $X_{15}$ est l'hydrogène ou le groupe méthyle ou éthyle, et $A_1$ est un groupe phényle substitué en position para par un groupe isobutyle, cyclohexyle, alcoxy ou 1-pyrrolinyle, et facultativement substitué en outre en position méta par un atome de fluor ou de chlore, ou un groupe phényle substitué en position méta par un groupe benzoyle ou phénoxy, ou un groupe phényle substitué en position ortho par le groupe 2,4-dichlorophénoxy ou 2,6-dichlorophénylamine);

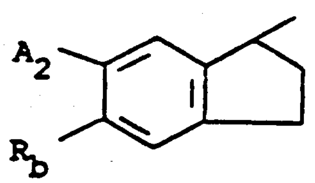

(où

$R_b$ est le, groupe cyclohexyle ou cyclophénylméthyle; et

$A_2$est l'hydrogène ou le chlore);

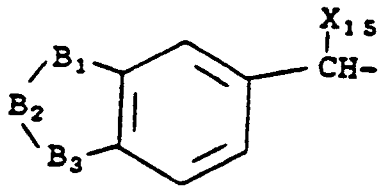

(où $X_{15}$ est tel que défini ci-dessus, et

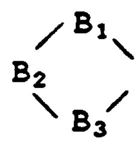

est

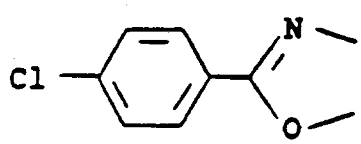

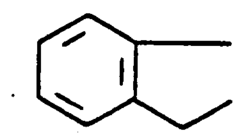

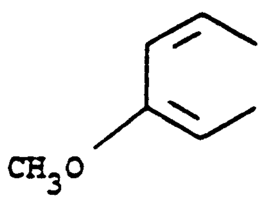

ou

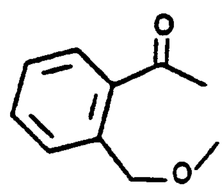

;

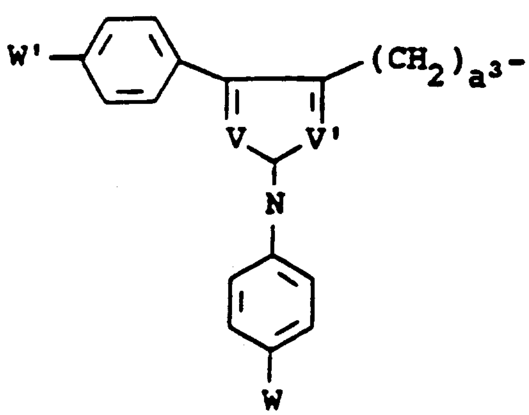

où $a^3$ est égal à 1, 2 ou 3;

W et W' sont identiques ou différents et chacun d'eux est un atome d'hydrogène, de fluor ou de chlore, et

l'un de V et V' est l'azote et l'autre est un reste méthyne facultativement substitué par un groupe phényle, et

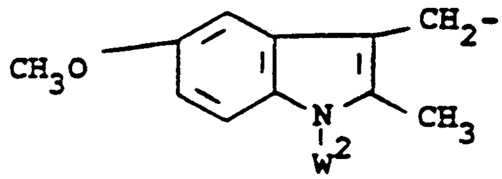

où $W^2$ est le groupe para-chlorobenzoyle ou cinnamoyle);

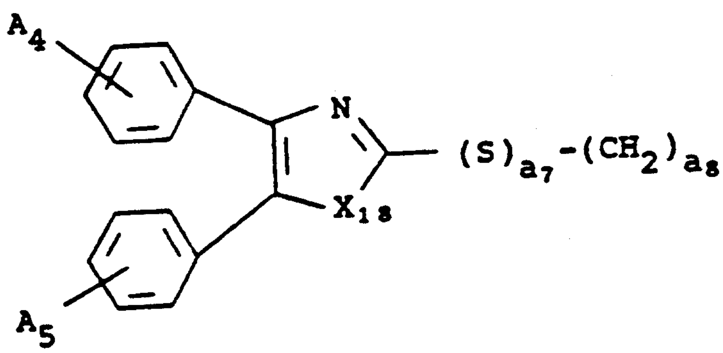

(où $A_4$ et $A_5$ sont identiques ou différents et sont H, OH, alcoxy inférieur ou halogène;
$X_{15}$ est O, S ou NH;
$a_7$ est égal à 0 ou 1;
$a_8$ est égal à 0 ou un nombre entier de 1 à 6);

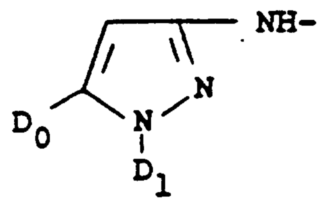

(où $D_0$ est l'hydrogène ou un groupe alkyle;
$D_1$ est l'hydrogène ou un groupe alkyle en $C_1$-$C_6$);

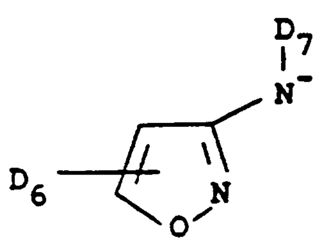

(où $D_6$ est l'hydrogène, un groupe alkyle de 1 à 10 atomes de carbone, cycloalkyle de 3 à 10 atomes de carbone, phényle, alcényle de 2 à 10 atomes de carbone (facultativement substitué par un groupe phényle) ou phényl(alkyl en $C_1$-$C_5$) (facultativement substitué sur le cycle par un groupe alcoxy en $C_1$-$C_5$);
$D_7$ es l'hydrogène ou un groupe alcanoyle en $C_2$-$C_6$);

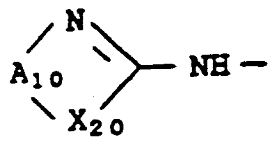

(où $A_{10}$ est un groupe de formule (a)-(c):

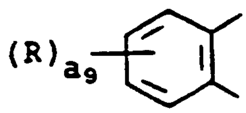

(a)

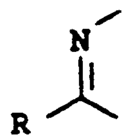

(b)

ou

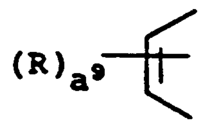

(c)

et $X_{20}$ est O, S ou NH);

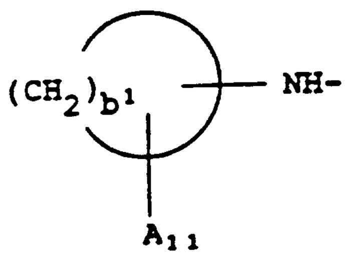

(où $A_{11}$ est l'hydrogène ou un groupe alkyle en $C_1$-$C_5$; $b_1$ est un nombre de 3 à 10);

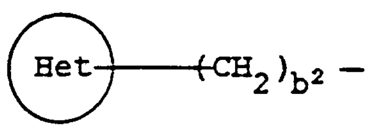

(où le cycle Het est un groupe de formule (A) ou (B):

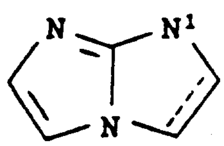

(A)

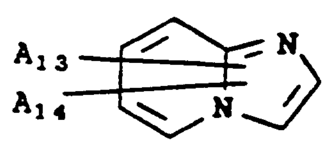

(B)

le trait interrompu représente une double liaison facultative; $A_{13}$ et $A_{14}$ sont l'hydrogène, un groupe alkyle en $C_1$-$C_5$, un atome d'halogène ou OH);

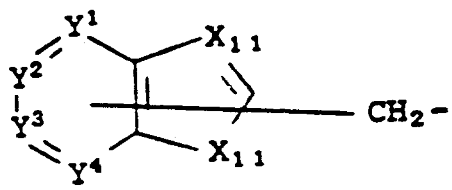

(où $X_{11}$ est dans les deux cas l'azote, ou bien l'un d'eux est l'azote et l'autre est CH; un de $Y^1$-$Y^4$ est l'azote et les autres sont CH);

et l'autre de $R^5$ et $R^6$ est l'hydrogène, un halogène, un groupe alkyle en $C_1$-$C_{30}$, amine, alkylamine, dialkylamine, uréido ($NH_2CO$-$N(R^{38})$- où $R^{38}$ est l'hydrogène, un groupe alkyle, benzyle, phényle facultativement substitué par le chlore ou par $CH_3$); alcénylamine, cycloalkylamine, aryloxy, pyridinium, guanidinium, ammonium, di-et tri-(alcanol inférieur) ammonium, hydroxy, arylalkyle, alcoxy, alkylaryloxy, $-CH_2CO_2H$, $-CH_2PO_3H_2$, $-CH(PO_3H_2)(OH)$, $-CH_2CO_2C_2H_5$, $-CH_2CH(PO_3H_2)_2$, un radical hydrocarbyle tel qu'il est défini ici, un radical hétérocyclique tel qu'il est défini ici, un groupe alcanoyle, un radical $R^6$ ou $R^5$ tel qu'il est défini ici, un ester de pro-médicament (tel que le groupe (1-alcanoyloxy)alkyle, par exemple $t$-$C_4H_9CO_2CH_2$-, $CH_3CO_2CH_2$-);

au moins l'un de $R^5$ et $R^6$ étant un groupe lipophile, ou bien $R^5$ et $R^6$ pouvant être réunis pour former un noyau carbocyclique contenant de 3 à 12 atomes de carbone ou un noyau hétérocyclique contenant des atomes d'azote, d'oxygène et/ou de soufre, tels ceux de formule

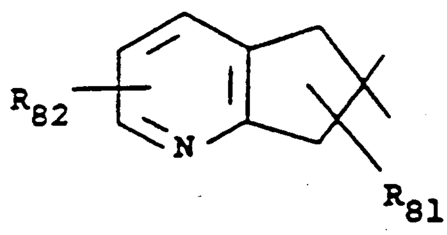

ou

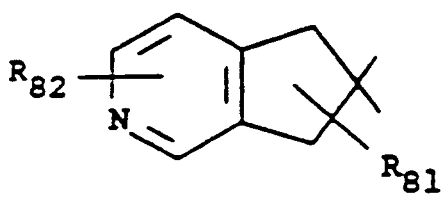

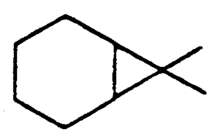

où $R_{81}$ et $R_{82}$ sont chacun un ou plusieurs substituants choisis entre l'hydrogène, un groupe alkyle en $C_1$-$C_6$ facultativement substitué, saturé ou insaturé, un groupe aryle facultativement substitué, un groupe benzyle facultativement substitué, un groupe amide, OH, un halogène, un groupe amine facultativement substitué, un groupe amide, COOH, un groupe carbonyle, carboxylate, alcoxy et $NO_2$, pour la fabrication d'un médicament permettant de traiter et/ou de prévenir les tumeurs liées aux ras en bloquant la farnésylation des produits oncogènes ras.

3. Utilisation d'un méthylène phosphonoalkylphosphinate, en tant qu'inhibiteur de protéine-prényl transférase, pour la fabrication d'un médicament permettant de traiter et/ou de prévenir les tumeurs liées aux ras en bloquant la prénylation des produits oncogènes ras.

4. Utilisation selon la revendication 3, dans laquelle le méthylène phosphonoalkylphosphinate inhibiteur de protéine-prényl transférase comporte au moins un groupe lipophile qui est un groupe qui contient au

moins 6 atomes de carbone et qui est exigé pour une forte fixation de l'inhibiteur de l'enzyme et pour l'inhibition de la squalène synthétase ou d'autres enzymes dans le processus de biosynthèse du cholestérol.

**5.** Utilisation selon la revendication 3, dans laquelle le méthylène phosphonoalkylphosphinate a pour formule

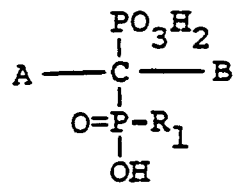

(1)

dans laquelle $R_1$ est choisi entre l'hydrogène, un groupe alkyle substitué et un groupe alkyle non substitué, et A et B sont des substituants indépendants, dont au moins un est un groupe lipophile qui est un groupe qui contient au moins 6 atomes de carbone et qui est exigé pour une forte fixation de l'inhibiteur de l'enzyme et pour l'inhibition de la ou des protéine-prényl transférase(s).

**6.** Utilisation selon la revendication 5, dans laquelle A est un élément choisi entre les suivants: hydrogène; halogène; nitro; alkyle; hétérocycle; aryle; hétéroaryle; amine non substitué, et son amide dérivé d'un acide carboxylique d'un groupe substituant; amine substitué par un groupe substituant, et son amide dérivé d'un acide carboxylique d'un groupe substituant; amine substitué indépendamment par un groupe alkyle et un groupe substituant; hydroxy, et son ester dérivé d'un acide carboxylique d'un groupe substituant; éther portant un groupe substituant; thiol, et son ester de thiol dérivé d'un acide carboxylique d'un groupe substituant; thioéther portant un groupe substituant, ainsi que son sulfoxyde et sa sulfone; $-SO_3H$, ses sels pharmaceutiquement acceptables, son ester dérivé d'un alcool d'un groupe substituant, son amide non substitué, et son amide substitué par un ou deux groupes alkyle; $-CO_2H$, ses sels pharmaceutiquement acceptables, son ester dérivé d'un alcool d'un groupe substituant, son amide non substitué, et son amide substitué par un ou deux groupes alkyle; aldéhyde; cétone portant un groupe substituant; carbamate, non substitué ou substitué par un ou deux groupes alkyle; peptides comportant environ une à 100 séquences d'acides aminés; ou bien les éléments A et B sont liés par covalence de façon à former un cycle ayant de 3 à 7 atomes, avec de 0 à 3 hétéroatomes choisis dans le groupe composé de l'azote, du soufre, du phosphore et de l'oxygène, le cycle n'étant pas substitué ou bien étant substitué par un ou plusieurs des substituants précités de A; ou bien les éléments A et B sont remplacés par un groupe alkyle non substitué ou substitué fixé au carbone géminé par une double liaison; et

B est un élément choisi entre les suivants: hydrogène; halogène; alkyle inférieur éventuellement substitué; cycloalkyle éventuellement substitué ayant de 3 à 7 atomes dans le cycle; hétérocycle éventuellement substitué ayant de 3 à 7 atomes dans le cycle; phényle éventuellement substitué; hydroxy, et son ester dérivé d'un acide carboxylique d'un groupe alkyle en $C_1$-$C_6$; thiol; amine non substitué, et son amide dérivé d'un acide carboxylique d'un groupe alkyle en $C_1$-$C_6$; amine substitué par un groupe alkyle en $C_1$-$C_6$, et son amide dérivé d'un acide carboxylique d'un groupe alkyle en $C_1$-$C_6$; amine substitué indépendamment par deux groupes alkyle en $C_1$-$C_6$ ; $-CO_2H$, ses sels pharmaceutiquement acceptables, son ester dérivé d'un alcool d'un groupe alkyle en $C_1$-$C_6$, son amide non substitué, et son amide substitué par un ou deux groupes alkyle en $C_1$-$C_6$.

**7.** Utilisation selon la revendication 6, dans laquelle le groupe alkyle est substitué par halogène, nitro, cyano, hétérocycle, aryle, hétéroaryle, amine non substitué, et son amide dérivé d'un acide carboxylique d'un groupe alkyle, hétérocyclique, aryle ou hétéroaryle, amine substitué par un groupe alkyle, hétérocyclique, aryle ou hétéroaryle et son amide dérivé d'un acide carboxylique d'un groupe alkyle, amine substitué indépendamment par un groupe alkyle et un groupe alkyle, hétérocyclique, aryle ou hétéroaryle, hydroxy, et son ester dérivé d'un acide carboxylique d'un groupe alkyle, hétérocyclique, aryle ou hétéroaryle; éther portant un groupe alkyle, hétérocyclique, aryle ou hétéroaryle; thiol, et son ester de thiol dérivé d'un acide carboxylique d'un groupe alkyle, hétérocyclique, aryle ou hétéroaryle; thioéther portant un groupe alkyle, hétérocyclique, aryle ou hétéroaryle, et ses dérivés sulfoxyde et sulfone; $-SO_3H$, ses sels pharmaceutiquement acceptables, son ester dérivé d'un alcool d'un groupe

alkyle, son amide non substitué, et son amide substitué par un ou deux groupes alkyle; $-CO_2H$, ses sels pharmaceutiquement acceptables, son ester dérivé d'un alcool d'un groupe alkyle, son amide non substitué, et son amide substitué par un ou deux groupes alkyle; $PO_3H_2$, ses sels pharmaceutiquement acceptables, son ester dérivé d'un alcool d'un groupe alkyle, son amide non substitué, et son amide substitué par un ou deux groupes alkyle; $-(R^8)PO_2H$ (où $R^8$ est l'hydrogène ou un groupe alkyle inférieur non substitué), ses sels pharmaceutiquement acceptables, son ester dérivé d'un alcool d'un groupe alkyle, son amide non substitué, et son amide substitué par un ou deux groupes alkyle; aldéhyde; cétone portant un groupe alkyle; carbamate, non substitué ou substitué par un ou deux groupes alkyle; peptidyle; et leurs combinaisons.

**8.** Utilisation selon la revendication 7, dans laquelle le terme "hétérocycle" désigne des noyaux non aromatiques chimiquement stables, y compris des noyaux non aromatiques soudés, ayant de 5 à 20 atomes, comprenant au moins un hétéroatome choisi entre l'azote, le soufre, le phosphore et l'oxygène.

**9.** Utilisation selon la revendication 8, dans laquelle le terme "aryle" désigne des noyaux aromatiques chimiquement stables, y compris des noyaux aromatiques soudés, ayant de 6 à 20 atomes de carbone.

**10.** Utilisation selon la revendication 6, dans laquelle A est un élément choisi entre

(1) l'hydrogène;
(2) un halogène;
(3) un groupe alkyle éventuellement substitué ayant la structure générale:

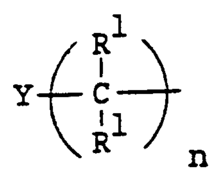

(2)

dans laquelle n est un nombre entier de 1 à 10, de préférence de 1 à 5, ou mieux est égal à 1 ou 2, et dans le meilleur des cas est égal à 1; chaque $R^1$ est choisi indépendamment de façon à donner des groupes chimiquement stables à partir de l'hydrogène, des halogènes, des groupes alkyle en $C_1$-$C_6$, d'un groupe amine non substitué ou de son amide dérivé d'un acide carboxylique d'un groupe alkyle en $C_1$-$C_6$, d'un groupe amine substitué par un groupe alkyle en $C_1$-$C_6$ ou de son amide dérivé d'un acide carboxylique d'un groupe alkyle inférieur, d'un groupe amine substitué indépendamment par deux groupes alkyle en $C_1$-$C_6$, d'un groupe hydroxy ou de son ester dérivé d'un acide carboxylique d'un groupe alkyle en $C_1$-$C_6$, de $-CO_2H$ ou de ses sels pharmaceutiquement acceptables, ou de son ester dérivé d'un alcool d'un groupe alkyle en $C_1$-$C_6$, ou de son amide non substitué, ou de son amide substitué par un ou deux groupes alkyle en $C_1$-$C_6$, d'un éther portant un groupe alkyle en $C_1$-$C_6$, de $-PO_3H_2$ ou de ses sels pharmaceutiquement acceptables, et du groupe nitro, ou bien deux $R^1$ sur le même atome de carbone sont $=O$ ou $=NR^9$ (où $R^9$ est un groupe alkyle en $C_1$-$C_6$ ou bien peut être l'hydrogène lorsqu'il y a un autre atome d'azote fixé au même atome de carbone que le groupe $=NR^9$), ou bien deux $R^1$ sur des atomes de carbone adjacents peuvent être remplacés par une liaison supplémentaire entre les atomes de carbone; ou bien un $R^1$ sur le premier atome de carbone (du côté droit de la structure (2) ci-dessus) et B (voir la structure (1) ci-dessus) peuvent être remplacés par une liaison supplémentaire; et Y est un substituant du groupe alkyle tel qu'il est défini précédemment; (pour des raisons de stabilité chimique des composés utilisés dans la présente invention, $R^1$ ne peut être tel qu'il y ait un atome d'halogène et un atome d'oxygène ou de soufre ou d'azote unis par une liaison simple au même atome de carbone, ou bien tel que deux d'un atome d'oxygène ou de soufre ou d'azote soient unis par une liaison simple au même atome de carbone);
(4) cycloalkyle ayant de 4 à 10 atomes de carbone, et de préférence 5 ou 6 atomes de carbone;
(5) hétérocycle ayant 5 ou 6 atomes dans le cycle; de préférence hétérocycles ayant 1 ou 2 atomes d'azote dans le cycle, ou mieux hétérocycles ayant un atome d'azote dans le cycle; ou encore mieux un groupe pipéridinyle, pyrrolidinyle, pipérazinyle ou morpholinyle éventuellement substitué;
(6) phényle éventuellement substitué; naphtyle;

(7) hétéroaryles à cycle de 5 ou 6 chaînons, éventuellement substitués, avec un ou deux hétéroatomes (notamment des hétéroatomes d'azote); le meilleur choix est le groupe pyridinyle;
(8) groupe aminé ayant la structure générale:

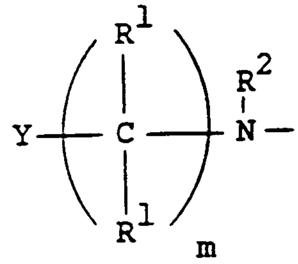

dans laquelle m est un nombre entier de 0 à 10, de préférence de 0 à 5, ou mieux égal à 0 ou 1, ou encore mieux m = 0; $R^1$ et Y sont tels que décrits précédemment; et $R^2$ est l'hydrogène, un groupe alkyle en $C_1$-$C_6$ ou un groupe acyle dérivé d'un acide carboxylique d'un groupe alkyle en $C_1$-$C_6$;
(9) groupe oxygéné ayant la structure générale:

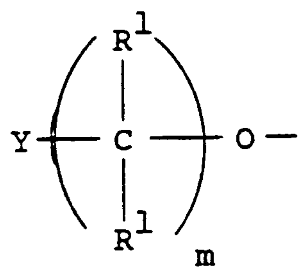

dans laquelle m est un nombre entier de 0 à 10, de préférence de 0 à 5, ou mieux égal à 0 ou 1, et dans le meilleur des cas m = 0; et $R^1$ et Y sont tels que décrits précédemment;
(10) groupe soufré ayant la structure générale:

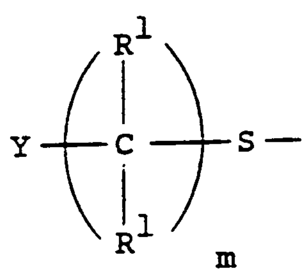

dans laquelle m est un nombre entier de 0 à 10, de préférence de 0 à 5, ou mieux égal à 0 ou 1, et dans le meilleur des cas m = 0; et $R^1$ et Y sont tels que décrits précédemment; et

(11) groupe contenant des peptides, ayant la structure générale:

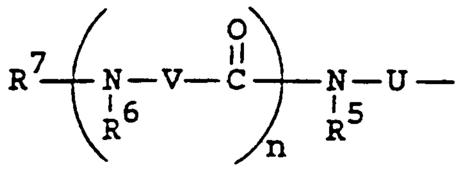

ou

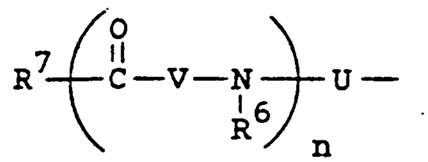

dans laquelle n est un nombre entier de 1 à 100, de préférence de 1 à 6; $R^5$, chaque $R^6$ et $R^7$ sont indépendamment l'hydrogène ou un groupe alkyle en $C_1$-$C_6$,de préférence $R^5$ et chaque $R^6$ et $R^7$ sont l'hydrogène; U et chaque V sont indépendamment des groupes alkyle en $C_1$-$C_6$ éventuellement substitués (substitués de telle sorte que le groupe soit chimiquement stable), ou bien $R^5$ et U ou chaque $R^6$ et V forment, avec l'atome d'azote auquel ils sont fixés, un cycle à 5 ou 6 chaînons qui est éventuellement substitué; ou bien U peut être inexistant; de préférence U et chaque V ou bien les cycles dans lesquels ils sont incorporés sont des éléments que l'on trouve dans les acides aminés naturels, c est-à-dire la lysine, la leucine, l'isoleucine, la valine, la phénylalanine, l'arginine, l'histidine, la méthionine, l'alanine, l'acide aspartique, la thréonine, la proline, la glycine, la sérine, la tyrosine, le tryptophane, la glutamine et la cystéine.